# EUROPEAN PATENT SPECIFICATION

(11) **EP 3 998 073 B1**
(45) Date of publication and mention of the grant of the patent: **06.11.2024**
(21) Application number: 21213252.6
(22) Date of filing: 02.12.2016
(51) Int. Cl.: A61K 35/407, C12N 5/00, C12N 5/071, C12M 1/12, C12M 3/00, C12M 3/06, B01L 3/00

(54) **DEVICES AND METHODS FOR SIMULATING A FUNCTION OF A LIVER TISSUE**
VORRICHTUNGEN UND VERFAHREN ZUR SIMULATION EINER FUNKTION EINES LEBERGEWEBES
DISPOSITIFS ET PROCÉDÉS DE SIMULATION D'UNE FONCTION D'UN TISSU HÉPATIQUE

(30) Priority: 04.12.2015 US 201562263246 P
(43) Date of publication of application: 18.05.2022
(62) Divisional of application: 16871652.0
(73) Proprietor: Emulate, Inc., Boston, MA 02110 (US)
(72) Inventor: Hamilton, Geraldine, Cambridge, MA 02141 (US); Jang, Kyung Jin, Andover, MA 01810 (US); Haney, Suzette, Dedham, MA 02026 (US); Ronxhi, Janey, Quincy, MA 02169 (US); Kodelia, Konstantia, Brookline, MA 02445 (US); Park, Hyoung Shin, Newton, MA 02458 (US); Sliz, Josiah Daniel, Cambridge, MA 02141 (US); Levner, Daniel, Brookline, MA 02446 (US); Otieno, Monicah, Robbinville, NJ 08691 (US); Petropolis, Debora, Cambridge, MA 02141 (US)
(74) Representative: Marks & Clerk LLP

(56) References cited:
- US-A1- 2013 236 972
- US-A1- 2014 342 445
- RENNERT KNUT ET AL: "A microfluidically perfused three dimensional human liver model", BIOMATERIALS, ELSEVIER SCIENCE PUBLISHERS BV., BARKING, GB, vol. 71, 25 August 2015 (2015-08-25), pages 119 - 131, XP029269412, ISSN: 0142-9612, DOI: 10.1016/J.BIOMATERIALS.2015.08.043
- JEAN MATTHIEU PROT ET AL: "The Current Status of Alternatives to Animal Testing and Predictive Toxicology Methods Using Liver Microfluidic Biochips", ANNALS OF BIOMEDICAL ENGINEERING, KLUWER ACADEMIC PUBLISHERS-PLENUM PUBLISHERS, NE, vol. 40, no. 6, 10 December 2011 (2011-12-10), pages 1228 - 1243, XP035062589, ISSN: 1573-9686, DOI: 10.1007/S10439-011-0480-5

## Description

### GOVERNMENT SUPPORT

### FIELD OF INVENTION

Various aspects described herein relate generally to microfluidic devices and methods of use and manufacturing thereof. In some embodiments, the microfluidic devices can be used for culture and/or support of living cells such as mammalian cells, and/or for simulating a function of a tissue, *e.g.,* a liver tissue. Endothelial cell culture media for long-term culture of endothelial cells are also provided herein. An in vitro liver-on-chip design is described that mimics the structure and function of the liver in vivo. Extended viability with sustained function over time is described based on enzymatic activity, allowing for drug testing, including pharmacokinetics, drug clearance, drug sensitivity, and drug toxicity.

### BACKGROUND

Conventional *in vitro* hepatic model systems, *e.g.,* 2-dimensional monolayers of primary hepatocytes, are limited by their inability to maintain normal phenotypic characteristics over time in culture, including stable expression of clearance and metabolic pathways (Phase I and Phase II metabolism). In addition, one of the main drawbacks of the existing cell culture system is its limitation in maintaining a long-term viability of the cells. Biomedical research and pharmaceutical drug development is currently a long and expensive process, with few guarantees for success. Drug testing today hinges on tools like static cell cultures and animal testing, both of which cannot accurately model the dynamic environment of the human body. Even drugs that succeed with animal testing often go on to fail in human clinical trials, wasting millions of dollars and years of research. What is needed is a better in vitro platform for drug testing.

US2013/236972 teaches the use of microfluidic devices as liver models
Rennert et al (Biomaterials, 71, 2015, pp119-131) discloses a microfluidcally perfused 3D human liver model comprising a vascular layer composed of endothelial cells and tissue macrophages, and a hepatic layer comprising stellate cells co-cultured with hepatocytes.

US2014/342445 discloses microfluidic liver chips comprising hepatocytes, which are placed on one side of a extracellular matrix (ECM)-coated membrane, with microvascular endothelial cells on the other side of the membrane

### SUMMARY OF THE INVENTION

Present invention is defined in accordance with the claims appended hereto. In one aspect, at least in part, design of devices that allow for a controlled and physiologically realistic co-culture of liver sinusoidal endothelial cells in one chamber with hepatocytes in other chamber(s) to establish hepatic function *in vitro.* In one embodiment, the chambers are aligned (*e.g*., vertically) with each other with one or more membranes separating them from each other ("liver-on-a-chip"). The liver-on-a-chip devices have been developed and optimized based on the basic design of an organ-on-a-chip as described in the U.S. Patent No. 8,647,861, and the International Patent App. No. PCT/US2014/071611,. In some aspects, the inventors have optimized the design of the liver-on-a chip devices and culture conditions to provide long-term hepatic culture with physiologically relevant hepatic function (*e.g.*, albumin and/or urea secretion, and/or CYP 450 metabolic capacity) for different animal models, *e.g*., human, rats, and dogs.

An in vitro liver-on-chip design is described that mimics the structure and function of the liver in vivo. Extended viability with sustained function over time is described based on enzymatic activity, allowing for drug testing, including pharmacokinetics, drug clearance, drug sensitivity, and drug toxicity.

The devices can comprise a monolayer of viable hepatocytes adhered a coating comprising a first extracellular matrix composition. The viable hepatocytes can be derived from different mammalian sources, including, *e.g*., but not limited to humans, rats, mice, and dogs. For human cells, a first extracellular matrix composition comprising collagen I coating produced good results. In the case of rat hepatocytes, a fibronectin mixture was found to work. In some embodiments, the hepatocytes are further overlayed with a coating comprising a second extracellular matrix composition. For human cells, a Matrigel overlay (with no fibronectin) produced good results. In the case of rat hepatocytes, a fibronectin mixture was found to work. Thus, the device can be used to simulate a function of a liver tissue in different animal model of interest.

In some embodiments of various aspects described herein, the devices can comprise a flowing culture medium in the first chamber, wherein the flowing culture medium generates a shear stress of no more than 0.2 dyne/cm². The inventors have discovered that addition of low shear stress improved liver cell morphology and long-term viability over static cultures.

In some embodiments of various aspects described herein, the hepatocytes growing in the devices described herein can display at least one or a combination of two or more of the following characteristics: (i) cuboidal morphology; (ii) formation of a bile canaliculus network; (iii) albumin secretion over a period of time; (iii) the presence of drug-metabolism function of at least one CYP450 enzyme or Phase II enzyme sustained over a period of time; and (iv) at least a 2-fold increase in activity of CYP450 drug metabolizing enzyme in the presence of an agent that induces CYP450 drug metabolizing enzyme.

The heights of the first chamber and the second chamber can vary to suit the needs of desired applications (*e.g*., to provide a low shear stress, and/or to accommodate cell size). The first chamber and the second chamber can have the same height or different heights. In some embodiments, a height ratio of the first chamber to the second chamber can range from 1:1 to about 20:1. In some embodiments, a height ratio of the first chamber to the second chamber can range from 1:1 to about 10:1.

In some embodiments, the height of the first chamber can range from about 100 µm to about 50 mm, or about 100 µm to about 5 mm. In one embodiment, the height of the first chamber can be about 150 µm. In one embodiment, the height of the first chamber can be about 1 mm.

In some embodiments, the height of the second chamber can range from about 20 µm to about 1 mm, or about 50 µm to about 500 µm. In one embodiment, the height of the second chamber can be about 150 µm. In one embodiment, the height of the second chamber can be about 100 µm.

The width of the first chamber and/or the second chamber can vary with desired cell growth surface area. In some embodiments, the width of the first chamber and/or the second chamber can range from about 100 µm to about 200 mm, or about 100 µm to about 5 mm. In one embodiment, the width of the first chamber and/or the second chamber can be about 1 mm.

The membrane separating the first chamber and the second chamber in some embodiments of the devices described herein can be rigid or at least partially flexible. In some embodiments, the membrane can be configured to deform in a manner (e.g., stretching, retracting, compressing, twisting and/or waving) that simulates a physiological strain experienced by the cells in its native microenvironment. In these embodiments, the membrane can be at least partially flexible.

The membrane can be of any thickness. In some embodiments, the membrane can have a thickness of about 1 µm to about 300 µm or about 100 nm to about 50 µm. In one embodiment, the liver chip has a 50 µm thick membrane.

The membrane can be non-porous or porous. In some embodiments where at least a portion of the membrane is porous, the pores can have a diameter of about 0.1 µm to about 15 µm, and more preferably 7 µm.

While the first chamber and the second chamber can be in any geometry or three-dimensional structure, in some embodiments, the first chamber and the second chamber can be configured to be form channels.

Not only have the inventors created a device for simulating a function of a liver tissue, the inventors have also identified a beneficial endothelial cell culture medium for long term culture of endothelial cells. Accordingly, in one teaching, described herein is a method of maintaining long-term viability of endothelial cells. The method comprises contacting a population of endothelial cells with an endothelial cell culture medium comprising: (a) a basal endothelial medium; and (b) a supplement cocktail comprising (or consisting of) Vitamin C (50 ug/mL, insulin, transferrin, selenium, dexamethasone (1uM), glutamx (1%) and pen-strep (1%), and FBS (at a concentration of about 0.3% to about 10%). In another embodiment, the method comprises contacting a population of endothelial cells with an endothelial cell culture medium comprising: (a) a basal endothelial medium; and (b) a supplement cocktail comprising (or consisting of) VEGF(Vascular endothelial growth factor), Heparin, EGF (Epidermal growth factor), Hydrocortisone, L-Glutamine, FBS (Fetal Bovine Serum) (at a concentration of about 0.3% to about 10%). Using the endothelial cell culture medium, the endothelial cells can be maintained as a viable monolayer for at least two weeks or longer.

Similarly, the inventors have also identified a beneficial epithelial cell culture medium for long-term culture of epithelial cells. Accordingly, in one aspect, described herein is a method of maintaining long-term viability of epithelial cells (e.g. hepatocytes). The method comprises contacting a population of epithelial cells with an epithelial cell culture medium comprising: (a) a basal epithelial medium; and (b) a supplement cocktail comprising (or consisting of) Vitamin C, insulin, transferrin, selenium, dexamethasone, glutamax and pen-strep, and FBS (at a concentration of about 1 to 10%) (as described above). In some embodiments, the basal epithelial medium is Williams E medium. Using the epithelial cell culture medium, the hepatocytes can be maintained as a viable monolayer for at least two weeks or longer.

Kits for cell culture are also described herein. In one background teaching, the kit comprises (a) a cell culture device; and (b) an endothelial cell culture medium according to one or more embodiments described herein.

In some embodiments, the basal medium and the supplement cocktail provided in the kit can be pre-mixed to form the endothelial cell culture medium. In some embodiments, the basal medium and the supplement cocktail provided in the kit can be pre-mixed to form the epithelial cell culture medium.

In some embodiments, the basal medium and the supplement cocktail provided in the kit can be separately packaged. For example, the basal medium and the supplement cocktail can be packaged independently, *e.g*., as powder or liquid.

In some embodiments, the kit can further comprise at least one vial of cells. In one embodiment, the cells can be endothelial cells. In one embodiment, the cells can be tissue-specific cells.

In one embodiment, said viable human hepatocytes are covered with a Matrigel overlay. The present invention contemplates additional cells. For example, in one embodiment, the method further comprises d) adding viable human Kupffer cells to said viable human liver sinusoidal endothelial cells. In one embodiment, the method further comprises e) culturing said viable Kupffer cells under conditions such that said Kupffer cells move about on said viable human liver sinusoidal endothelial cells. In one embodiment, the method further comprises f) monitoring the movement of said Kupffer cells in real time.

Kupffer cells can mediate or participate in various forms of hepatotoxicity and liver pathology, including drug-induced liver injury, immune-mediated adverse drug reactions, and lesion growth. Accordingly, the present invention contemplates using Kupffer cells to model the associated Kupffer cell-mediated toxicity. In some embodiments, Liver-Chips comprising Kupffer cells are used to assess toxicity, potentially identifying Kupffer-mediated effects. In some embodiments, the response of Liver-Chips with and without Kupffer cells may be compared in order to evaluate the participation of Kupffer cells in the response (this participation may cause, for example, elevated or diminished toxicity, elevated or diminished drug metabolism, etc.).

In some embodiments, Liver-Chips comprise Kupffer cells corresponding to different species (e.g. rat, dog, human; the other cells in the Liver-Chip typically match the Kupffer cells in species, but this is not required). Such embodiments may be employed to evaluate species differences in Liver-Chip response or drug-metabolism and especially species-differences in the participation of Kupffer cells in the response. Species differences in Kupffer mediation are known to exist; thus, there is a need for a reliable microfluidic model to assess them.

In one embodiment, the present invention contemplates a microfluidic open top 3D liver-on-chip device comprising three different cell layers. These three cell layers include but are not limited to 1) a Liver Stellate cell and 3D ECM layer, 2) a Hepatocytes layer, and 3) a LSEC and Kupffer cells layer. In one embodiment, Liver Stellate cells are embedded inside a 3D ECM matrix layer. In one embodiment, the hepatocytes layer is a layer of primary hepatocytes. In one embodiment, the hepatocytes (layer) are cultured on the top of the 3D ECM gel (layer). In one embodiment, the 3D liver-on-chip has a LSEC (primary cells) layer at the bottom channel of the chip. In one embodiment, after the LSEC layer is seeded then Kupffer primary cells are added by seeding into the top of the LSEC layer.

In one embodiment, a microfluidic open top 3D liver-on-chip device comprising 3D ECM allows Kupffer cell migration between the chip compartments. As a nonlimiting example, Kupffer cells in the bottom channel may migrate into the top channel. Thus in one embodiment, when present, a device comprising a membrane has pore sizes allowing Kupffer cells to move through it. In one embodiment, the ECM layer has openings (pore sizes) allowing Kupffer cells to move through it. Thus in one embodiment, measuring Kupffer cells migration between compartments can be used to study liver toxicity mechanisms. Cell migration may be measured by one or more parameters, including but not limited to the number of Kupffer cells having migratory morphology, the number of cells counted in comparative compartments, i.e. in specific layers or channels, distances individual cells move, etc.

In one embodiment, a microfluidic open top 3D liver-on-chip device comprising 3D ECM containing embedded Liver Stellate cells provides an *in vitro* mimic of natural liver sinusoid architecture. In one embodiment, Stellate cells in a 3D environment are contemplated to recreate the liver tissue architecture. In one embodiment, a 3D environment is contemplated for enabling the Stellate cells to develop a more complete profile of cellular phenotypes related to sick and health state, e.g. a fibrotic state.

Because increasing liver ECM fiber density is associated with fibrotic liver disease, the following embodiments are contemplated for modeling changes in ECM fiber density, including but not limited to increasing fiber density or decreasing fiber density. Thus, in one embodiment, a microfluidic open top 3D liver-on-chip device comprising 3D ECM containing embedded Liver Stellate cells provides an *in vitro* model for fibrotic liver diseases. In one embodiment, a microfluidic open top 3D liver-on-chip device comprising 3D ECM containing embedded Liver Stellate cells provides an *in vitro* model for inflammatory fibrotic liver diseases. In one embodiment, a microfluidic open top 3D liver-on-chip device comprising 3D ECM containing embedded Liver Stellate cells provides an *in vitro* model for fibrotic liver disease scarring.

In one embodiment, a microfluidic open top 3D liver-on-chip device comprising 3D ECM containing embedded Liver Stellate cells provides an *in vitro* model for inflammatory fibrotic liver disease characteristics in relation to exposure to test toxic liver compounds. Thus, in one embodiment, higher levels of Stellate cell migration in 3D liver-on-chip devices are contemplated in response to test compounds that show toxic effects on liver cells as compared to Liver Stellate cells migration in microfluidic chips that do not contain 3D ECM. In one embodiment, a microfluidic open top 3D liver-on-chip device comprising 3D ECM has an increased in deposition of ECM materials including but not limited to Collagen I, other ECM components. ECM materials contemplated for use may be modified from that found in tissue, such as modifications of Collagen I fibers structure, e.g. for increasing ECM fiber density.

In one embodiment, a microfluidic open top 3D liver-on-chip device comprising 3D ECM can be use as a platform to screen for anti-fibrotic compounds. In one embodiment, a microfluidic open top 3D liver-on-chip device comprising 3D ECM can be use as a platform to screen for pro-fibrotic compounds. In one embodiment, a microfluidic open top 3D liver-on-chip device comprising 3D ECM finds use as a platform to identify mechanisms of action for a test compound.

Because activated Stellate cells are associated with increasing fiber density linked to fibrotic liver disease, the following embodiments are contemplated for modeling inhibition of Stellate activation. In one embodiment, the ECM porosity is modified for inhibiting nonactivated embedded Stellate cells migration. In one embodiment, the ECM porosity is modified for inhibiting Stellate cell activation. In one embodiment, the ECM stiffness is modified for inhibiting nonactivated embedded Stellate cell migration. In one embodiment, the ECM stiffness is modified for inhibiting Stellate cell activation. In one embodiment, a test compound is added to inhibit activation and/or migration of ECM embedded Stellate cells.

Different cells types respond different to chemical/mechanical 3D ECM stimulus. Thus, in one embodiment, a microfluidic open top 3D liver-on-chip device comprising 3D ECM containing embedded Liver Stellate cells will compare Liver Stellate cell responding to various types of 3D ECM compositions. While not intending to limit the source of Liver Stellate cells from a particular species, in one embodiment, the Liver Stellate cells are human Stellate cells, in one embodiment, the Liver Stellate cells are rat Stellate cells. While not intending to limit the condition of Liver Stellate cells, in one embodiment, the Liver Stellate cells are primary cells, in one embodiment, the Liver Stellate cells are cultured cells.

Types of 3D ECM are contemplated to modify hepatic cell culture parameters, including but not limited to maximum culture time of healthy hepatic cells, expression of mature hepatic phenotype, sensitivity to CYP induction, levels of albumin expression, etc. Thus, in one embodiment, a microfluidic open top 3D liver-on-chip device comprises different types of ECM, including but not limited to ECM described herein.

Nutrient perfusion through the cells in a microfluidic open top 3D liver-on-chip device is different between 3D and 2D cell culture environments. In other words, different chip designs have different perfusion rates and/or patterns. Thus, in some embodiments, the flow rate parameter at the bottom channel is modified for desired rates and/or for a particular cell type used in the device.

### BRIEF DESCRIPTION OF THE DRAWINGS (any embodiments and examples, which do not fall under the scope of the claims, are provided by way of background or reference purposes)

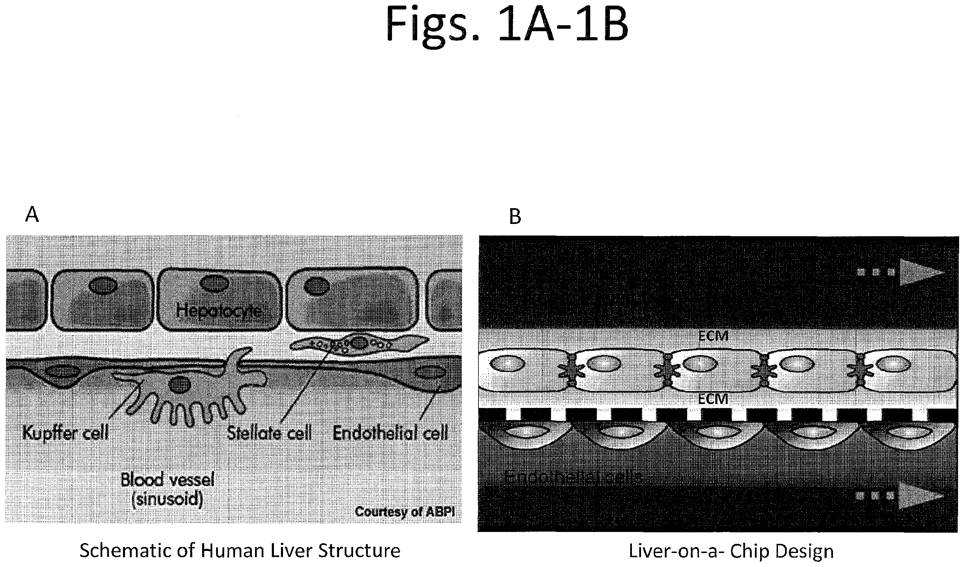

**Figs. 1A-1B** are schematic diagrams showing a cross-sectional view of a human liver structure **(****Fig. 1A****)** and of a liver-on-a- chip device according to one embodiment described herein **(****Fig. 1B****),** respectively. The device is designed to mimic liver architecture and to support normal hepatocyte polarization for optimal hepatocyte viability and function.
**Figs. 2A-2D** are images showing different embodiments of the devices described herein and seeding of hepatocytes and human liver sinusoidal endothelial cells (hLSECs) through different ports on the devices. **Fig. 2A** is a schematic diagram showing one embodiment of a liver-on-a-chip device. **Fig. 2B** is a schematic diagram showing another embodiment of a liver-on-a-chip device. **Fig. 2C** is a picture showing seeding of hepatocytes into the first chamber through a first port. **Fig. 2D** is a picture showing seeding of hLSECs into the second chamber through a second port.

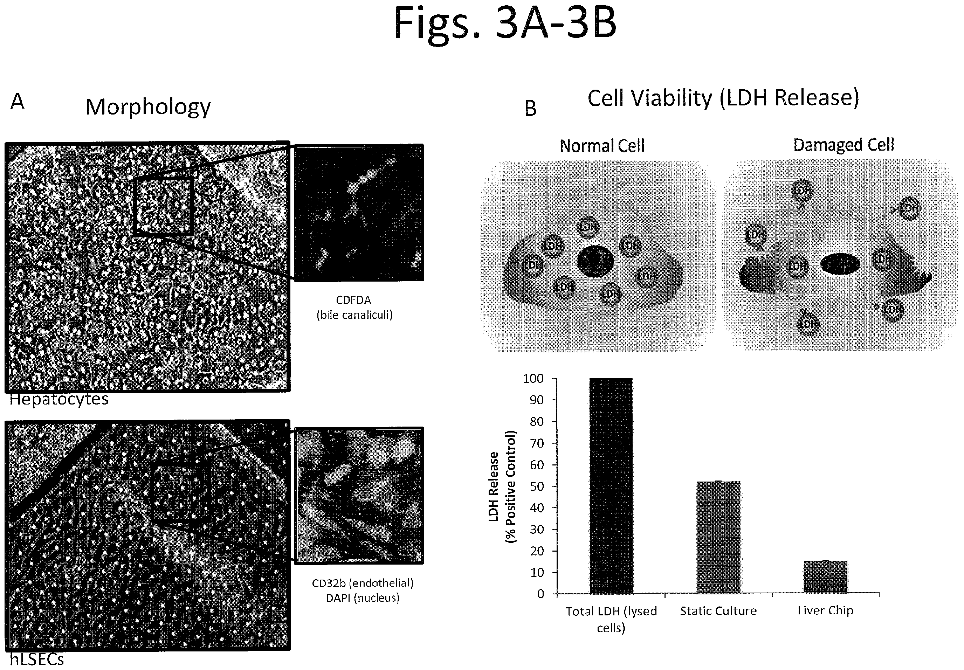

**Figs. 3A-3B** are experimental data showing human hepatocytes and hLSECs cultured under flow in liver-on-a-chip device according to one embodiment described herein. **Fig. 3A** is a set of images showing hepatocytes (top panel) and hLSECs (bottom panel) at Day 13 of culture. The insets are fluorescent images showing (top panel) a live stain for CDFDA (a marker that is excreted into the bile via efflux transporters) to show formation of bile canaliculi; and (bottom panel) a stain for CD32b and DAPI to show viability of endothelial cells. **Fig. 3B** includes a bar graph showing that hepatocytes (on a chip that contains both hepatocytes and liver sinusoidal endothelial cells) released significantly lower levels of lactate dehydrogenase (LDH) than cells in a static culture, indicating that subjecting the cells to a low shear stress (*e.g*., a fluid flow) can facilitate maintenance of cell viability.

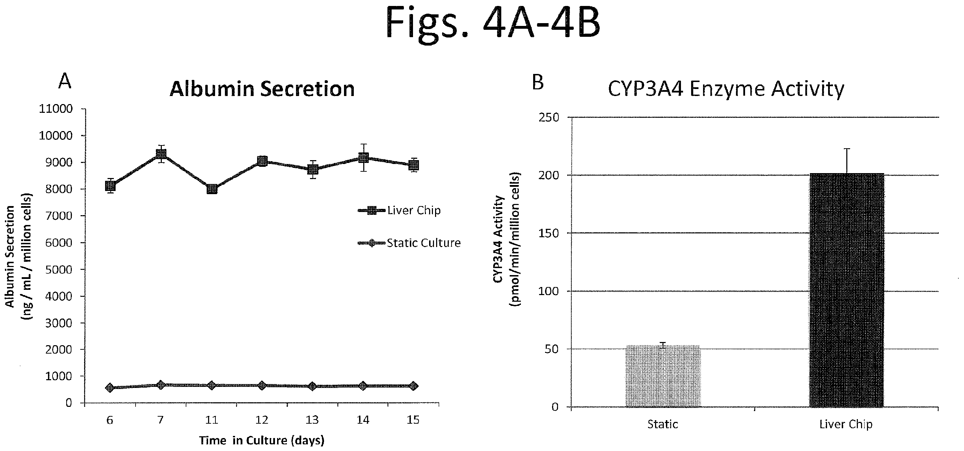

**Figs. 4A-4B** are data graphs showing hepatic function of a liver-on-a-chip device (also referred to as "liver chip") according to one embodiment described herein. **Fig. 4A** is a line graph showing that albumin secretion was significantly higher in the liver chip than in conventional static culture, and the high albumin secretion was maintained in the liver chip for at least 2 weeks or longer, indicating improved viability of hepatocyte-hLSEC co-culture in the liver chip than in the static culture. **Fig. 4B** is a bar graph showing that the liver chip's basal CYP3A4 enzyme activity level was at least 4 times greater than the level in static culture.

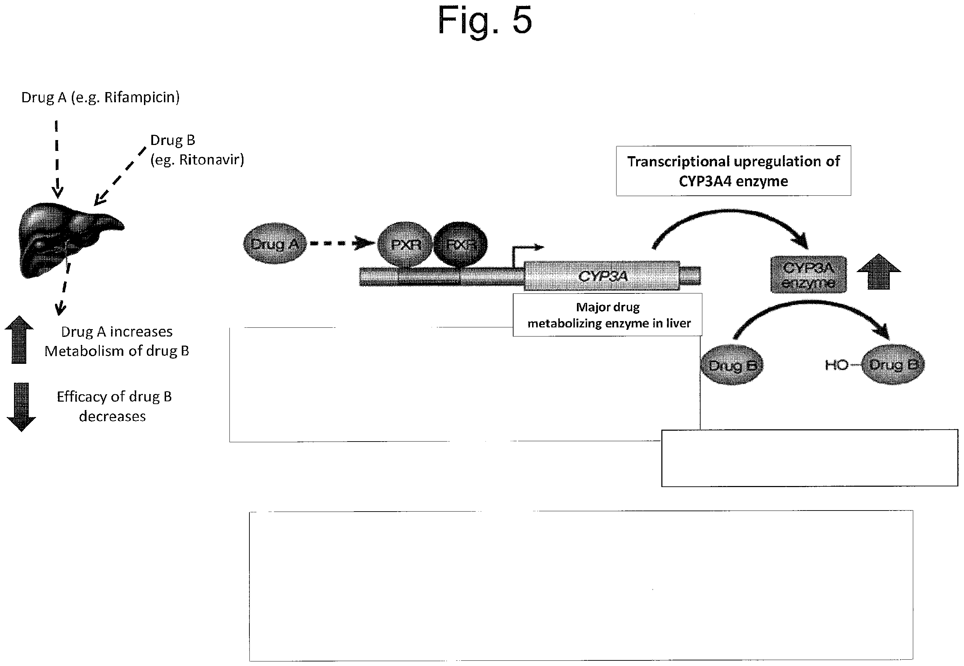

**Fig. 5** is a schematic illustration of functional relevance of liver CYP450 enzyme induction. When a subject takes both Drug A and Drug B together and both drugs interact with a CYP3A4 enzyme, Drug A can increase the metabolism of Drug B or decrease the efficacy of drug B. For example, Drug A can bind the nuclear receptor PXR in the hepatocytes and cause upregulation of the CYP3A4 enzyme, which will in turn increase metabolite formation of Drug B and thus reduce the level of Drug B in circulation and its efficacy. In addition, the increased metabolite formation of Drug B can increase toxic metabolites. In other circumstances, Drug A can lead to the inhibition of a liver enzyme such as a CYP450 enzyme. Such circumstances may also lead to drug-drug interaction, and it is often beneficial to identify them.

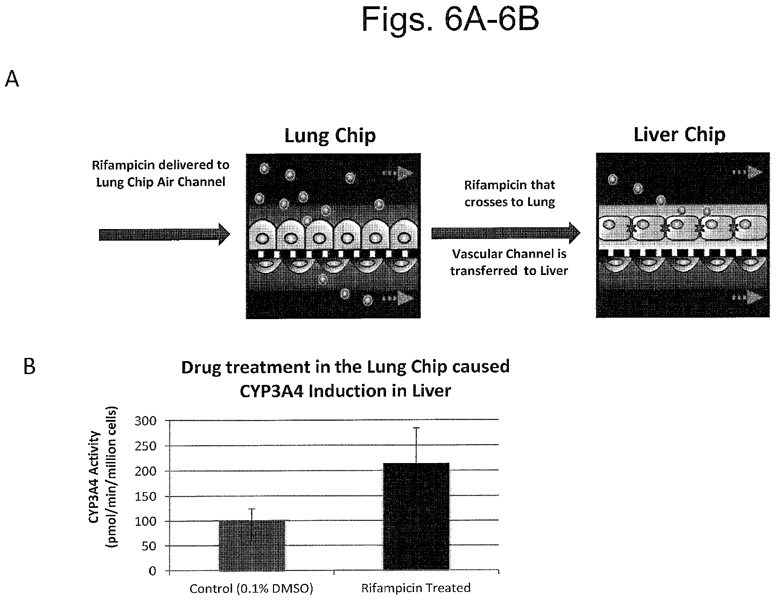

**Figs. 6A-6B** include experimental data showing drug treatment in a lung-on-a-chip device can cause CYP3A4 induction in a liver-on-a- chip device. **Fig. 6A** is a schematic diagram showing physiological coupling between a lung chip (*e.g.,* as described in the U.S. Patent No. 8,647,861, the content of which is incorporated herein by reference in its entirety) and a liver chip. Rifampicin was introduced to the "air" channel" with lung epithelial cells of the lung chip. The rifampicin delivered across the lung epithelial cells and endothelial cells to the "vascular" channel of the lung chip was then directed to hepatocytes in the liver chip. **Fig. 6B** is a bar graph showing that a ~2 fold increase in CYP3A4 drug metabolizing enzyme activity was detected in the liver chip when rifampicin was added in the lung chip.
**Figs. 7A-7D** are images showing different embodiments of the devices described herein and seeding of hepatocytes and human liver sinusoidal endothelial cells (hLSECs) through different ports on the devices. **Fig. 7A** is a schematic diagram showing one embodiment of a liver-on-a-chip device. **Fig. 7B** is a schematic diagram showing another embodiment of a liver-on-a-chip device. **Fig. 7C** is a picture showing seeding of hepatocytes into the first chamber through a first port. **Fig. 7D** is a picture showing seeding of hLSECs into the second chamber through a second port.
**Fig. 8** is a bar graph showing that basal CYP3A4 activity levels steadily increased in the liver chip shown in **Fig. 7A** (Liver chip v1), while the liver chip shown in **Fig. 7B** (Liver chip v2) showed comparable activity levels to fresh hepatocytes and the basal CYP3A4 activity was maintained in Liver chip v2 for at least about 2 weeks.
**Figs. 9A-9B** are bar graphs showing hepatic function of hepatocytes that were collected from human subjects cultured in the liver chip. Hepatocytes from Donor 1 **(****Fig. 9A****)** and Donor 2 **(****Fig. 9B****)** exhibited *in vivo*-relevant basal CYP3A4 levels for at least about 2 weeks when they were cultured in the liver chips, as compared to static cultures.
**Fig. 10** is a bar graph showing that basal levels of CYP3A4 activity were higher in the liver chips than in static Matrigel^{®} sandwich culture, the industry "gold standard." The CYP3A4 activity was reflected by measuring formation of 6-b-hydroxytestosterone from testosterone due to an increased CYP3A4 activity induced by rifampin.
**Fig. 11** is a bar graph showing that induction of CY3A4 activity in the human liver chips increased with concentrations of dexamethasone.
**Figs. 12A-12B** are fluorescent images comparing formation of bile canaliculi in the liver chips and static culture. A live stain for CDCFDA (a marker that is excreted into the bile via efflux transporters) was used. The stains showed increased bile canaliculi active transport by multidrug resistance-associated protein 2 (MRP2) in the liver chip **(****Fig. 12B****),** as compared to static culture **(****Fig. 12A****).** This shows increased formation of bile canaliculi in the liver chip, as compared to the static culture.
**Fig. 13A** are data graphs showing that both hepatocytes and hLSEC monolayer maintained viable over a period of at least 2 weeks. **Fig. 13A** is a bar graph showing that both hepatocytes and hLSECs maintained low LDH release levels over a period of at least about 2 weeks. **Fig. 13B** is a line graph showing measurement of albumin production over time, indicating that albumin secretion was maintained in the liver chip for at least 2 weeks or longer.
**Figs. 14A-14B** outline an exemplary optimization process to achieve long-term maintenance (*e.g*., viability and function) of a co-culture model of rat hepatocytes and rat LSECs (rLSECs) in a device as shown in **Fig. 7B****.** **Fig. 14A** shows four optimization parameters, namely (1) substrate surface treatment (*e.g*., PDMS surface treatment); (2) extracellular matrix (ECM) coating; (3) ECM overlay; and (4) culture medium composition. **Fig. 14B** is a schematic diagram showing exemplary surface treatment methods, *e.g*., but not limited to APTES treatment and/or plasma treatment.

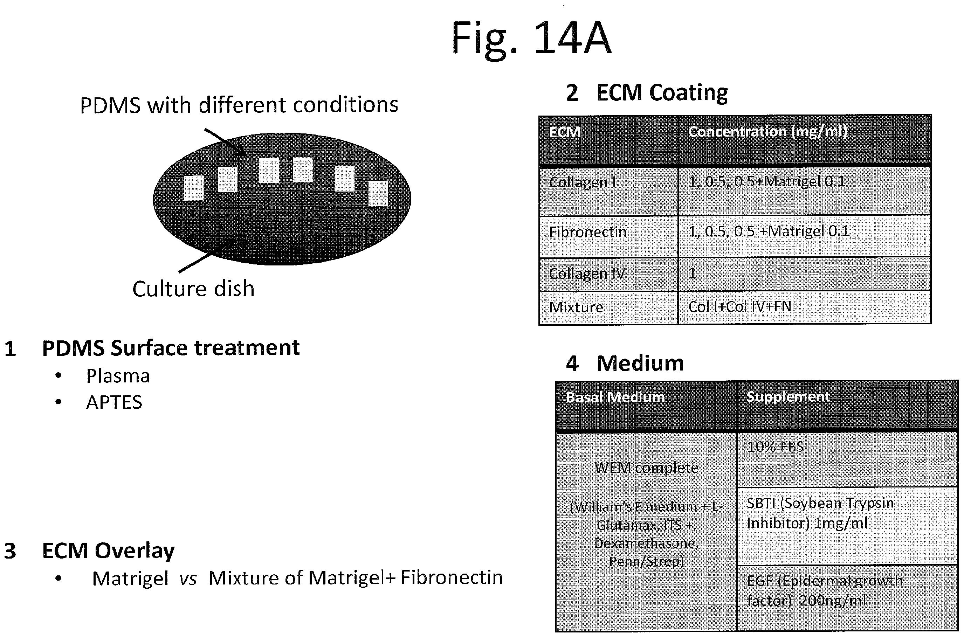

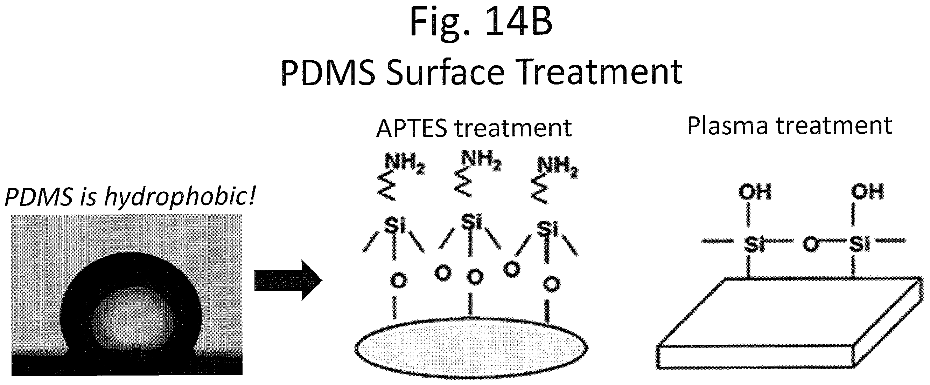

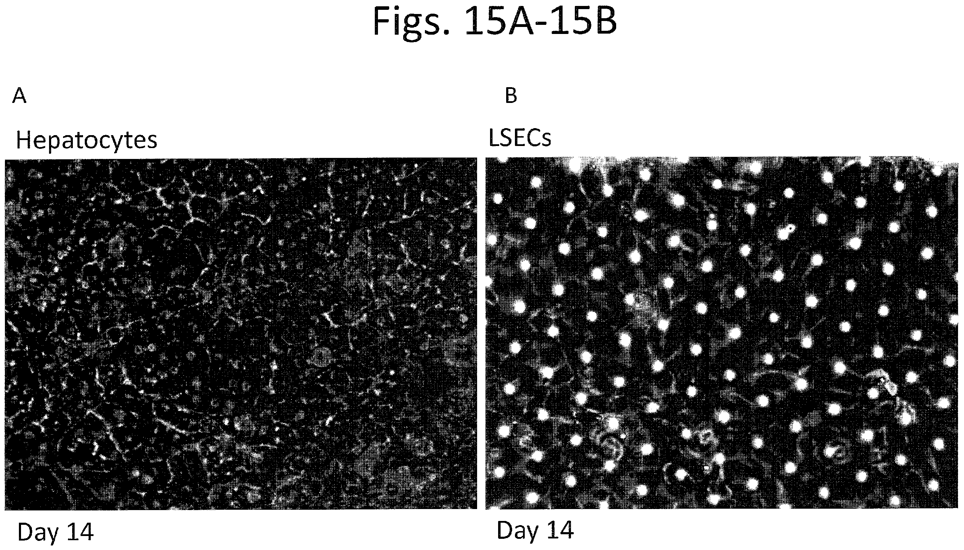

**Figs. 15A-15B** are phase contrast images showing a viable 2-week co-culture of rat hepatocytes **(****Fig. 14A****)** and rLSECs **(****Fig. 14B****)** in a liver-on-a-chip device that was optimized according to **Fig. 14A****.** Prior to culturing hepatocytes, the PDMS surface was plasma-treated, followed by an ECM coating of fibronectin (higher than 0.5 mg/mL) or an ECM coating mixture of fibronectin, collagen IV and collagen I. The ECM coating was further overlaid with a mixture of Matrigel^{®} and fibronectin. The WEM complete basal medium supplemented with about 10% FBS was used for the culture.
**Fig. 16A** are line graphs showing improved viability of rat hepatocytes and rLSECs in the rat liver chips than in static cultures (*e.g*., plate culture). **Fig. 16A** shows that unlike static culture (*e.g*., plate culture), both rat hepatocytes and rLSECs maintained low LDH release levels over a period of at least about 2 weeks. **Fig. 16B** showed measurement of albumin production over time, indicating that albumin secretion in the rat liver chip was maintained or increased over at least 2 weeks.

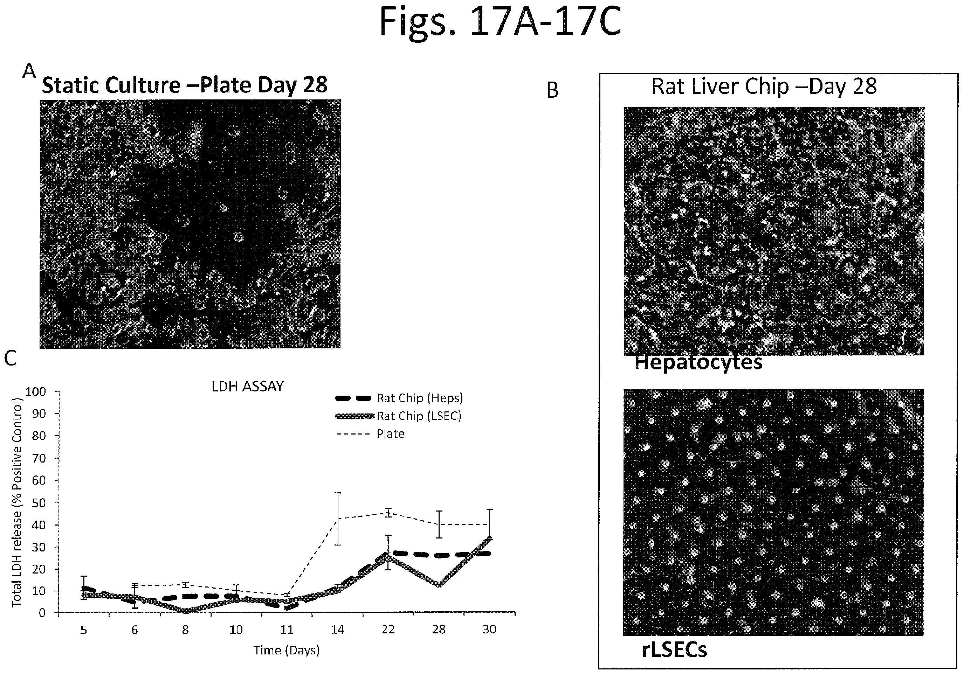

**Figs. 17A-17C** are experimental data showing a long-term co-culture (4 weeks) of rat hepatocytes and rLSECs in a rat liver chip. **Fig. 17A** is a phase contrast image showing cell morphology of a static plate culture after 4 weeks. **Fig. 17B** is a set of phase contrast images showing cell morphology of rat hepatocytes (top panel) and rLSECs (bottom panel) cultured in the rat liver chip after 4 weeks. **Fig. 17C** is a line graph showing improved viability of rat hepatocytes and rLSECs in the liver chip over a period of at least 4 weeks than in a static culture, as evidenced by a lower LDH release level measured in the liver chip.
**Figs. 18A-18B** are bar graphs showing effects of serum on CYP450 mRNA expression and enzyme activity of rat hepatocytes. **Fig. 18A** shows that serum inhibited mRNA expression of cyp1a1, but no significant effect on cyp2b1 and cyp3a1. **Fig. 18B** shows that the activity of CYP3A and CYP2B are serum-independent, as different concentrations of serum showed no effect on CYP3A and CYP2B activity.
**Figs. 19A-19C** are bar graphs showing a time course of CYP450 mRNA expression in rat hepatocytes. The mRNA expression of cyp2b1 **(****Fig. 19A****),** cyp3a1 **(****Fig. 19B****),** cyp1a1 **(****Fig. 19C****)** in rat hepatocytes declined over time.
**Figs. 20A-20B** are bar graphs showing a time course of CYP3A activity in rat hepatocytes cultured in static cultures and liver chips. **Fig. 20A** shows that CYP3A1/A2 enzyme activity in rat hepatocytes declined over time in static cultures (e.g., conventional sandwich plate culture). **Fig. 20B** shows improved CYP3A4 activity in rat liver chips over static culture and maintenance of the CYP3A4 activity in the liver chip for at least two weeks.

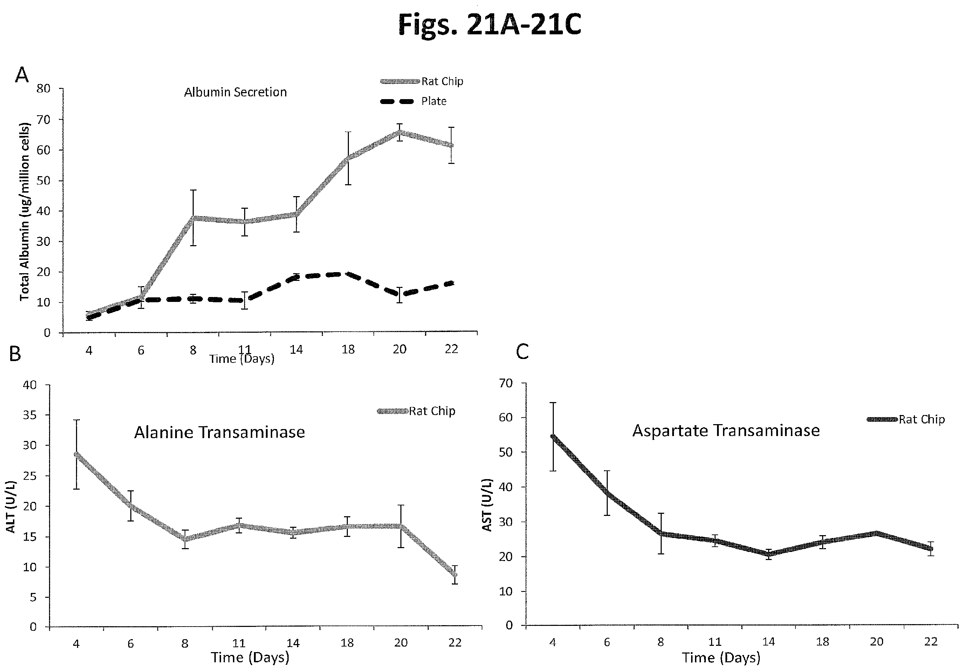

**Figs. 21A-21C** are line graphs showing liver specific functions measured in a rat liver chip. **Fig. 21A** shows high levels of albumin secretion in the rat liver chip, whereas the static plate shows low levels of albumin, indicating that the rat liver chips had improved hepatocyte viability over the static cultures. **Fig. 21B** shows a time course of alanine transaminase (ALT) activity measured in the liver chip. **Fig. 21C** shows a time course of aspartate transaminase (AST) measured in the liver chip. The ALT and AST activity levels measured in the liver chips were within the corresponding normal ranges in rat and human *in vivo* as shown in Table 3 in the Examples.
**Fig. 22** is a line graph showing urea synthesis function measured in a rat liver chip. Urea levels were maintained in the rat liver chips for at least 2 weeks and the levels were higher than static cultures (*e.g*., plates).
**Figs. 23A-23B** are line graphs showing hepatic function of rat hepatocytes obtained from different vendors. **Fig. 23A** shows albumin secretion of rat hepatocytes from Xenotech cultured in rat liver chips or in static culture plates. **Fig. 23B** shows albumin secretion of rat hepatocytes from Biopredic cultured in rat liver chips or in static culture plates.
**Figs. 24A-24D** are phase contrast images showing cell morphology of dog hepatocytes growing on a surface with various ECM coatings. An ECM coating of collagen I was used in **Figs. 24A** and **24C****,** while an ECM mixture coating comprising fibronectin, collagen IV, and laminin was used in **Figs. 24B** and **24D. Figs. 24A -24B** correspond to 1-day cultures; and **Figs. 24C-24D** correspond to 6-day cultures. The figures show that an ECM coating of collagen I provided improved viability and growth of dog hepatocytes.

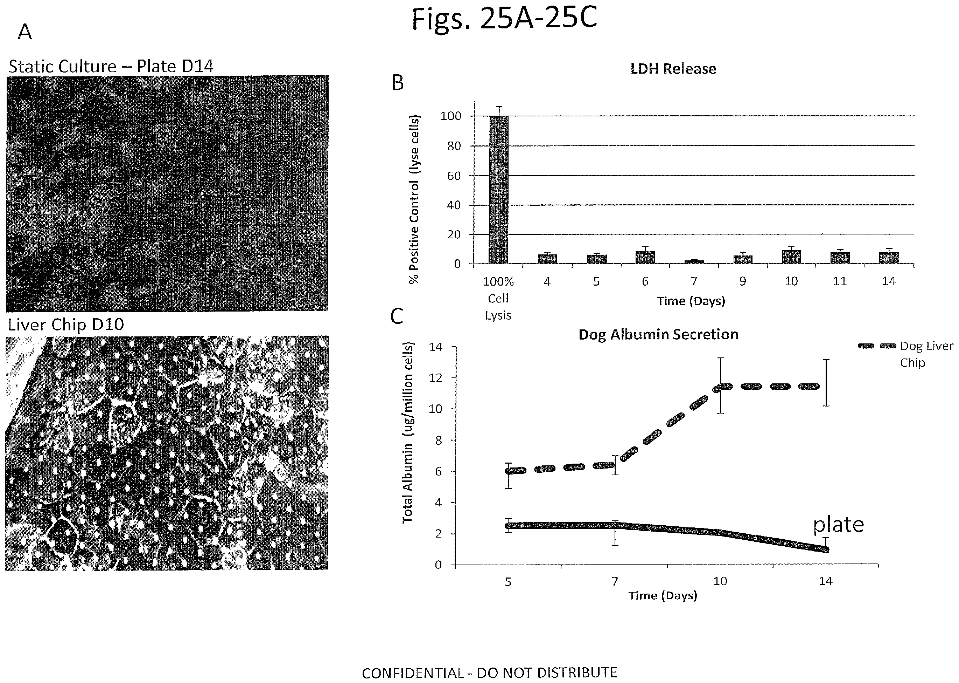

**Figs. 25A-25C** are experimental data showing cell viability and function of dog hepatocytes and dLSECs cultured in a dog liver chip. **Fig. 25A** is a set of phase contrast images showing cell morphology of dog hepatocytes in static cultures (top panel) and liver chips (bottom panel). **Fig. 25B** is a bar graph showing dog hepatocytes maintained low LDH release levels over a period of at least about 2 weeks, indicating viability and health of the hepatocytes monolayer being maintained in the dog liver chip over a period of at least 2 weeks. **Fig. 25C** is a line graph showing measurement of albumin production over time, indicating that the dog liver chip remained viable and maintained a high level of albumin secretion for at least about 2 weeks, as compared to static plate cultures.
**Figs. 26A-26B** are microscopic images showing cell morphology of dog hepatocytes cultured in the liver chips after 2 weeks. **Fig. 26A** is a phase contrast image showing cuboidal morphology of dog hepatocytes. **Fig. 26B** is a fluorescent image showing F-actin staining of pericanalicular distribution in the hepatocyte monolayer cultured in the dog liver chips.
**Fig. 27A** illustrates a perspective view of a device in accordance with an embodiment. **Fig. 27B** illustrates an exploded view of the device in accordance with an embodiment.
**Fig. 28** illustrates a system diagram employing at least one device described herein, which can be fluidically connected to another device described herein, an art-recognized organ-on-a-chip device, and/or to fluid sources.
Figure 29 provides an in vivo schematic (left side) showing the normal cellular architecture of the liver, and three in vitro schematics (right) showing a two cell arrangement (top right), a three cell arrangement (middle right) with the addition of Kupffer cells, and a four cell arrangement (bottom right) with the addition of both Kupffer cells and Stellate cells, on a microfluidic chip comprising two channels separated by a membrane.

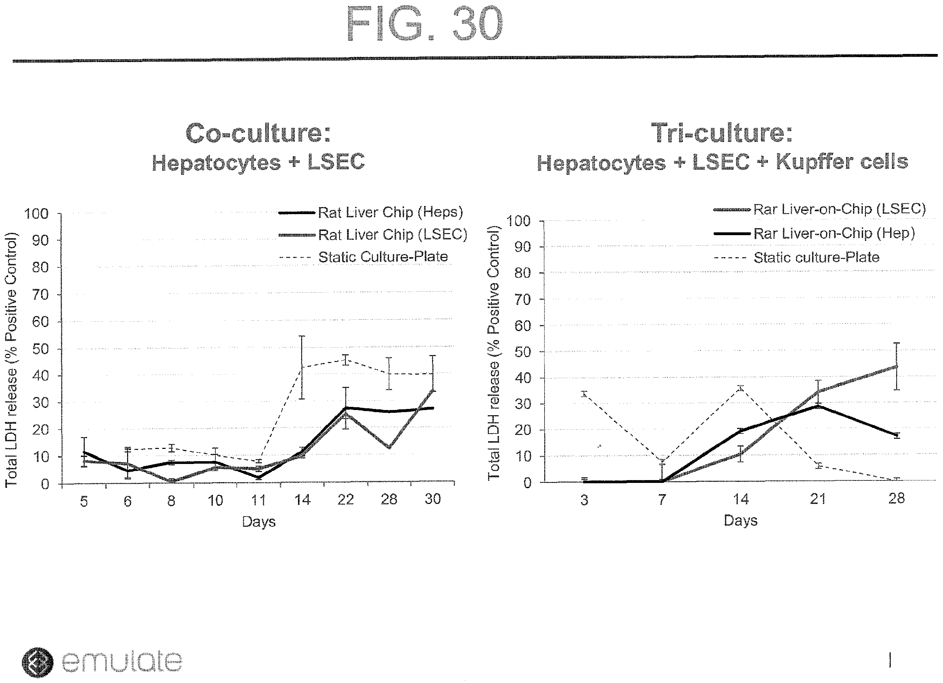

Figure 30 provides two graphs measuring LDH release in a two cell arrangement (left) and a three cell arrangement (right) on a microfluidic "Rat Liver-on-chip," as compared to static culture plates.

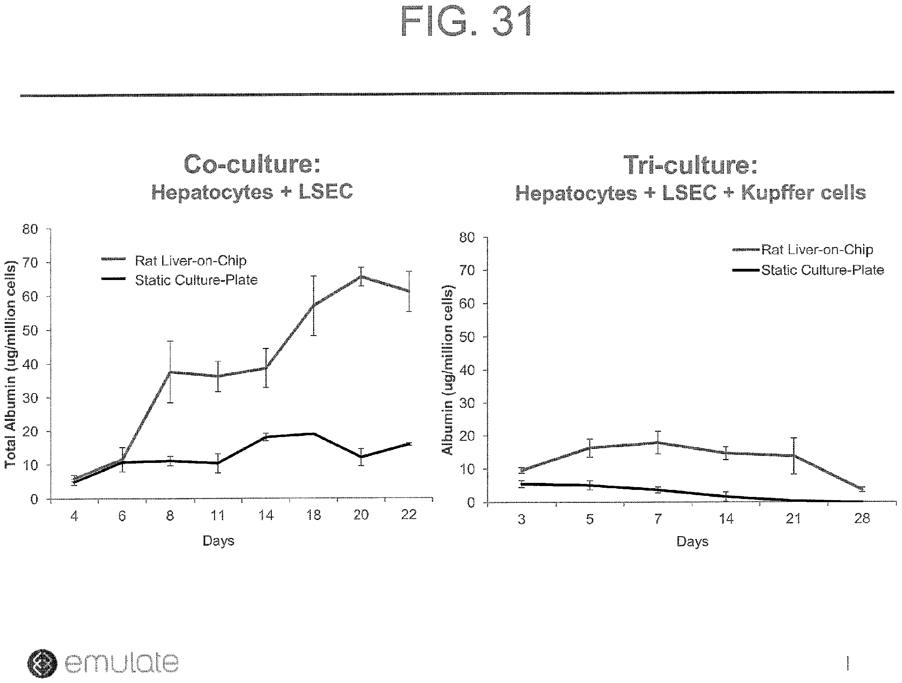

Figure 31 provides two graphs measuring albumin in a two cell arrangement (left) and a three cell arrangement (right) on a microfluidic "Rat Liver-on-chip," as compared to static culture plates.

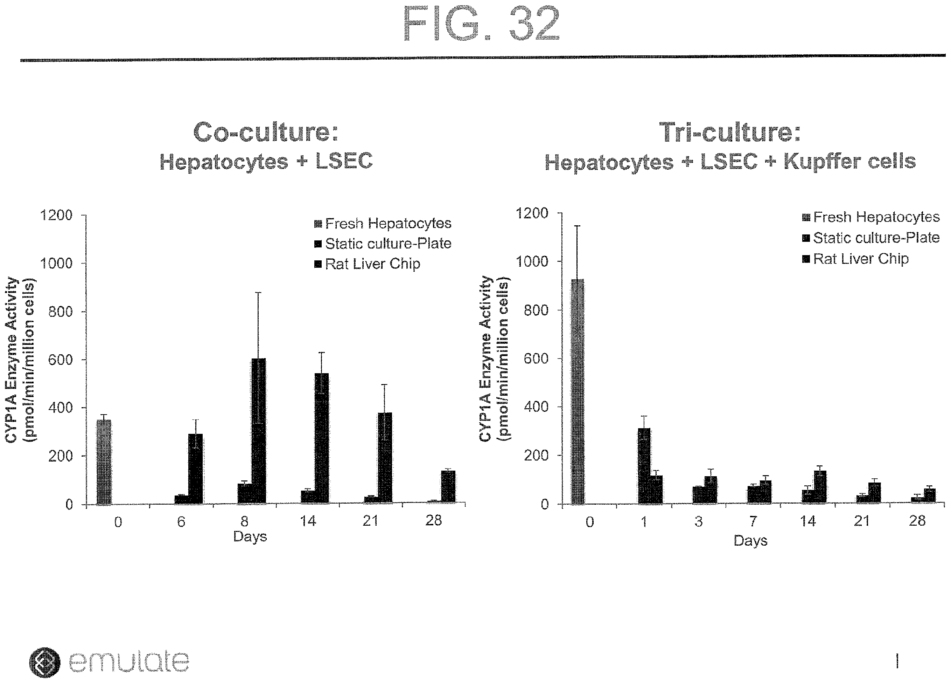

Figure 32 provides two graphs measuring CYP1A enzyme activity in a two cell arrangement (left) and a three cell arrangement (right) on a microfluidic "Rat Liver-on-chip," as compared to static culture plates.

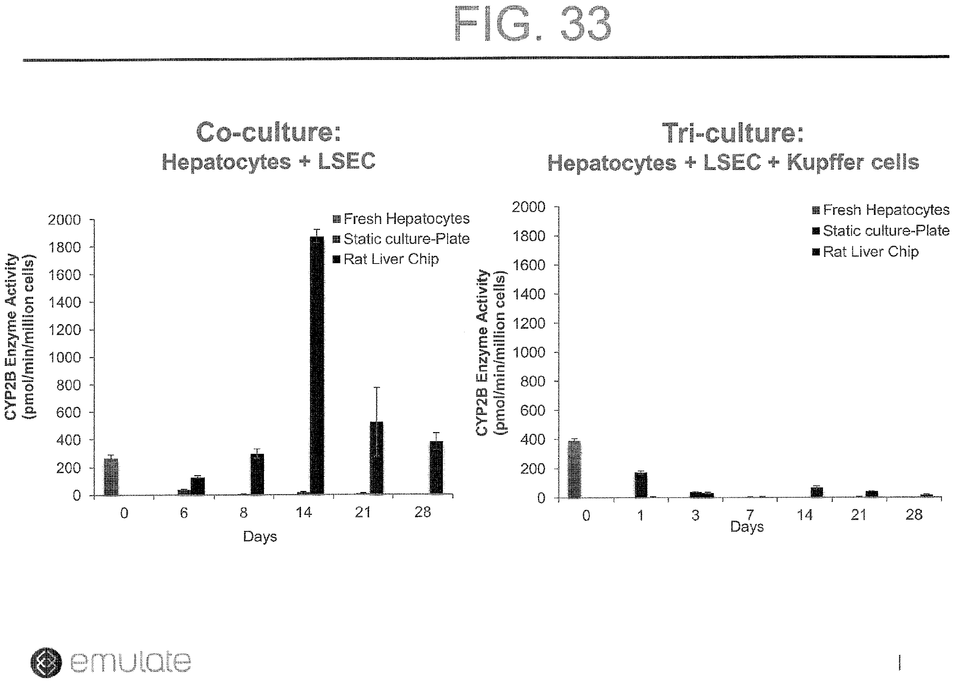

Figure 33 provides two graphs measuring CYP2B enzyme activity in a two cell arrangement (left) and a three cell arrangement (right) on a microfluidic "Rat Liver-on-chip," as compared to static culture plates.

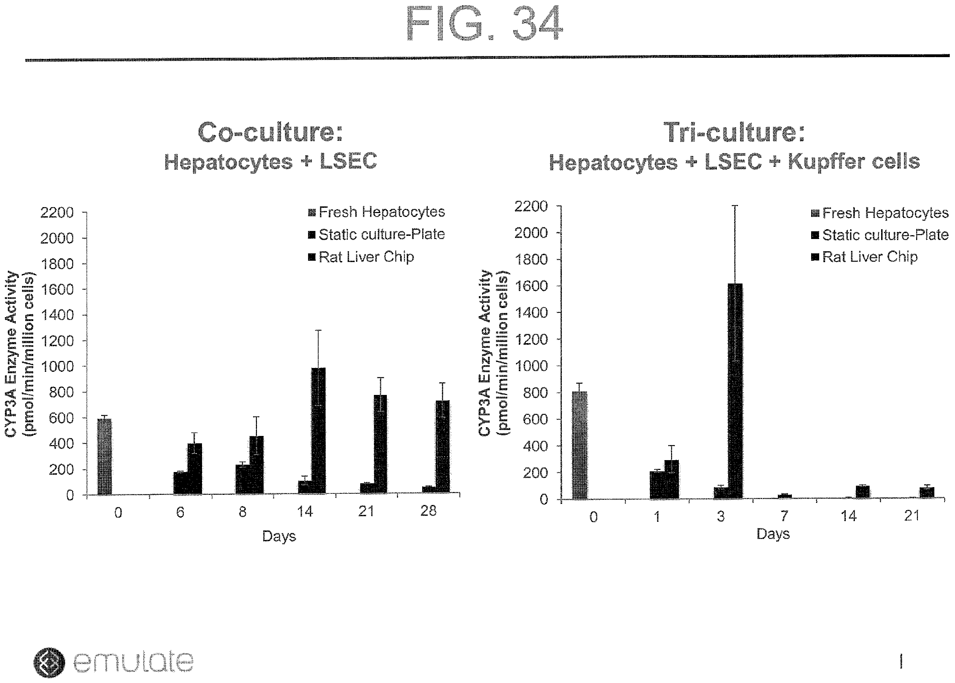

Figure 34 provides two graphs measuring CYP3A enzyme activity in a two cell arrangement (left) and a three cell arrangement (right) on a microfluidic "Rat Liver-on-chip," as compared to static culture plates.

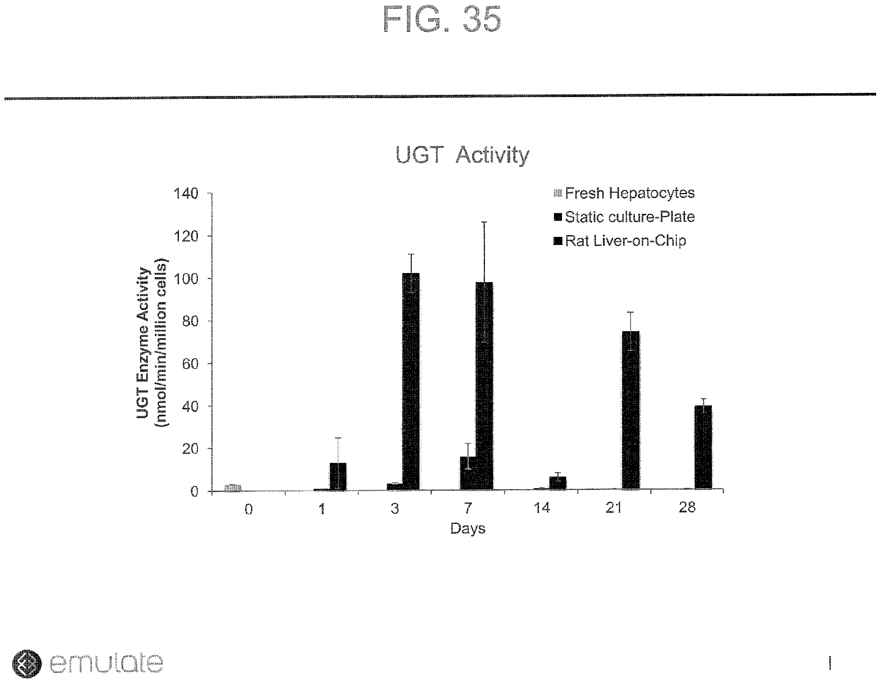

Figure 35 provides a graph measuring UGT enzyme activity among fresh hepatocytes, hepatocytes on a static culture plate, and hepatocytes on a "Rat Liver-on-Chip" embodiment.

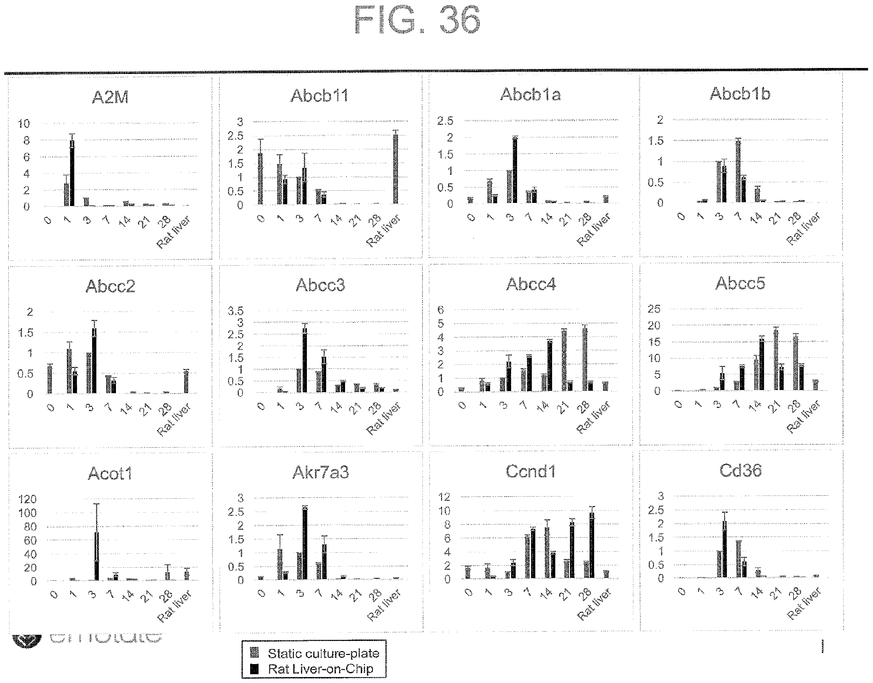

Figure 36 provides twelve graphs measuring relative protein expression comparing hepatocytes on a static culture plate and hepatocytes on a "Rat Liver-on-Chip" embodiment, with each graph related to a different protein.

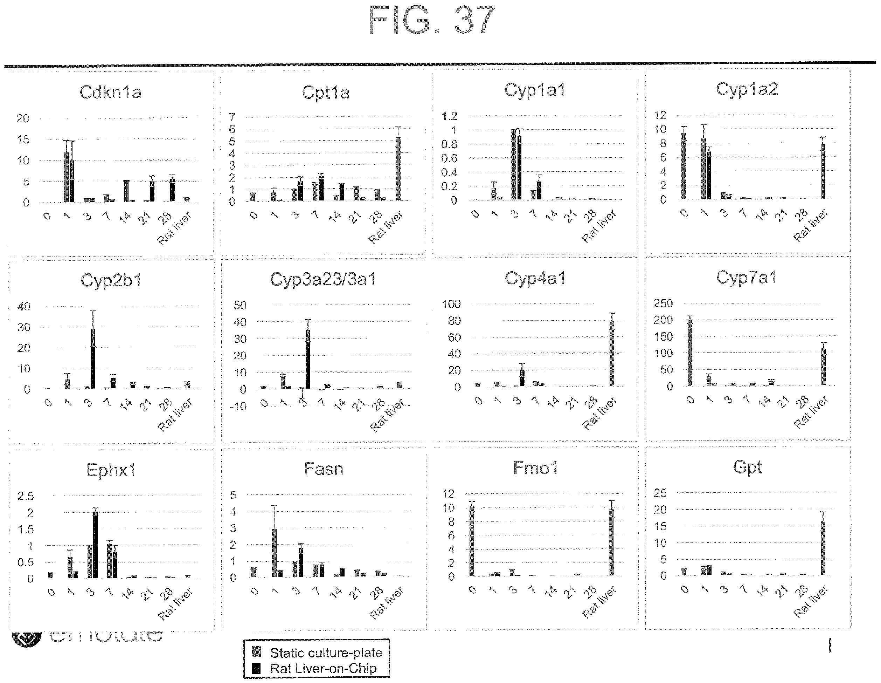

Figure 37 provides twelve additional graphs measuring relative protein expression comparing hepatocytes on a static culture plate and hepatocytes on a "Rat Liver-on-Chip" embodiment, with each graph related to a different protein.

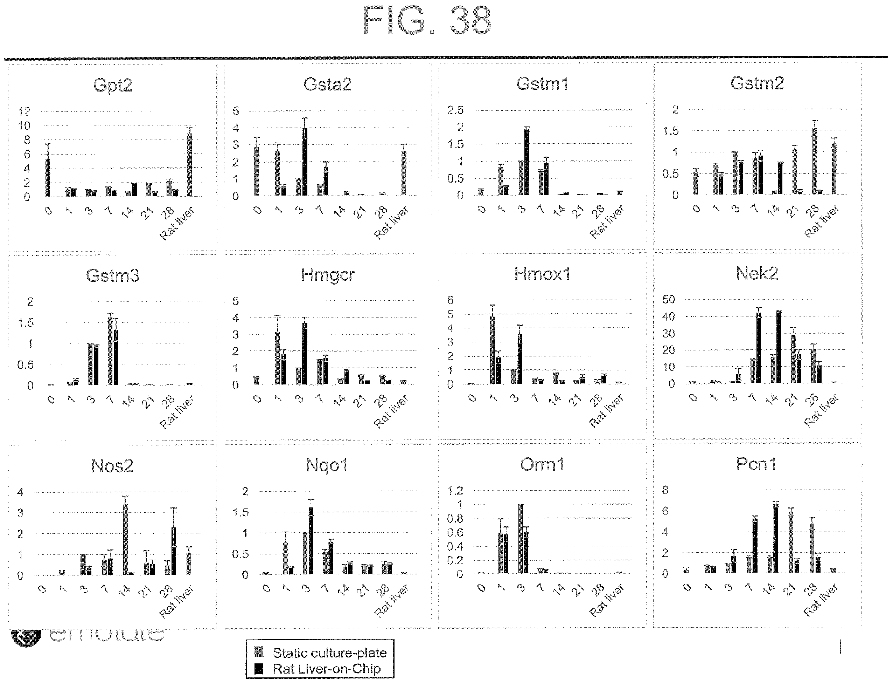

Figure 38 provides yet an additional twelve graphs measuring relative protein expression comparing hepatocytes on a static culture plate and hepatocytes on a "Rat Liver-on-Chip" embodiment, with each graph related to a different protein.

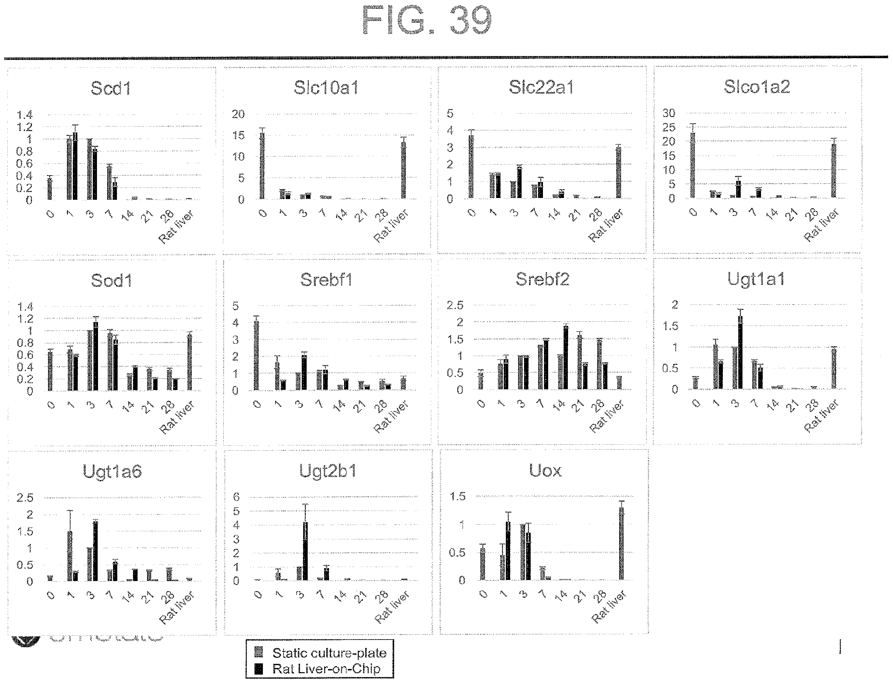

Figure 39 provides an additional eleven graphs measuring relative protein expression comparing hepatocytes on a static culture plate and hepatocytes on a "Rat Liver-on-Chip" embodiment, with each graph related to a different protein.

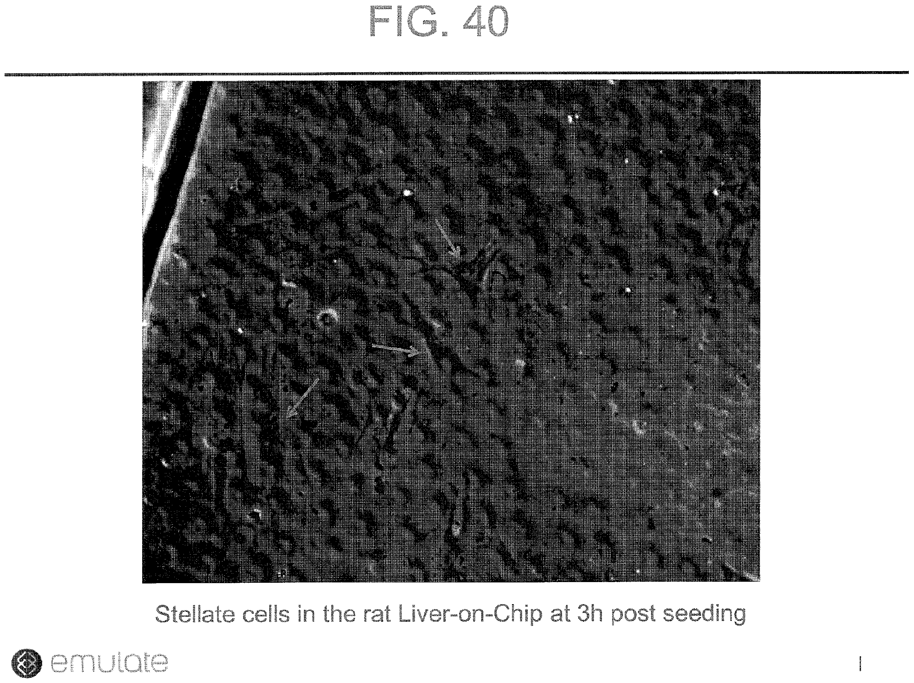

Figure 40 is a photograph showing the morphology of rat stellate cells by microscopic examination.
Figure 41 provides three photographs of hepatocyte morphology comparing a two cell system (left), a three cell system (middle) and a four cell system (right) on a microfluidic "Rat Liver -on-chip".

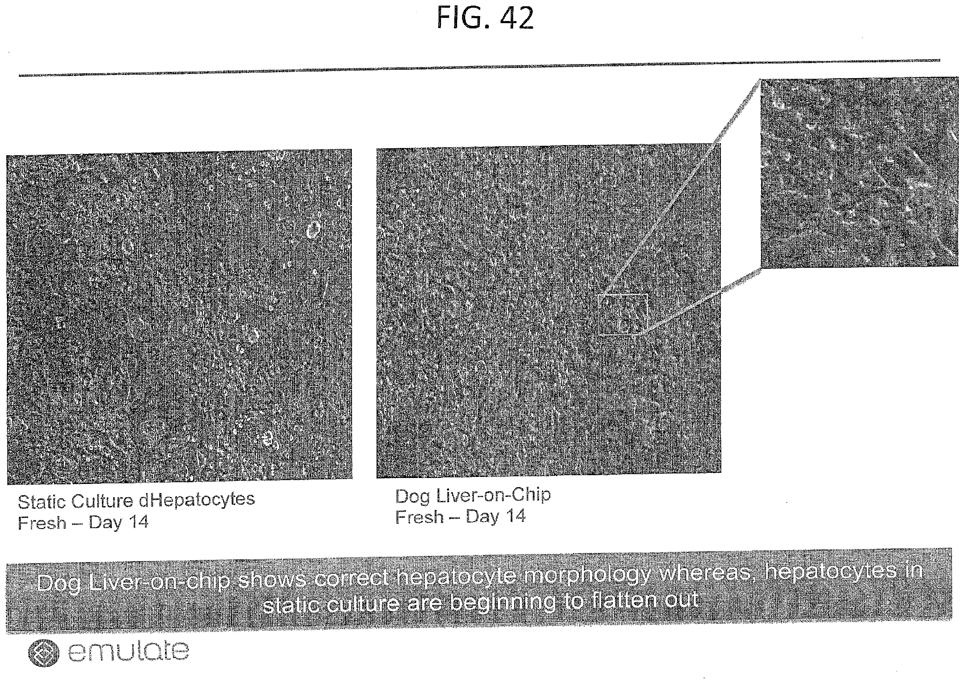

Figure 42 provides two photographs of dog hepatocyte morphology comparing static cultures with a microfluidic "Dog Liver-on-chip" embodiment at day 14.

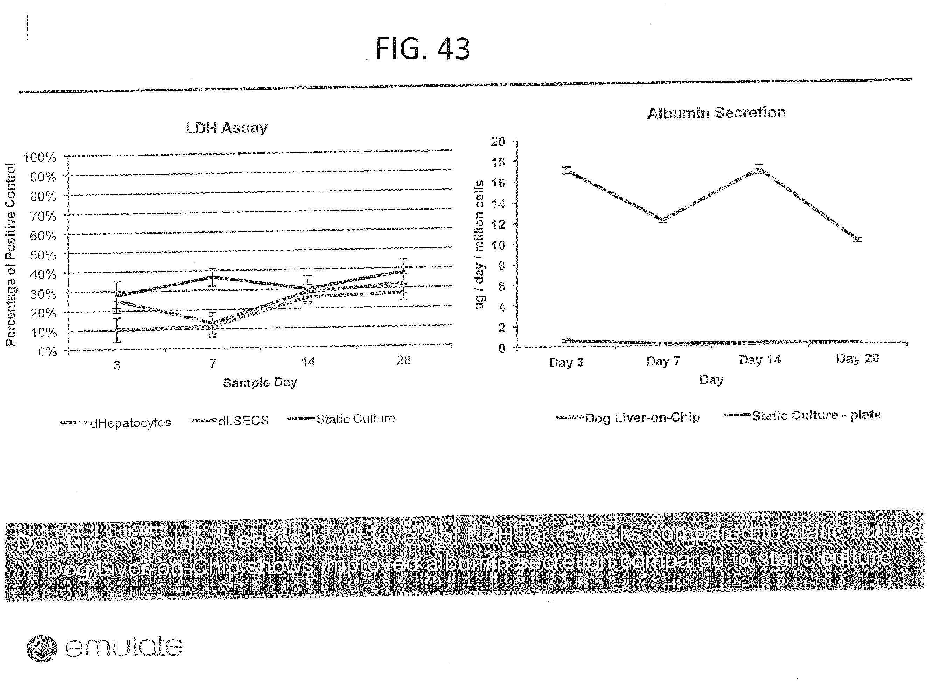

Figure 43 provides two graphs, one measuring LDH release over a 28 day period (left) and albumin secretion over a 28 day period (right) on a microfluidic "Dog Liver-on-chip," as compared to static culture plates.

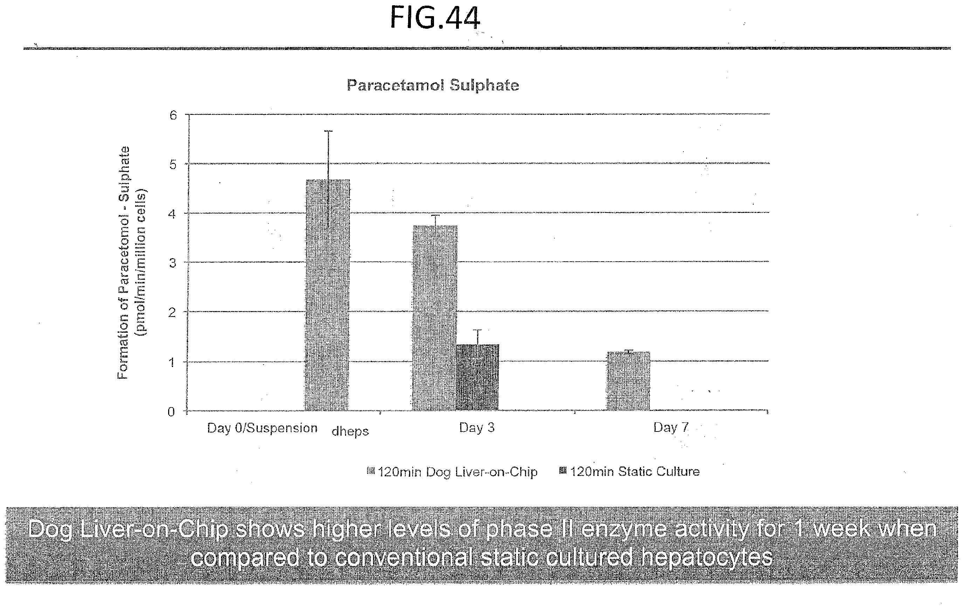

Figure 44 provides a graph measuring CYP enzyme activity on a microfluidic "Dog Liver-on-chip," as compared to static culture plates.

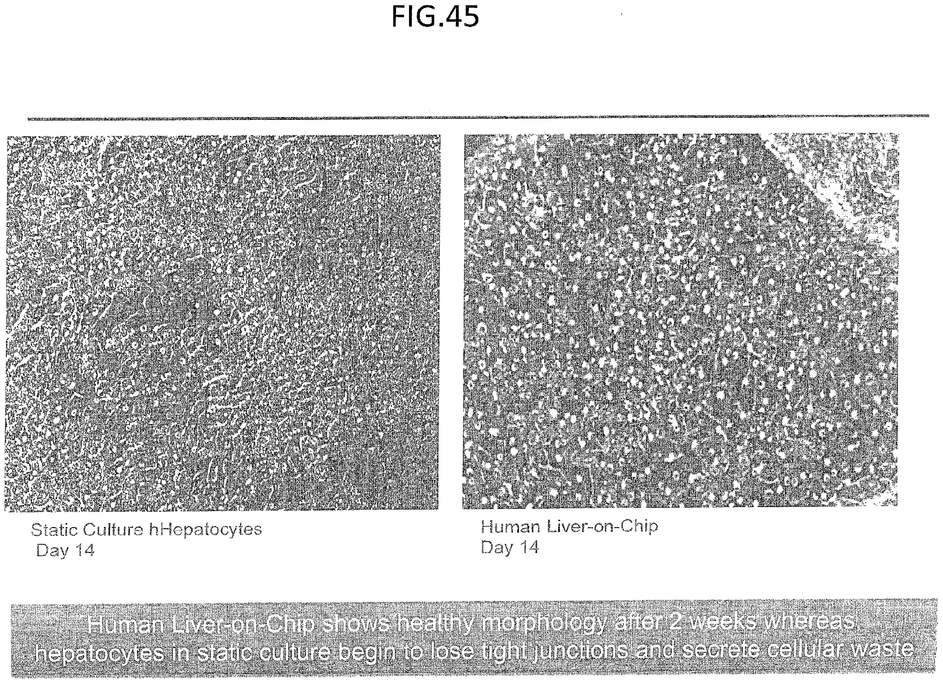

Figure 45 provides two photographs of human hepatocyte morphology comparing static cultures with a microfluidic "Human Liver-on-chip" embodiment at day 14.

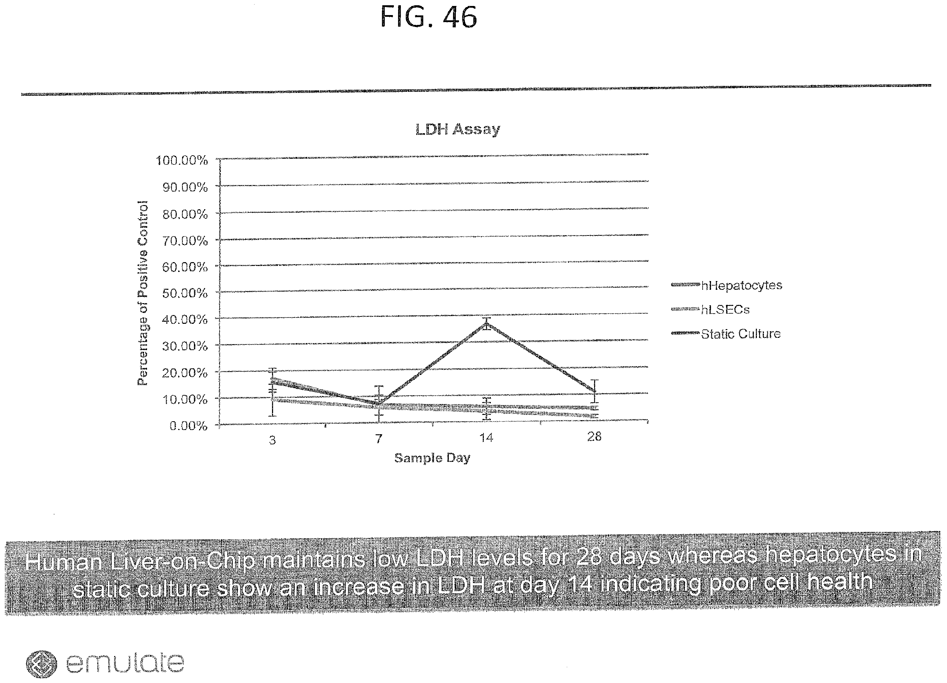

Figure 46 provides a graph measuring LDH release over a period of 28 days by human hepatocytes on a microfluidic "Rat Liver-on-chip," as compared to static culture plates.

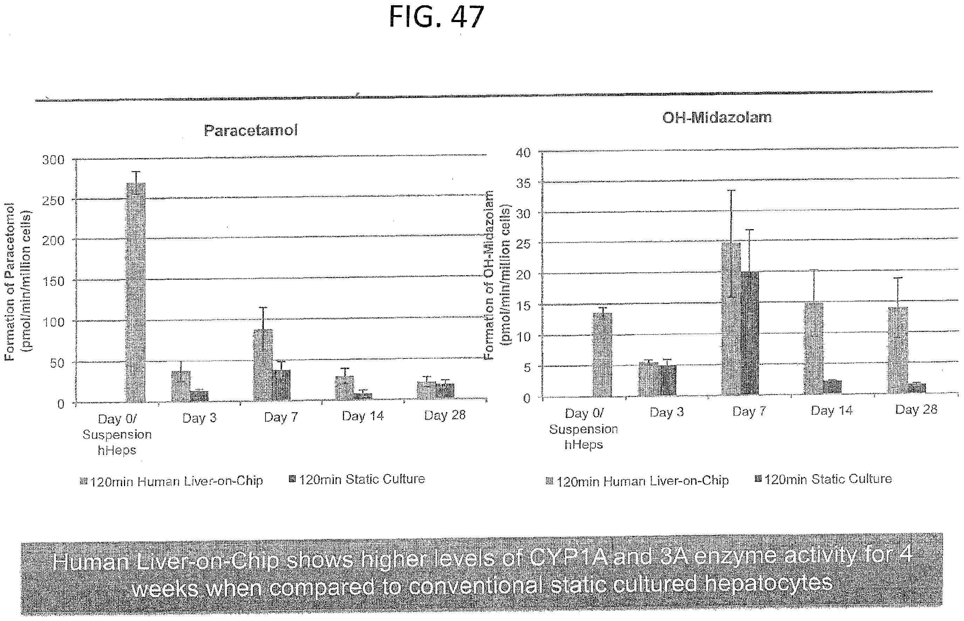

Figure 47 provides two graphs measuring CYP1A enzyme activity (left) and CYP3A enzyme activity (right) over a 28 day period on a microfluidic "Human Liver-on-chip," as compared to static culture plates.

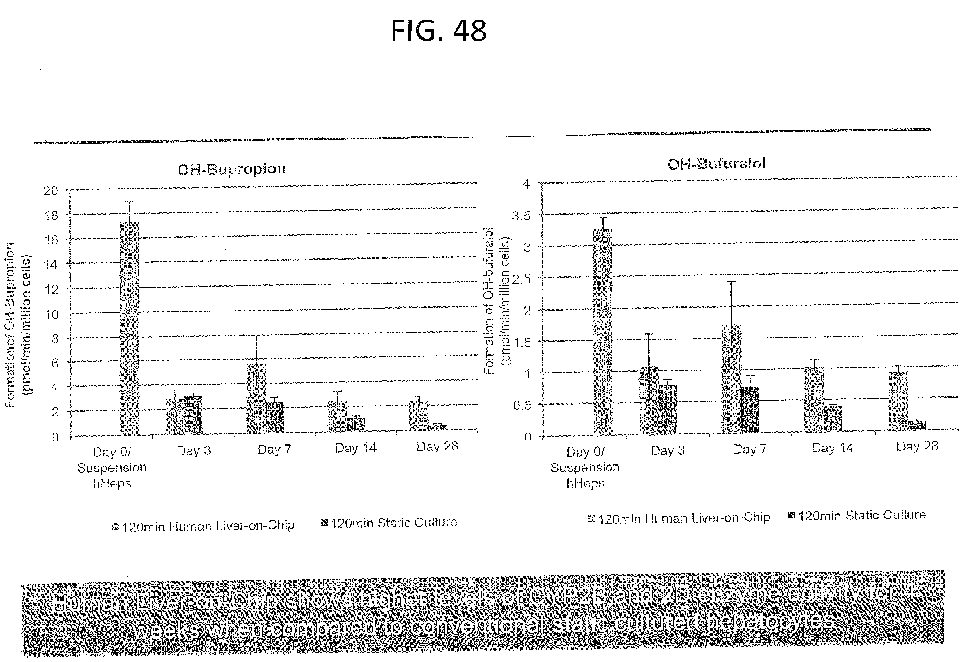

Figure 48 provides two graphs measuring CYP2B enzyme activity (left) and CYP2D enzyme activity (right) over a 28 day period on a microfluidic "Human Liver-on-chip," as compared to static culture plates.

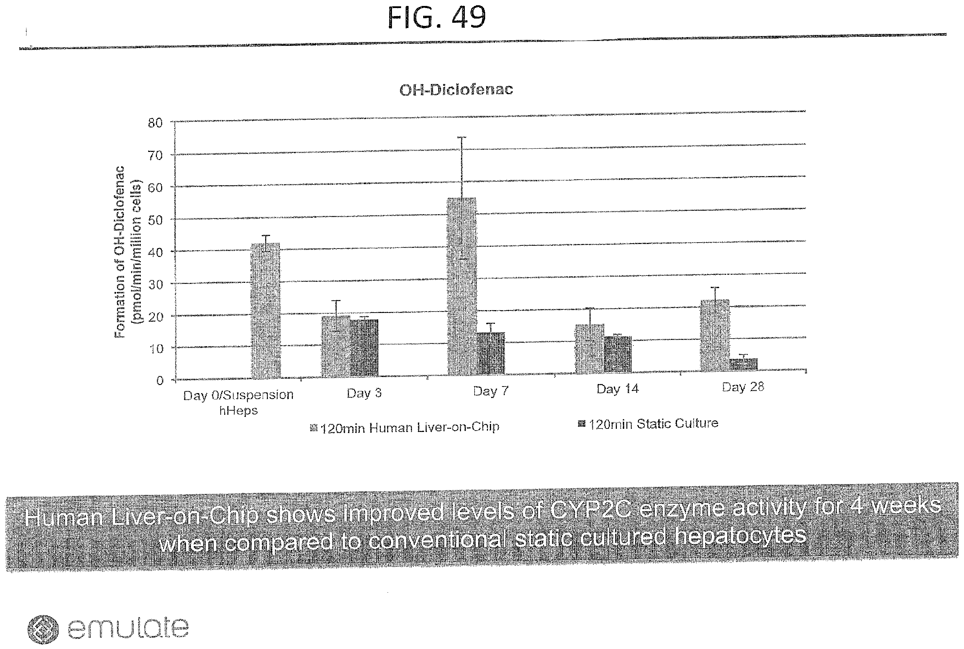

Figure 49 provides a graph measuring CYP2C enzyme activity over a 28-day period on a microfluidic "Human Liver-on-chip," as compared to static culture plates.
Figure 50 provides a graph measuring CYP1A1 Enzyme Activity in three different types of microfluidic "Rat Liver-on-Chip" as compared to static conventional sandwich plate cultures over time (0-14 days) and to a freshly thawed hepatocyte suspension (Day 0).
Figure 51 provides a graph measuring CYP2B1 Enzyme Activity in three different types of microfluidic Rat Liver-on-Chip as compared to static conventional sandwich plate culture over time (0-14 days) and to a freshly thawed hepatocyte suspension (Day 0).
Figure 52 provides a graph measuring CYP3A1 Enzyme Activity in three different types of microfluidic Rat Liver-on-Chip as compared to static conventional sandwich plate culture over time (0-14 days) and to a freshly thawed hepatocyte suspension (Day 0).
Figure 53 provides a graph measuring CYP1A2 Enzyme Activity in a microfluidic Dog Liver-on-Chip as compared to static conventional sandwich plate culture (0-14 days) and to a freshly isolated hepatocyte suspension (Day 0).
Figure 54 provides a graph measuring CYP1A2 Gene Expression in a microfluidic Dog Liver-on-Chip as compared to static conventional sandwich plate culture over time (0-14 days) and to a freshly isolated hepatocyte suspension (Day 0).
Figure 55 provides a graph measuring CYP2B11 Enzyme Activity in a microfluidic Dog Liver-on-Chip as compared to static conventional sandwich plate culture over time (0-14 days) and to a freshly isolated hepatocyte suspension (Day 0).
Figure 56 provides a graph measuring CYP2B11 Gene Expression in a microfluidic Dog Liver-on-Chip as compared to static conventional sandwich plate culture over time (0-14 days) and to a freshly isolated hepatocyte suspension (Day 0).
Figure 57 provides a graph measuring CYP3A12 Enzyme Activity in a microfluidic Dog Liver-on-Chip as compared to static conventional sandwich plate culture over time (0-14 days) and to a freshly isolated hepatocyte suspension (Day 0).
Figure 58 provides a graph measuring liver-specific function: albumin secretion in a microfluidic Human Liver-on-Chip Function as compared to static conventional sandwich plate culture over time (3-28 days).
Figure 59 provides a graph measuring liver specific function: urea secretion in a microfluidic Human Liver-on-Chip Function as compared to static conventional sandwich plate culture over time (3-28 days).
Figure 60 provides a graph measuring CYP1A2 Enzyme Activity in a microfluidic Human Liver-on-Chip as compared to static conventional sandwich plate culture over time (0-14 days) and to a freshly thawed hepatocyte suspension (Day 0).
Figure 61 provides an exemplary schematic image of 3D interaction between fluid flow in upper and lower chambers (see arrows above and below) in relation to an exemplary pathway for diffusion/active transportation from the fluid in the upper chamber through the cell layers to the lower chamber, see vertical arrow pointing down. In additional or other embodiments, there is an exemplary pathway for diffusion/active transportation from the fluid in the lower chamber through the cell layers into the upper chamber.
Figure 62 provides a graph measuring liver specific function: albumin secretion in two different types of microfluidic Rat Liver-on-Chip, tri and quad cultures, as compared to static conventional sandwich plate culture over time (1-2 weeks).
Figure 63 provides a graph measuring liver specific function: urea secretion in two different types of microfluidic Rat Liver-on-Chip, tri and quad cultures, as compared to static conventional sandwich plate culture over time (1-2 weeks).
Figure 64 provides a graph measuring CYP1A2 Enzyme Activity in a microfluidic Human Liver-on-Chip as compared to static conventional sandwich plate culture over time (0-14 days) and to a freshly thawed hepatocyte suspension (Day 0).
Figure 65 provides a graph measuring CYP2B6 Enzyme Activity in a microfluidic Human Liver-on-Chip as compared to static conventional sandwich plate culture over time (0-15 days) and to a freshly thawed hepatocyte suspension (Day 0).
Figure 66 provides a graph measuring CYP3A4 Enzyme Activity in a microfluidic Human Liver-on-Chip as compared to static conventional sandwich plate culture over time (0-15 days) and to a freshly thawed hepatocyte suspension (Day 0).
Figure 67 provides a graph measuring CYP2C9 Enzyme Activity in a microfluidic Human Liver-on-Chip as compared to static conventional sandwich plate culture over time (0-14 days) and to a freshly thawed hepatocyte suspension (Day 0).
Figure 68 provides a graph measuring CYP2D6 Enzyme Activity in a microfluidic Human Liver-on-Chip as compared to static conventional sandwich plate culture over time (0-15 days) and to a freshly thawed hepatocyte suspension (Day 0).
Figure 69 provides a graph measuring CYP3A4 Activity in three different types of microfluidic "Human Liver-on-Chip" as compared to static conventional sandwich plate culture and to a freshly thawed hepatocyte suspension (Day 0).
Figure 70 provides a graph measuring CYP3A4 mRNA Expression in three different types of microfluidic "Human Liver-on-Chip" after 10 days in culture, as compared to a freshly thawed hepatocyte suspension (Day 0).
Figure 71 provides a graph measuring CYP3A4 mRNA Expression in a microfluidic "Human Liver-on-Chip" as compared to static conventional sandwich plate culture over time (1-15 days) and to freshly thawed (cryo) hepatocyte suspension (Day 0).
Figure 72 provides a graph measuring CYP1A2 mRNA Expression in a microfluidic "Human Liver-on-Chip" as compared to static conventional sandwich plate culture over time (1-15 days) and to freshly thawed (cryo) hepatocyte suspension (Day 0).
Figure 73 provides a graph measuring CYP2B6 mRNA Expression in a microfluidic "Human Liver-on-Chip" as compared to static conventional sandwich plate culture over time (1-15 days) and to freshly thawed (cryo) hepatocyte suspension (Day 0).
Figure 74 provides an exemplary structure of Bosentan (Tracleer).
Figure 75 provides graphs measuring Bosentan Toxicity as a percentage of LDH Leakage in three different species of microfluidic chips: A) "RAT Liver-on-Chip" (7 days of treatment); B) microfluidic "DOG Liver-on-Chip" (7 days of treatment); and C) microfluidic "Human Liver-on-Chip" (3 days of treatment) increasing concentrations from 1.2 (1.3 for human)-100 µM. Samples were removed from the hepatocyte channel and the LSEC channel of the microfluidic chip with a sample from a plate sandwich culture shown for comparison.
Figure 76 provides graphs measuring Bosentan Toxicity as Albumin Secretion in three different species of microfluidic chips: A) "RAT Liver-on-Chip"; B) "DOG Liver-on-Chip"; increasing concentrations from 1.2-100 µM and C) "Human Liver-on-Chip" increasing concentrations from 0.4-100 µM. Samples were removed from the microfluidic chip with a sample from a plate sandwich culture shown for comparison.
Figure 77 provides a micrograph of a microfluidic "Human Liver-on-Chip" showing significant hepatocyte degradation as an exemplary Bosentan induced toxicity in a co-culture area of hepatocytes with liver sinusoidal endothelial cells (LSEC).
Figure 78 provides a micrograph of functional Rat Kupffer cells (after 4 days of incubation in a plate) with engulfed red particles: A) Kupffer cells alone showing numerous red particles outside of cells; B) Kupffer cells and hepatocytes showing a few cells that arguably contain red particles, and C) Kupffer cells and liver sinusoidal endothelial cells (LSECs) (bar = 50 mico(µ)meters). Thick-short white arrows point to exemplary red particles outside of cell while thin-long arrows point to cells containing red particles.
Figure 79 provides a micrograph of functional Rat Kupffer cells having engulfed red particles in a culture of Kupffer cells with liver sinusoidal endothelial cells (LSECs) (after 4 days of incubation in a plate). Kupffer cells have migratory morphology, *i.e.* exemplary two elongated cells containing red particles, one cell in the lower half and one cell in the lower right area of the micrograph. Thin-long arrows point to cells containing red particles.
Figure 80 provides a micrograph of a microfluidic tri-culture "Rat Liver-on-Chip" (Hepatocytes + LSECs + Kupffer cells). A) Phase contrast micrograph. B) Fluorescent micrograph showing latex red fluorescent beads and DAPI (blue) nuclear staining.
Figure 81 provides a micrograph of a microfluidic tri-culture "Rat Liver-on-Chip" demonstrating a Kupffer cell marker CD68 staining. A) Phase contrast micrograph; and B) Fluorescent micrograph showing CD68 in red (see exemplary cell with a thin blue arrow) and DAPI (blue) nuclear staining.
Figure 82 provides a micrograph of isolated Rat Stellate Cells that were seeded within a microfluidic Quadruple-culture "Rat Liver-on-Chip". A) shows an exemplary culture before activation having DAPI (blue) nuclear staining with faint staining of a few Alpha-smooth muscle actin (α-SMA) fibers; and B) On Day 7, Stellate cells were activated by treatment with TGFβ-1 (1ng/ml) for 48 hours resulting in (strong) expression of α-SMA (green) fibers. DAPI (blue) stained nuclei.
Figure 83 provides a graph measuring a low clearance compound Antipyrine in a Clearance assay in microfluidic "Human Liver-on-Chip" under continuous flow.
Figure 84 provides a graph measuring low clearance compound Antipyrine in a Clearance assay in microfluidic "Human Liver-on-Chip" under non-dynamic conditions.
Figure 85 provides an exemplary schematic of an open top microfluidic chip.

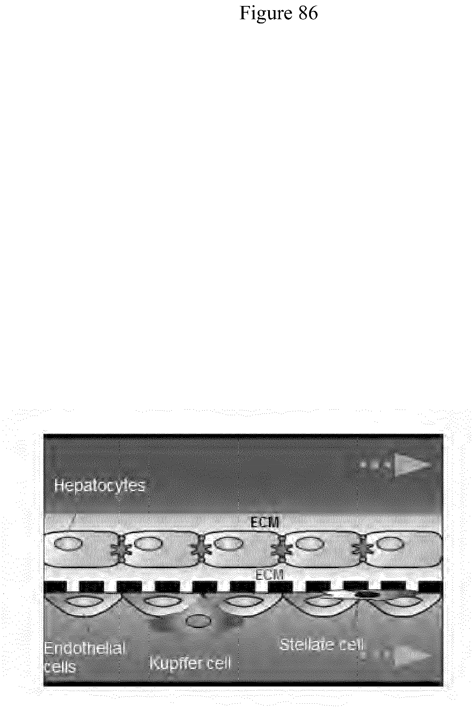

Figure 86 provides an exemplary schematic of a microfluidic "Liver-on-Chip" as an Open Top Chip (3D Liver-on-chip) showing Quadruple "Liver-on-chip" Architecture.

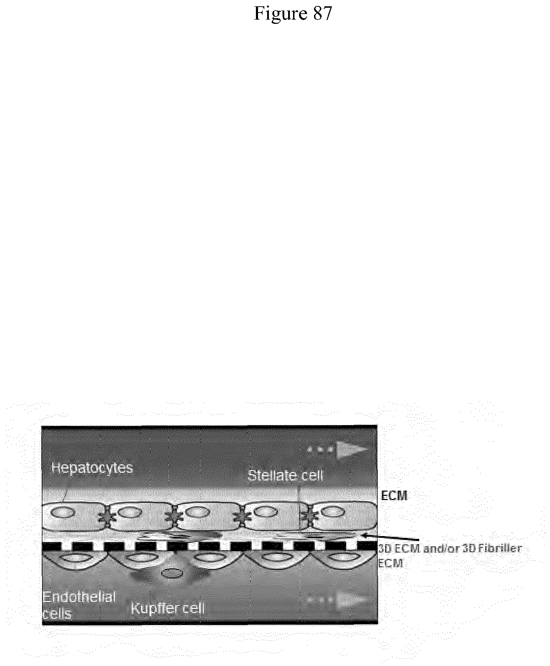

Figure 87 provides an exemplary schematic of a microfluidic "Liver-on-Chip" as an Open Top Chip (3D Liver-on-chip) Architecture including 3D Fibrilar ECM (see arrow pointing to thready material in between Stellate cells).
Figure 88 provides a graph measuring mRNA shows alanine aminotransferase (ALT), aspartate aminotransferase (AST) and Glutamate dehydrogenase (GLDH) (as fold changes over changes in compound concentration) in response to a test compound X added to a microfluidic "Human Liver-on-Chip."
Figure 89 provides exemplary micrographs showing results from hepatocyte cells seeding density experiments for providing Hepatocyte Seeding Guidelines in the microfluidic "Human Liver-on-Chips." Seeding density in a tall channel chip at 3 million cells/ml, in 40 µl. Phase contrast micrographs: A) immediately after seeding hepatocytes onto chips, B) 24 hours after seeding and C) 4 days after seeding hepatocyte cells.
Figure 90 provides exemplary micrographs showing results from hepatocyte cells seeding density experiments showing underseeded microfluidic "Human Liver-on-Chips." Seeding density in a tall channel chip at 2 million cells/ml, in 40 µl. Phase contrast micrographs: A) immediately after seeding hepatocytes onto chips, B) 24 hours after seeding and C) 4 days after seeding hepatocyte cells.
Figure 91 provides exemplary micrographs showing results from hepatocyte cells seeding density experiments for showing overseeded microfluidic "Human Liver-on-Chips." Seeding density in a tall channel chip at 3.5 million cells/ml, 40 µl. Phase contrast micrographs: A) immediately after seeding hepatocytes onto chips, B) 24 hours after seeding and C) 4 days after seeding hepatocyte cells.

### GENERAL DESCRIPTION OF THE INVENTION

Conventional in vitro hepatic model systems, e.g., 2-dimensional monolayers of primary hepatocytes, are limited by their inability to maintain normal phenotypic characteristics over time in culture, including stable expression of clearance and metabolic pathways (Phase I and Phase II metabolism). In addition, one of the main drawbacks of the existing cell culture system is its limitation in maintaining a long-term culture system. Typically, cells in long-term culture (7-14 days) generally exhibit a deteriorating monolayer, cell dysfunction, and/or cell death.

The present invention contemplates an in vitro liver-on-chip design that mimics the structure and function of the liver in vivo. Importantly, extended viability with sustained function over long-term culture (14-28 days) time is demonstrated based on enzymatic activity, allowing for drug testing, including pharmacokinetics, drug clearance, drug sensitivity, and drug toxicity.

Microfluidic chips based on human, dog and rat liver cells are exemplified. With regard to human liver cells, Human Liver-on-Chip shows improved morphology and viability (LDH) compared to static cultured hepatocytes during the 28 day time course. Human Liver-on-Chip shows improved enzyme activity levels of CYP1A, 2B, 2D, 2C and 3A for 4 weeks compared to static cultured hepatocytes. With regard to dog liver cells, fresh dog hepatocytes on the Dog Liver-on-chip showed improved morphology and viability (LDH/albumin) as compared to static cultured hepatocytes during 28 day time course. Dog Liver-on-Chip shows improved enzyme activity levels of CYP1A, 2B, and 3A for 2 weeks compared to static cultured dog hepatocytes.

Dog Liver-on-Chip shows higher phase II enzyme activity for 1 week compared to static cultured dog hepatocytes. Finally, with regard to rat liver cells, Rat Liver-on-chip displayed healthy cell morphology during a 4 week time course. Rat Liver-on-Chip revealed improved expression and localization of efflux transporter (MRP2) compared to conventional static culture rat hepatocytes. Rat Liver-on-Chip maintained low levels of LDH release and high levels of albumin secretion during 4 week timecourse, indicating viable and functioning cells. Rat Liver-on-Chip maintained enzyme activity for CYP1A, 2B, 2D and 3A during the 4 week time course, at robust levels compared to conventional static cultured rat hepatocytes. Rat Liver-on-Chip showed improved phase II enzyme activity compared to conventional static cultured rat hepatocytes.

### DETAILED DESCRIPTION OF THE INVENTION

Teachings described herein stem from, at least in part, design of devices that allow for a controlled and physiologically realistic co-culture of liver sinusoidal endothelial cells in one chamber with hepatocytes in other chamber(s) to establish hepatic function *in vitro.* In one embodiment, the chambers are aligned (e.g., vertically) with each other with one or more membranes separating them from each other ("liver-on-a-chip"). The liver-on-a-chip devices have been developed and optimized based on the basic design of an organ-on-a-chip as described in the U.S. Patent No. 8,647,861, and the International Patent App. No. PCT/US2014/071611. In some teachings, the inventors have optimized the design of the liver-on-a chip devices and culture conditions to provide long-term hepatic culture with physiologically relevant hepatic function (e.g., albumin and/or urea secretion, and/or CYP 450 metabolic capacity) for different animal models, e.g., human, rats, and dogs. Further, in some teachings (not part of the claimed invention), the inventors have formulated a universal endothelial cell culture medium for long-term culture, which performs superior to the existing commercially available media. The novel endothelial cell culture media developed by the inventors allow co-cultures of endothelial and epithelial cells of different tissue origins *(e.g.,* but not limited to lung, gut, kidney, and liver) to maintain functionality and viability for at least one month (e.g. 28 - 30 days) in a cell culture device, which is otherwise impossible using the commercial culture media. Accordingly, embodiments of various aspects described herein relate to devices for simulating a function of a tissue and methods of using the same. Novel endothelial cell culture media (not part of the claimed invention) for long-term culture and methods of using the same are also described herein.

Those of ordinary skill in the art will realize that the following description is illustrative only and is not intended to be in any way limiting. Reference will now be made in detail to implementations of the example embodiments as illustrated in the accompanying drawings. The same reference indicators will be used throughout the drawings and the following description to refer to the same or like items. It is understood that the phrase "an embodiment" encompasses more than one embodiment and is thus not limited to only one embodiment for brevity's sake.

### Exemplary devices for simulating a function of a tissue

As used herein, the terms "extracellular matrix" and "ECM" refer to a natural or artificial composition of extracellular molecules generally secreted by cells that provide structural and/or biochemical support for self and/or surrounding cells. In some embodiments, the terms "extracellular matrix" and "ECM" can refer to a composition comprising a solubilized basement membrane extracted from a tissue. The composition and structure of ECMs can vary depending on the source of the tissue. For example, sarcoma, small intestine submucosa (SIS), urinary bladder matrix (UBM) and liver stroma ECM each differ in their overall structure and composition due to the unique cellular niche needed for each tissue. Examples of ECM molecules include, but are not limited to, collagen, fibronectin, laminin, vitronectin, tenascin, entactin, thrombospondin, elastin, gelatin, fibrillin, merosin, anchorin, chondronectin, link protein, bone sialoprotein, osteocalcin, osteopontin, epinectin, hyaluronectin, undulin, epiligrin, and Kalinin, glycosaminoglycans, proteoglycans, chemoattractants, cytokines, growth factors, and any combinations thereof. Examples of commercially available extracellular matrices that can be used to prepare the ECM coating layer and/or ECM gel layer include, for example, but not limited to an ECM gel by Sigma-Aldrich or Matrigel^{™} Matrix by Becton, Dickinson & Company. Matrigel^{™} matrix is a reconstituted basement membrane isolated from mouse Engelbreth-Holm-Swarm (EHS) tumor. Matrigel^{™} matrix comprises predominantly laminin, collagen IV, entactin and heparan sulfate proteoglycans. Matrigel^{™} matrix can also comprise a number of growth factors, including, e.g., but are not limited to EGF, IGF-1, PDGF, TGF-β, VEGF, bFGF, and any combinations thereof. In some embodiments, the terms "extracellular matrix" and "ECM" also encompass a purified composition comprising a specific type of ECM molecules.

As used herein, the term "ECM coating layer" refers to a layer comprising at least one type or a mixture of extracellular matrix (ECM) molecules adsorbed or bound or coupled to a surface, *e.g.,* a membrane surface. The ECM coating layer can be formed on a membrane surface by depositing a solution of an ECM coating solution on the surface and allowing the ECM molecules to adsorb or bound to the surface, and/or patterning or "stamping" ECM molecules directly onto the surface, *e.g.,* in a specific pattern or arrangement.

As used herein, the term "ECM gel layer" refers to a gel layer (and more typically, a very thin coating of gel) comprising at least one type or a mixture of extracellular matrix molecules. The ECM gel layer or coating can be placed on top of the ECM coating layer by depositing an ECM gel solution on top of the ECM coating layer and allowing the ECM gel solution to form a gel layer.

The composition and concentration(s) of extracellular matrix component(s) in the ECM coating solution and/or ECM gel solution can vary with and be optimized for cells of different tissue types and/or of different species (*e.g.*, human cells vs. rat cells or dog cells) using methods known in the art. For example, as shown in the Example 3, the inventors cultured rat hepatocytes on surfaces treated with different compositions of an ECM coating layer and/or ECM gel layer, and then monitored cell morphology and/or growth to determine an optimal ECM coating and/or ECM gel composition that allows for consistent performance of the cell culture surface in maintaining viability and function of the hepatocytes. The inventors have determined that an ECM coating of Matrigel (250 ug/mL) provides good results for liver cells on chips.

In some embodiments, the total extracellular matrix components can be applied to a membrane surface (*e.g*., the first side of the membrane) in a concentration of between about 0.05 mg/mL to about 5 mg/mL. In some embodiments, the total concentration of the extracellular matrix components applied to the membrane surface (*e.g*., the first side of the membrane) can range from 0.1 mg/mL to about 5 mg/mL. In some embodiments, the total concentration of the extracellular matrix components applied to the membrane surface (*e.g*., the first side of the membrane) can range from 1 mg/mL to about 3 mg/mL.

The inventors have also determined that an ECM gel coating or layer formed from an ECM gel solution containing no fibronectin, and about 250 mg/mL of basement membrane-derived proteins (*e.g*., Matrigel^{™}) provided an optimal condition for cell attachment/growth of rat hepatocytes on a membrane surface.

Accordingly, in some embodiments, the ECM gel layer can comprise, essentially consist of, or consist of a protein mixture gel derived from a basement membrane. In one embodiment, the protein mixture gel derived from a basement membrane comprises Matrigel^{™}.

In some embodiments of various teachings described herein, the device can further comprise a monolayer, or a multi-layered or three-dimensional structure, comprising at least one type of tissue-specific cells adhered on the extracellular matrix composition. Appropriate tissue-specific cells can be selected depending on the organization and/or function of a tissue to be modeled. For example, tissue-specific cells are generally parenchymal cells (*e.g.,* epithelial cells) derived from a tissue or an organ including, *e.g*., but not limited to, a liver, a lung, a kidney, a skin, an eye, a brain, a blood-brain-barrier, a heart, a gastrointestinal tract, airways, a reproductive organ, and a combination of two or more thereof.

The viable hepatocytes used on connection with the claimed invention can be derived from different mammalian sources, including, *e.g*., but not limited to humans, rats, mice, and dogs. Thus, the devices can be used to simulate a function of a liver tissue in different animal model of interest.

As used herein, the term "monolayer" refers to a single layer of cells on a growth surface, on which no more than 10% (*e.g.,* 10%, 9%, 8%, 7%, 6%, 5%, 4%, 3%, 2%, 1%, or 0%) of the cells are growing on top of one another, and at least about 90% or more (*e.g.,* at least about 95%, at least 98%, at least 99%, and up to 100%) of the cells are growing on the same growth surface. In some embodiments, all of the cells are growing side-by side, and can be touching each other on the same growth surface. The condition of the cell monolayer can be assessed by any methods known in the art, *e.g*., microscopy, and/or immunostaining for cell-cell adhesion markers. In some embodiments where the cell monolayer comprises a hepatocyte monolayer, the condition of the hepatocyte monolayer can be assessed by presence of cuboidal cell morphology and/or staining for any art-recognized markers in hepatocytes (e.g. staining for bile caniculi, mrp2 transporter or CDFDA). In some embodiments where the cell monolayer comprises an endothelial cell monolayer, the condition of the endothelial cell monolayer can be assessed by staining for any art-recognized cell-cell adhesion markers in endothelial cells, *e.g.,* but not limited to VE-cadherin.

In some embodiments of various aspects described herein, the device can further comprise a monolayer of endothelial cells adhered on the second side of the membrane. The endothelial cells can be derived from vascular endothelial cells and/or lymphatic endothelial cells. In some embodiments, the endothelial cells can be derived from the same tissue origin as the tissue-specific cells cultured in the first chamber.

### Exemplary liver-on-a-chip devices

The inventors have developed liver-on-a-chip devices based on the basic design of an organ-on-a-chip as described in the U.S. Patent No. 8,647,861, and the International Patent App. No. PCT/US2014/071611, and also optimized culture conditions to provide long-term hepatic culture with physiologically relevant hepatic function (*e.g*., albumin and/or urea secretion, and/or CYP 450 metabolic capacity) for different animal models, *e.g*., human, rats, and dogs.

Accordingly, some teachings described herein relate to devices for simulating a function of a liver tissue (also referred to as "liver-on-a-chip device"). The liver-on-a-chip devices described herein can be used to simulate at least one or more (*e.g.,* 1, 2, 3, 4, 5 or more) phenotypes and/or functions of a liver tissue. For examples, in some embodiments, the liver-on-a-chip devices described herein can simulate at least one or more of the phenotypes and/or functions of a liver tissue selected from the group consisting of (i) cuboidal morphology; (ii) a substantially constant level of albumin secretion over a period of time (*e.g.,* at least 2 weeks, at least 4 weeks or longer); (iii) a substantially constant level of urea synthesis over a period of time (*e.g.,* at least 2 weeks, at least 4 weeks or longer); (iv) at least a 2-fold increase (including, *e.g.,* at least 3-fold increase or higher) in activity of CYP450 drug metabolizing enzyme in the presence of an agent that induces CYP450 drug metabolizing enzyme; (v) formation of a bile canaliculus network and active transport by multidrug resistance-associated protein 2 (MRP2), and a combination of two or more thereof.

In one teaching, the liver-on-a-chip device comprises (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber. The first side comprises an extracellular matrix composition and hepatocytes adhered on the extracellular matrix composition. Matrigel is the preferred ECM. In the case of the human liver cells, collagen I may be used.

In some embodiments of this teaching and other teachings described herein, the second side of the membrane can comprise a monolayer of endothelial cells adhered thereon. In some embodiments, the monolayer of endothelial cells can comprise a monolayer of liver sinusoidal endothelial cells adhered thereon. Sinusoidal endothelial cells are generally derived from sinusoidal blood vessels with fenestrated, discontinuous endothelium, and can be identified by the presence of at least one or more markers, including, *e.g.*, but not limited to CD31 and CD32. CD32b.

The inventors have also successfully created a co-culture microfluidic device to simulate a function of a dog liver tissue. Accordingly, a further teaching described herein relates to a device for simulating a function of a dog liver tissue. The device comprises: (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber. The first side of the membrane has dog hepatocytes adhered thereon and the second side has dog liver sinusoidal endothelial cells adhered thereon.

In some embodiments, the first side of the membrane can comprise an extracellular matrix composition. In some embodiments, the extracellular matrix can comprise an ECM coating of collagen I (approx.. 100-200 ug/mL). In others, it comprises a Matrigel overlay (250ug/mL).

In some embodiments of various aspects described herein, the devices can comprise a flowing culture medium in the first chamber and/or the second chamber, wherein the flowing culture medium generates a shear stress. The inventors have discovered that addition of low shear stress improved liver cell morphology and long-term viability over static cultures.

Based on the viscosity of the culture medium and/or dimensions of the chambers, one of skill in the art can determine appropriate flow rates of culture medium through the chambers to achieve desired shear stress. In some embodiments, the flow rate of the culture medium through the first chamber can range from about 5 µL/hr to about 50 µL/hr. In some embodiments, the flow rate of the culture medium through the second chamber can range from about 15 µL/hr to about 150 µL/hr.

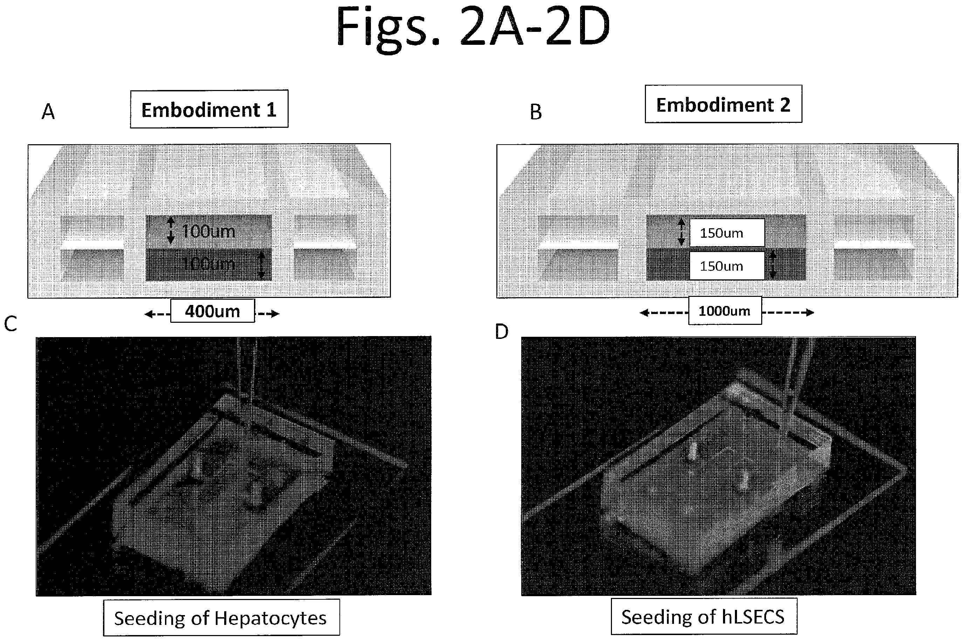

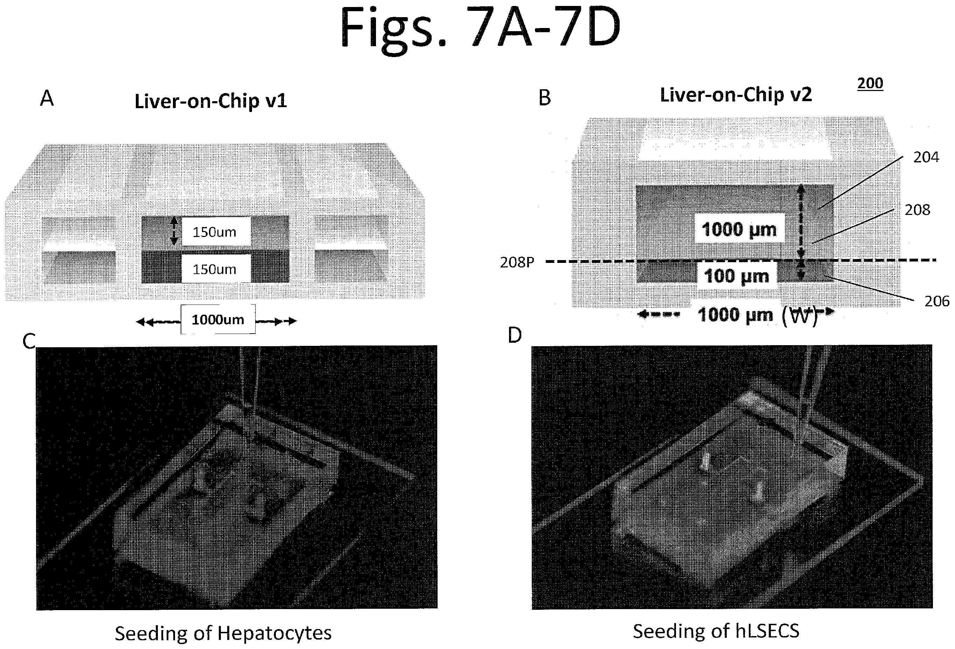

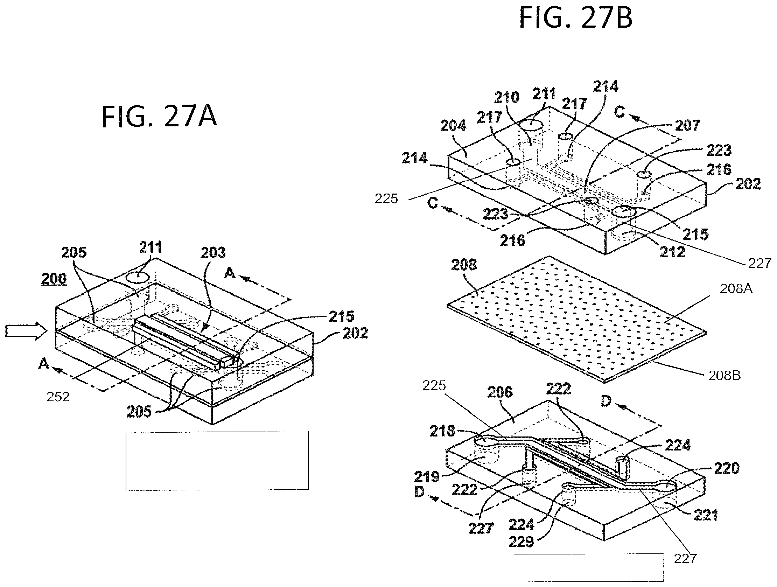

***Chamber-defining first structure and second structure:*** In accordance with various teachings of certain embodiments described herein, the first structure defines a first chamber, and the second structure defines a second chamber. While the first chamber and the second chamber can be in any geometry or three-dimensional structure, in some embodiments, the first chamber and the second chamber can be configured to be form channels. **Figs. 2A-2B**, **7A-7B**, and **27A-27B** illustrate a perspective view of the devices in accordance with some embodiments described herein. For example, as shown in **Figs. 27A-****27B,** the device 200 can include a body 202 comprising a first structure 204 and a second structure 206 in accordance with an embodiment. The body 202 can be made of an elastomeric material, although the body can be alternatively made of a non-elastomeric material, or a combination of elastomeric and non-elastomeric materials. It should be noted that the microchannel design 203 is only exemplary and not limited to the configuration shown in **Figs. 27A-27B****.** While operating chambers 252 (*e.g.,* as a pneumatics means to actuate the membrane 208, see the International Appl. No. PCT/US2009/050830 for further details of the operating chambers are shown in **Figs. 27A-27B****,** they are not required in all of the embodiments described herein. In some embodiments, the devices do not comprise operating chambers on either side of the first chamber and the second chamber. For example, **Fig. 7B** shows a device that does not have an operating channel on either side of the first chamber and the second chamber. In other embodiments, the devices described herein can be configured to provide other means to actuate the membrane, *e.g*., as described in the International Pat. Appl. No. PCT/US2014/071570.

In some embodiments, various organ chip devices described in the International Patent Application Nos. PCT/US2009/050830; PCT/US2012/026934; PCT/US2012/068725; PCT/US2012/068766; PCT/US2014/071611; and PCT/US2014/071570, can be modified to form the devices described herein. For example, the organ chip devices described in those patent applications can be modified in accordance with the devices described herein.

The first structure 204 and/or second structure 206 can be fabricated from a rigid material, an elastomeric material, or a combination thereof. As used herein, the term "rigid" refers to a material that is stiff and does not bend easily, or maintains very close to its original form after pressure has been applied to it. The term "elastomeric" as used herein refers to a material or a composite material that is not rigid as defined herein. An elastomeric material is generally moldable and curable, and has an elastic property that enables the material to at least partially deform (*e.g*., stretching, expanding, contracting, retracting, compressing, twisting, and/or bending) when subjected to a mechanical force or pressure and partially or completely resume its original form or position in the absence of the mechanical force or pressure. In some embodiments, the term "elastomeric" can also refer to a material that is flexible/stretchable but does not resume its original form or position after pressure has been applied to it and removed thereafter. The terms "elastomeric" and "flexible" are interchangeably used herein.

In some embodiments, the material used to make the first structure and/or second structure or at least the portion of the first structure 204 and/or second structure 206 that is in contact with a gaseous and/or liquid fluid can comprise a biocompatible polymer or polymer blend, including but not limited to, polydimethylsiloxane (PDMS), polyurethane, polyimide, styrene-ethylene-butylene-styrene (SEBS), polypropylene, polycarbonate, cyclic polyolefin polymer/copolymer (COP/COC), or any combinations thereof. As used herein, the term "biocompatible" refers to any material that does not deteriorate appreciably and does not induce a significant immune response or deleterious tissue reaction, e.g., toxic reaction or significant irritation, over time when implanted into or placed adjacent to the biological tissue of a subject, or induce blood clotting or coagulation when it comes in contact with blood.

Additionally or alternatively, at least a portion of the first structure 204 and/or second structure 206 can be made of non-flexible or rigid materials like glass, silicon, hard plastic, metal, or any combinations thereof.

The membrane 208 can be made of the same material as the first structure 204 and/or second structure 206 or a material that is different from the first structure 204 and/or second structure 206 of the devices described herein. In some embodiments, the membrane 208 can be made of a rigid material. In some embodiments, the membrane is a thermoplastic rigid material. Examples of rigid materials that can be used for fabrication of the membrane include, but are not limited to, polyester, polycarbonate or a combination thereof. In some embodiments, the membrane 208 can comprise a flexible material, e.g., but not limited to PDMS. Additional information about the membrane is further described below.

In some embodiments, the first structure and/or second structure of the device and/or the membrane can comprise or is composed of an extracellular matrix polymer, gel, and/or scaffold. Any extracellular matrix can be used herein, including, but not limited to, silk, chitosan, elastin, collagen, proteoglycans, hyaluronic acid, collagen, fibrin, and any combinations thereof.

The device in **Fig. 27A** can comprise a plurality of access ports 205. In addition, the branched configuration 203 can comprise a tissue-tissue interface simulation region (membrane 208 in **Fig. 27B****)** where cell behavior and/or passage of gases, chemicals, molecules, particulates and cells are monitored. **Fig. 27B** illustrates an exploded view of the device in accordance with an embodiment. In one embodiment, the body 202 of the device 200 comprises a first outer body portion (first structure) 204, a second outer body portion (second structure) 206 and an intermediary membrane 208 configured to be mounted between the first and second outer body portions 204, 206 when the portions 204, 206 are mounted to one another to form the overall body.

The first outer body portion or first structure 204 can have a thickness of any dimension, depending, in part, on the height of the first chamber 204. In some embodiments, the thickness of the first outer body portion or first structure 204 can be about 1 mm to about 100 mm, or about 2 mm to about 75 mm, or about 3 mm to about 50 mm, or about 3 mm to about 25 mm. In some embodiments, the first outer body portion or first structure 204 can have a thickness that is more than the height of the first chamber by no more than 5 mm, no more than 4 mm, no more than 3 mm, no more than 2 mm, no more than 1 mm, no more than 500 microns, no more than 400 microns, no more than 300 microns, no more than 200 microns, no more than 100 microns, no more than 70 microns or less. In some embodiments, it is desirable to keep the first outer body portion or first structure 204 as thin as possible such that cells on the membrane can be visualized or detected by microscopic, spectroscopic, and/or electrical sensing methods.

The second outer body portion or second structure 206 can have a thickness of any dimension, depending, in part, on the height of the second chamber 206. In some embodiments, the thickness of the second outer body portion or second structure 206 can be about 50 µm to about 10 mm, or about 75 µm to about 8 mm, or about 100 µm to about 5 mm, or about 200 µm to about 2.5 mm. In one embodiment, the thickness of the second outer body portion or second structure 206 can be about 1 mm to about 1.5 mm. In one embodiment, the thickness of the second outer body portion or second structure 206 can be about 0.2 mm to about 0.5 mm. In some embodiments, the second outer first structure and/or second structure portion 206 can have a thickness that is more than the height of the second chamber by no more than 5mm, no more than 4 mm, no more than 3 mm, no more than 2 mm, no more than 1 mm, no more than 500 microns, no more than 400 microns, no more than 300 microns, no more than 200 microns, no more than 100 microns, no more than 70 microns or less. In some embodiments, it is desirable to keep the second outer body portion or second structure 206 as thin as possible such that cells on the membrane can be visualized or detected by microscopic, spectroscopic, and/or electrical sensing methods.

In some embodiments, the first chamber and the second chamber can each independently comprise a channel. The channel(s) can be substantially linear or they can be non-linear. In some embodiments, the channels are not limited to straight or linear channels and can comprise curved, angled, or otherwise non-linear channels. It is to be further understood that a first portion of a channel can be straight, and a second portion of the same channel can be curved, angled, or otherwise non-linear. Without wishing to be bound by a theory, a non-linear channel can increase the ratio of culture area to device area, thereby providing a larger surface area for cells to grow. This can also allow for a higher amount or density of cells in the channel.

**Fig. 27B** illustrates an exploded view of the device in accordance with an embodiment. As shown in **Fig. 27B****,** the first outer body portion or first structure 204 includes one or more inlet fluid ports 210 in communication with one or more corresponding inlet apertures 211 located on an outer surface of the first structure 204. The device 200 can be connected to a fluid source via the inlet aperture 211 in which fluid travels from the fluid source into the device 200 through the inlet fluid port 210.

Additionally, the first outer body portion or first structure 204 can include one or more outlet fluid ports 212 in communication with one or more corresponding outlet apertures 215 on the outer surface of the first structure 204. In some embodiments, a fluid passing through the device 200 can exit the device to a fluid collector or other appropriate component via the corresponding outlet aperture 215. It should be noted that the device 200 can be set up such that the fluid port 210 is an outlet and fluid port 212 is an inlet.

In some embodiments, as shown in **Fig. 27B****,** the device 200 can comprise an inlet channel 225 connecting an inlet fluid port 210 to the first chamber 204. The inlet channels and inlet ports can be used to introduce cells, agents (*e.g*., but not limited to, stimulants, drug candidate, particulates), air flow, and/or cell culture media into the first chamber 204.

The device 200 can also comprise an outlet channel 227 connecting an outlet fluid port 212 to the first chamber 204. The outlet channels and outlet ports can also be used to introduce cells, agents (*e.g*., but not limited to, stimulants, drug candidate, particulates), air flow, and/or cell culture media into the first chamber 204.

Although the inlet and outlet apertures 211, 215 are shown on the top surface of the first structure 204 and are located perpendicular to the inlet and outlet channels 225, 227, one or more of the apertures 211, 215 can be located on one or more lateral surfaces of the first structure and/or second structure such that at least one of the inlet and outlet apertures 211, 215 can be in-plane with the inlet and/or outlet channels 225, 227, respectively, and/or be oriented at an angle from the plane of the inlet and/or outlet channels 225, 227.

In another embodiment, the fluid passing between the inlet and outlet fluid ports can be shared between the first chamber 204 and second chamber 206. In either embodiment, characteristics of the fluid flow, such as flow rate, fluid type and/or composition, and the like, passing through the first chamber 204 can be controllable independently of fluid flow characteristics through the second chamber 206 and vice versa.

In some embodiments, while not necessary, the first structure 204 can include one or more pressure inlet ports 214 and one or more pressure outlet ports 216 in which the inlet ports 214 are in communication with corresponding apertures 217 located on the outer surface of the device 200. Although the inlet and outlet apertures are shown on the top surface of the first structure 204, one or more of the apertures can alternatively be located on one or more lateral sides of the first structure and/or second structure. In operation, one or more pressure tubes (not shown) connected to an external force source (*e.g*., pressure source) can provide positive or negative pressure to the device via the apertures 217. Additionally, pressure tubes (not shown) can be connected to the device 200 to remove the pressurized fluid from the outlet port 216 via the apertures 223. It should be noted that the device 200 can be set up such that the pressure port 214 is an outlet and pressure port 216 is an inlet. It should be noted that although the pressure apertures 217, 223 are shown on the top surface of the first structure 204, one or more of the pressure apertures 217, 223 can be located on one or more side surfaces of the first structure 204.

Referring to **Fig. 27B****,** in some embodiments, the second structure 206 can include one or more inlet fluid ports 218 and one or more outlet fluid ports 220. As shown in **Fig. 27B****,** the inlet fluid port 218 is in communication with aperture 219 and outlet fluid port 220 is in communication with aperture 221, whereby the apertures 219 and 221 are located on the outer surface of the second structure 206. Although the inlet and outlet apertures are shown on the surface of the second structure, one or more of the apertures can be alternatively located on one or more lateral sides of the second structure.

As with the first outer body portion or first structure 204 described above, one or more fluid tubes connected to a fluid source can be coupled to the aperture 219 to provide fluid to the device 200 via port 218. Additionally, fluid can exit the device 200 via the outlet port 220 and outlet aperture 221 to a fluid reservoir/collector or other component. It should be noted that the device 200 can be set up such that the fluid port 218 is an outlet and fluid port 220 is an inlet.

In some embodiments, the second outer body portion and/or second structure 206 can include one or more pressure inlet ports 222 and one or more pressure outlet ports 224. In some embodiments, the pressure inlet ports 222 can be in communication with apertures 227 and pressure outlet ports 224 are in communication with apertures 229, whereby apertures 227 and 229 are located on the outer surface of the second structure 206. Although the inlet and outlet apertures are shown on the bottom surface of the second structure 206, one or more of the apertures can be alternatively located on one or more lateral sides of the second structure. Pressure tubes connected to an external force source (e.g., pressure source) can be engaged with ports 222 and 224 via corresponding apertures 227 and 229. It should be noted that the device 200 can be set up such that the pressure port 222 is an outlet and fluid port 224 is an inlet.

In some embodiments where the operating channels (*e.g.*, 252 shown in **Fig. 27A****)** are not mandatory, the first structure 204 does not require any pressure inlet port 214, pressure outlet port 216. Similarly, the second structure 206 does not require any pressure inlet port 222 or pressure outlet port 224.

In an embodiment, the membrane 208 is mounted between the first structure 204 and the second structure 206, whereby the membrane 208 is located within the first structure 204 and/or second structure 206 of the device 200 (see, *e.g.,* **Fig. 7B****).** In an embodiment, the membrane 208 is a made of a material having a plurality of pores or apertures therethrough, whereby molecules, cells, fluid or any media is capable of passing through the membrane 208 via one or more pores in the membrane 208. As discussed in more detail below, the membrane 208 in one embodiment can be made of a material which allows the membrane 208 to undergo stress and/or strain in response to an external force (*e.g.,* cyclic stretching or pressure). In one embodiment, the membrane 208 can be made of a material which allows the membrane 208 to undergo stress and/or strain in response to pressure differentials present between the first chamber 204, the second chamber 206 and the operating channels 252. Alternatively, the membrane 208 is relatively inelastic or rigid in which the membrane 208 undergoes minimal or no movement.

The first chamber 204 and/or the second chamber 206 can have a length suited to the need of an application (*e.g.*, a physiological system to be modeled), desirable size of the device, and/or desirable size of the view of field. In some embodiments, the first chamber 204 and/or the second chamber 206 can have a length of about 0.5 cm to about 10 cm. In one embodiment, the first chamber 204 and/or the second chamber 206 can have a length of about 1 cm to about 3 cm. In one embodiment, the first chamber 204 and/or the second chamber 206 can have a length of about 2 cm.

The width of the first chamber and/or the second chamber can vary with desired cell growth surface area. The first chamber 204 and the second chamber 206 can each have a range of width dimension (shown as W in **Fig. 7B****)** between 100 microns and 50 mm, or between 200 microns and 10 mm, or between 200 microns and 1500 microns, or between 400 microns and 1 mm, or between 50 microns and 2mm, or between 100 microns and 5 mm. In some embodiments, the first chamber 204 and the second chamber 206 can each have a width of about 500 microns to about 2 mm. In some embodiments, the first chamber 204 and the second chamber 206 can each have a width of about 1 mm.

In some embodiments, the widths of the first chamber and the second chamber can be configured to be different, with the centers of the chambers aligned or not aligned. In some embodiments, the channel heights, widths, and/or cross sections can vary along the length of the devices described herein.

The heights of the first chamber and the second chamber can vary to suit the needs of desired applications (*e.g*., to provide a low shear stress, and/or to accommodate cell size). The first chamber and the second chamber of the devices described herein can have the same heights or different heights. In some embodiments, the height of the second chamber 206 can be substantially the same as the height of the first chamber 204.

In some embodiments, the height of at least a length portion of the first chamber 204 (*e.g.,* the length portion where the cells are designated to grow) can be substantially greater than the height of the second chamber 206 within the same length portion. For example, the height ratio of the first chamber to the second chamber can be greater than 1:1, including, for example, greater than 1.1:1, 1.5:1, 2:1, 2.5:1, 3:1, 3.5:1, 4:1, 4.5:1, 5:1, 6:1, 7:1, 8:1, 9:1, 10:1, 11:1, 12:1, 13:1, 14:1, 15:1, 16:1, 17:1, 18:1, 19:1, 20:1, 25:1, 30:1, 35:1, 40:1, 45:1, 50:1. In some embodiments, the height ratio of the first chamber to the second chamber can range from 1.1:1 to about 50:1, or from about 2.5:1 to about 50:1, or from 2.5 to about 25:1, or from about 2.5:1 to about 15:1. In one embodiment, the height ratio of the first chamber to the second chamber ranges from about 1:1 to about 20:1. In one embodiment, the height ratio of the first chamber to the second chamber ranges from about 1:1 to about 15:1. In one embodiment, the height ratio of the first chamber to the second chamber can be about 10:1.

The height of the first chamber 204 can be of any dimension, *e.g*., sufficient to accommodate cell height and/or to permit a low shear flow. For example, in some embodiments, the height of the first chamber can range from about 100 µm to about 50 mm, about 200 µm to about 10 mm, about 500 µm to about 5 mm, or about 750 µm to about 2 mm. In one embodiment, the height of the first chamber can be about 150 µm. In one embodiment, the height of the first chamber can be about 1 mm.

The height of the second chamber 206 can be of any dimension provided that the flow rate and/or shear stress of a medium flowing in the second chamber can be maintained within a physiological range, or does not cause any adverse effect to the cells. In some embodiments, the height of the second chamber can range from 20 µm to about 1 mm, or about 50 µm to about 500 µm, or about 75 µm to about 400 µm, or about 100 µm to about 300 µm. In one embodiment, the height of the second chamber can be about 150 µm. In one embodiment, the height of the second chamber can be about 100 µm.

The first chamber and/or the second chamber can have a uniform height along the length of the first chamber and/or the second chamber, respectively. Alternatively, the first chamber and/or the second chamber can each independently have a varying height along the length of the first chamber and/or the second chamber, respectively. For example, a length portion of the first chamber can be substantially taller than the same length portion of the second chamber, while the rest of the first chamber can have a height comparable to or even smaller than the height of the second chamber.

In some embodiments, the first structure and/or second structure of the devices described herein can be further adapted to provide mechanical modulation of the membrane. Mechanical modulation of the membrane can include any movement of the membrane that is parallel to and/or perpendicular to the force/pressure applied to the membrane, including, but are not limited to, stretching, bending, compressing, vibrating, contracting, waving, or any combinations thereof. Different designs and/or approaches to provide mechanical modulation of the membrane between two chambers have been described, *e.g*., in the International Patent App. Nos. PCT/US2009/050830, and PCT/US2014/071570, and can be adapted herein to modulate the membrane in the devices described herein.

In some embodiments, the devices described herein can be placed in or secured to a cartridge. In accordance with some embodiments of some aspects described herein, the device can be integrated into a cartridge and form a monolithic part. Some examples of a cartridge are described in the International Patent App. No. PCT/US2014/047694. The cartridge can be placed into and removed from a cartridge holder that can establish fluidic connections upon or after placement and optionally seal the fluidic connections upon removal. In some embodiments, the cartridge can be incorporated or integrated with at least one sensor, which can be placed in direct or indirect contact with a fluid flowing through a specific portion of the cartridge during operation. In some embodiments, the cartridge can be incorporated or integrated with at least one electric or electronic circuit, for example, in the form of a printed circuit board or flexible circuit. In accordance with some embodiments of some teachings described herein, the cartridge can comprise a gasketing embossment to provide fluidic routing.

In some embodiments, the cartridge and/or the device described herein can comprise a barcode. The barcode can be unique to types and/or status of the cells present on the membrane. Thus, the barcode can be used as an identifier of each device adapted to mimic function of at least a portion of a specific tissue and/or a specific tissue-specific condition. Prior to operation, the barcode of the cartridge can be read by an instrument so that the cartridge can be placed and/or aligned in a cartridge holder for proper fluidic connections and/or proper association of the data obtained during operation of each device. In some embodiments, data obtained from each device include, but are not limited to, cell response, immune cell recruitment, intracellular protein expression, gene expression, cytokine/chemokine expression, cell morphology, functional data such as effectiveness of an endothelium as a barrier, concentration change of an agent that is introduced into the device, or any combinations thereof.

In some embodiments, the device can be connected to the cartridge by an interconnect adapter that connects some or all of the inlet and outlet ports of the device to microfluidic channels or ports on the cartridge. Some examples interconnect adapters are disclosed in U.S. Provisional Application No. 61/839,702, filed on June 26, 2013, and the International Patent Application No. PCT/US2014/044417 filed June 26, 2014. The interconnect adapter can include one or more nozzles having fluidic channels that can be received by ports of the device described herein. The interconnect adapter can also include nozzles having fluidic channels that can be received by ports of the cartridge.

In some embodiments, the interconnect adaptor can comprise a septum interconnector that can permit the ports of the device to establish transient fluidic connection during operation, and provide a sealing of the fluidic connections when not in use, thus minimizing contamination of the cells and the device. Some examples of a septum interconnector are described in U.S. Provisional Application No. 61/810,944 filed April 11, 2013.

***Membrane:*** The membrane 208 is oriented along a plane 208P parallel to the x-y plane between the first chamber 204 and the second chamber 206 as shown in **Fig. 7B****.** It should be noted that although one membrane 208 is shown in **Fig. 7B****,** more than one membrane 208 can be configured in devices which comprise more than two chambers.

The membrane separating the first chamber and the second chamber in the devices described herein can be porous (*e.g.,* permeable or selectively permeable), non-porous (*e.g.,* non-permeable), rigid, flexible, elastic or any combinations thereof. Accordingly, the membrane 208 can have a porosity of about 0% to about 99%. As used herein, the term "porosity" is a measure of total void space (*e.g*., through-holes, openings, interstitial spaces, and/or hollow conduits) in a material, and is a fraction of volume of total voids over the total volume, as a percentage between 0 and 100% (or between 0 and 1). A membrane with substantially zero porosity is non-porous or non-permeable.

As used interchangeably herein, the terms "non-porous" and "non-permeable" refer to a material that does not allow any molecule or substance to pass through.

In some embodiments, the membrane can be porous and thus allow molecules, cells, particulates, chemicals and/or media to migrate or transfer between the first chamber 204 and the second chamber 206 via the membrane 208 from the first chamber 204 to the second chamber 206 or vice versa.

As used herein, the term "porous" generally refers to a material that is permeable or selectively permeable. The term "permeable" as used herein means a material that permits passage of a fluid (*e.g.,* liquid or gas), a molecule, a whole living cell and/or at least a portion of a whole living cell, *e.g.,* for formation of cell-cell contacts. The term "selectively permeable" as used herein refers to a material that permits passage of one or more target group or species, but act as a barrier to non-target groups or species. For example, a selectively-permeable membrane can allow passage of a fluid (*e.g.,* liquid and/or gas), nutrients, wastes, cytokines, and/or chemokines from one side of the membrane to another side of the membrane, but does not allow whole living cells to pass therethrough. In some embodiments, a selectively-permeable membrane can allow certain cell types to pass therethrough but not other cell types.

The permeability of the membrane to individual matter/species can be determined based on a number of factors, including, *e.g.,* material property of the membrane (*e.g.,* pore size, and/or porosity), interaction and/or affinity between the membrane material and individual species/matter, individual species size, concentration gradient of individual species between both sides of the membrane, elasticity of individual species, and/or any combinations thereof.

A porous membrane can have through-holes or pore apertures extending vertically and/or laterally between two surfaces 208A and 208B of the membrane **(****Fig. 27B****),** and/or a connected network of pores or void spaces (which can, for example, be openings, interstitial spaces or hollow conduits) throughout its volume. The porous nature of the membrane can be contributed by an inherent physical property of the selected membrane material, and/or introduction of conduits, apertures and/or holes into the membrane material.

In some embodiments, a membrane can be a porous scaffold or a mesh. In some embodiments, the porous scaffold or mesh can be made from at least one extracellular matrix polymer (*e.g*., but not limited to collagen, alginate, gelatin, fibrin, laminin, hydroxyapatite, hyaluronic acid, fibroin, and/or chitosan), and/or a biopolymer or biocompatible material (*e.g*., but not limited to, polydimethylsiloxane (PDMS), polyurethane, styrene-ethylene-butylene-styrene (SEBS), poly(hydroxyethylmethacrylate) (pHEMA), polyethylene glycol, polyvinyl alcohol and/or any biocompatible material described herein for fabrication of the device first structure and/or second structure) by any methods known in the art, including, *e.g*., but not limited to, electrospinning, cryogelation, evaporative casting, and/or 3D printing. See, *e.g.,* Sun et al. (2012) "Direct-Write Assembly of 3D Silk/Hydroxyapatite Scaffolds for Bone Co-Cultures." Advanced Healthcare Materials, no. 1: 729-735; Shepherd et al. (2011) "3D Microperiodic Hydrogel Scaffolds for Robust Neuronal Cultures." Advanced Functional Materials 21: 47-54; and Barry III et al. (2009) "Direct-Write Assembly of 3D Hydrogel Scaffolds for Guided Cell Growth." Advanced Materials 21: 1-4, for examples of a 3D biopolymer scaffold or mesh that can be used as a membrane in the device described herein.

In some embodiments, a membrane can comprise an elastomeric portion fabricated from a styrenic block copolymer-comprising composition, *e.g.,* as described in the International Pat. App. No. PCT/US2014/071611, can be adopted in the devices described herein. In some embodiments, the styrenic block copolymer-comprising composition can comprise SEBS and polypropylene.

In some embodiments, a membrane can be a hydrogel or a gel comprising an extracellular matrix polymer, and/or a biopolymer or biocompatible material. In some embodiments, the hydrogel or gel can be embedded with a conduit network, *e.g*., to promote fluid and/or molecule transport. See, *e.g.,* Wu et al. (2011) "Omnidirectional Printing of 3D Microvascular Networks." Advanced Materials 23: H178-H183; and Wu et al. (2010) "Direct-write assembly of biomimetic microvascular networks for efficient fluid transport." Soft Matter 6: 739-742, for example methods of introducing a conduit network into a gel material.

In some embodiments, a porous membrane can be a solid biocompatible material or polymer that is inherently permeable to at least one matter/species (*e.g*., gas molecules) and/or permits formation of cell-cell contacts. In some embodiments, through-holes or apertures can be introduced into the solid biocompatible material or polymer, *e.g*., to enhance fluid/molecule transport and/or cell migration. In one embodiment, through-holes or apertures can be cut or etched through the solid biocompatible material such that the through-holes or apertures extend vertically and/or laterally between the two surfaces of the membrane 208A and 208B. It should also be noted that the pores can additionally or alternatively incorporate slits or other shaped apertures along at least a portion of the membrane 208 which allow cells, particulates, chemicals and/or fluids to pass through the membrane 208 from one section of the central channel to the other.

The pores of the membrane (including pore apertures extending through the membrane 208 from the top 208A to bottom 208B surfaces thereof and/or a connected network of void space within the membrane 208) can have a cross-section of any size and/or shape. For example, the pores can have a pentagonal, circular, hexagonal, square, elliptical, oval, diamond, and/or triangular shape.

The cross-section of the pores can have any width dimension provided that they permit desired molecules and/or cells to pass through the membrane. In some embodiments, the pore size of the membrane should be big enough to provide the cells sufficient access to nutrients present in a fluid medium flowing through the first chamber and/or the second chamber. In some embodiments, the pore size can be selected to permit passage of cells (*e.g.,* immune cells and/or cancer cells) from one side of the membrane to the other. In some embodiments, the pore size can be selected to permit passage of nutrient molecules. In some embodiments, the width dimension of the pores can be selected to permit molecules, particulates and/or fluids to pass through the membrane 208 but prevent cells from passing through the membrane 208. In some embodiments, the width dimension of the pores can be selected to permit cells, molecules, particulates and/or fluids to pass through the membrane 208. Thus, the width dimension of the pores can be selected, in part, based on the sizes of the cells, molecules, and/or particulates of interest. In some embodiments, the width dimension of the pores (*e.g*., diameter of circular pores) can be in the range of 0.01 microns and 20 microns, or in one embodiment, approximately 0.1-15 microns, or approximately 1-10 microns. In one embodiment, the pores have a width of about 7 microns.

In an embodiment, the porous membrane 208 can be designed or surface patterned to include micro and/or nanoscopic patterns therein such as grooves and ridges, whereby any parameter or characteristic of the patterns can be designed to desired sizes, shapes, thicknesses, filling materials, and the like.

The membrane 208 can have any thickness to suit the needs of a target application. In some embodiments, the membrane can be configured to deform in a manner (*e.g*., stretching, retracting, compressing, twisting and/or waving) that simulates a physiological strain experienced by the cells in its native microenvironment. In these embodiments, a thinner membrane can provide more flexibility. In some embodiments, the membrane can be configured to provide a supporting structure to permit growth of a defined layer of cells thereon. Thus, in some embodiments, a thicker membrane can provide a greater mechanical support. In some embodiments, the thickness of the membrane 208 can range between 70 nanometers and 100 microns, or between 1 micron and 100 microns, or between 10 and 100 microns. In one embodiment, the thickness of the membrane 208 can range between 10 microns and 80 microns. In one embodiment, the thickness of the membrane 208 can range between 30 microns and 80 microns. In one embodiment, the thickness of the membrane 208 can be about 50 uM.

While the membrane 208 generally have a uniform thickness across the entire length or width, in some embodiments, the membrane 208 can be designed to include regions which have lesser or greater thicknesses than other regions in the membrane 208. The decreased thickness area(s) can run along the entire length or width of the membrane 208 or can alternatively be located at only certain locations of the membrane 208. The decreased thickness area can be present along the bottom surface of the membrane 208 (i.e. facing second chamber 206), or additionally/alternatively be on the opposing surface of the membrane 208 (i.e. facing second chamber 204). It should also be noted that at least portions of the membrane 208 can have one or more larger thickness areas relative to the rest of the membrane, and capable of having the same alternatives as the decreased thickness areas described above.

In some embodiments, the membrane can be coated with substances such as various cell adhesion promoting substances or ECM proteins, such as fibronectin, laminin, various collagen types, glycoproteins, vitronectin, elastins, fibrin, proteoglycans, heparin sulfate, chondroitin sulfate, keratin sulfate, hyaluronic acid, fibroin, chitosan, or any combinations thereof. In some embodiments, one or more cell adhesion molecules can be coated on one surface of the membrane 208 whereas another cell adhesion molecule can be applied to the opposing surface of the membrane 208, or both surfaces can be coated with the same cell adhesion molecules. In some embodiments, the ECMs, which can be ECMs produced by cells, such as primary cells or embryonic stem cells, and other compositions of matter are produced in a serum-free environment.

In an embodiment, one can coat the membrane with a cell adhesion factor and/or a positively-charged molecule that are bound to the membrane to improve cell attachment and stabilize cell growth. The positively charged molecule can be selected from the group consisting of polylysine, chitosan, poly(ethyleneimine) or acrylics polymerized from acrylamide or methacrylamide and incorporating positively-charged groups in the form of primary, secondary or tertiary amines, or quaternary salts. The cell adhesion factor can be added to the membrane and is fibronectin, laminin, various collagen types, glycoproteins, vitronectin, elastins, fibrin, proteoglycans, heparin sulfate, chondroitin sulfate, keratin sulfate, hyaluronic acid, tenascin, antibodies, aptamers, or fragments or analogs having a cell binding domain thereof. The positively-charged molecule and/or the cell adhesion factor can be covalently bound to the membrane. In another embodiment, the positively-charged molecule and/or the cell adhesion factor are covalently bound to one another and either the positively-charged molecule or the cell adhesion factor is covalently bound to the membrane. Also, the positively-charged molecule or the cell adhesion factor or both can be provided in the form of a stable coating non-covalently bound to the membrane.

In an embodiment, the cell attachment-promoting substances, matrix-forming formulations, and other compositions of matter are sterilized to prevent unwanted contamination. Sterilization can be accomplished, for example, by ultraviolet light, filtration, gas plasma, ozone, ethylene oxide, and/or heat. Antibiotics can also be added, particularly during incubation, to prevent the growth of bacteria, fungi and other undesired microorganisms. Such antibiotics include, by way of non-limiting example, gentamicin, streptomycin, penicillin, amphotericin and ciprofloxacin.

In some embodiments, the membrane and/or other components of the devices described herein can be treated using gas plasma, charged particles, ultraviolet light, ozone, or any combinations thereof.

Using the devices described herein, one can study biotransformation, absorption, as well as drug clearance, metabolism, delivery and toxicity. The activation of xenobiotics can also be studied. The bioavailability and transport of chemical and biological agents across epithelial layers as in a tissue or organ, *e.g.,* lung, and endothelial layers as in blood vessels, and across the liver for drug metabolism can also be studied. The acute basal toxicity, acute local toxicity or acute organ-specific toxicity, teratogenicity, genotoxicity, carcinogenicity, and mutagenicity, of chemical agents can also be studied. Effects of infectious biological agents, biological weapons, harmful chemical agents and chemical weapons can also be detected and studied. Infectious diseases and the efficacy of chemical and biological agents to treat these diseases, as well as optimal dosage ranges for these agents, can be studied. The response of organs *in vivo* to chemical and biological agents, and the pharmacokinetics and pharmacodynamics of these agents can be detected and studied using the devices described herein. The impact of genetic content on response to the agents can be studied. The amount of protein and gene expression in response to chemical or biological agents can be determined. Changes in metabolism in response to chemical or biological agents can be studied as well using devices described herein.

The Liver-on-Chip can be used to assess the clearance of a test compound. For clearance studies, the disappearance of a test compound can be measured (e.g. using mass spec) in the media of the top chamber, bottom chamber, or both chambers (divided by a membrane comprising liver cells).

In accordance to one teachnig of the invention, Liver-on-Chip drug-metabolising performance is measured by i) disposing a substrate compound with known liver metabolites in the media of the top chamber, bottom chamber or both chambers; and ii) measuring the amount of generated metabolite in the media of the top chamber, bottom chamber or both chambers (e.g. using mass spec). As is known in the art, the choice of the substrate and measured metabolite can help provide information on specific liver drug-metabolism enzymes (e.g. CYP450 isoforms, Phase II enzymes, etc.)

The Liver-on-Chip can be used to assess the induction or inhibition potential of a test compound. For induction or inhibition studies a variety of tests are contemplated. For example, induction of CYP3A4 activity in the liver is one of main causes of drug-drug interactions, which is a mechanism to defend against exposure to drugs and toxin, but can also lead to unwanted side-effects (toxicity) or change the efficacy of a drug. A reliable and practical CYP3A induction assay with human hepatocytes in a 96-well format has been reported, where various 96-well plates with different basement membrane were evaluated using prototypical inducers, rifampicin, phenytoin, and carbamazepine. See Drug Metab. Dispo. (2010) Nov;38(11):1912-6. According to one aspect of the invention, the induction or inhibition potential of a test compound at a test concentration is evaluated by i) disposing the test compound in the media of the top chamber, bottom chamber or both chambers at the test concentration; ii) exposing the device for a selected period of time; and iii) assessing the induction or inhibition of liver enzymes by comparing liver performance to a measurement performed before the test compound was applied, to a measurement performed on a Liver-on-Chip that was subjected to a lower concentration of test compound (or no test compound at all), or both. In some embodiments, the liver performance measurement comprises an RNA expression level. In some embodiments, the liver performance measurement comprises assessing drug-metabolising capacity.

The Liver-on-Chip can be used to identify the in vivo metabolites of a test compound, and optionally the in vivo ratio of these metabolites. According to one aspect of the invention, in vivo metabolites are identified by i) disposing a test compound in the media of the top chamber, bottom chamber or both chambers; and ii) measuring the concentration of metabolites in the media of the top chamber, bottom chamber, or both chambers. In some embodiments, the measuring of the concentration of metabolites comprises mass spectroscopy.

The Liver-on-Chip can be used to identify the toxicity of a test compound at a test concentration. According to one teaching of the disclosure, toxicity is evaluated by i) disposing a test compound in the media of the top chamber, bottom chamber or both chambers; and ii) measuring one or more toxicity endpoints selected from the list of leakage of cellular enzymes (e.g. lactose dehydrogenase, alanine aminotransferase, aspartate aminotransferase) or material (e.g. adenosine triphosphate), variation in RNA expression, inhibition of drug-metabolism capacity, reduction of intracellular ATP (adenosine triphosphate), cell death, apoptosis, and cell membrane degradation.

### Exemplary methods of making the devices described herein

Embodiments of various devices comprising a first chamber, a second chamber, and a membrane can enable us to leverage the control of microfluidic technology for device fabrication. In some embodiments, the devices described herein can be manufactured using any conventional fabrication methods, including, e.g., injection molding, embossing, etching, casting, machining, stamping, lamination, photolithography, or any combinations thereof. Soft lithography techniques are described in "Soft Lithography in Biology and Biochemistry," by Whitesides, et al., published Annual Review, Biomed Engineering, 3.335-3.373 (2001), as well as "An Ultra-Thin PDMS Membrane As A Bio/Micro-Nano Interface: Fabrication And Characterization", by Thangawng et al., Biomed Microdevices, vol. 9, num. 4, 2007, p. 587-95, both of which are hereby incorporated by reference.

Without wishing to be limiting, in some embodiments, the devices described herein can be produced as a monolithic device or as individual components (e.g., a first structure comprising a first chamber, a second structure comprising a second chamber, and a membrane), which can then be assembled together to form a device described herein. Each individual component can be produced by a conventional manufacturing method such as injection molding, extrusion, casting, lamination, embossing, compression molding, solvent casting, an additive manufacturing method *(e.g.,* 3D printing), or any combinations thereof.

Once the first and second structures 204, 206 are formed and removed from their respective molds, the access ports can be made to access the chambers.

The membrane 208 can be engineered for a variety of purposes, some discussed above.

In some embodiments, the membrane 208 can be sandwiched between the first structure and the second structure, *e.g*., using an appropriate adhesive or epoxy, physical clamping and/or plasma bond between the two PDMS surfaces, in order to form a fluidic seal between the membrane with the first structure and the second structure.

After forming the body of the devices described herein, the first side of the membrane can be coated with an ECM composition according to one or more embodiments described herein. After formation of the ECM composition, tissue specific cells, *e.g.,* hepatocytes, can be grown thereon.

In some embodiments, at least one layer of cells comprising blood vessel-associated cells (*e.g*., fibroblasts, smooth muscle cells, and/or endothelial cells) can be cultured on the second side of the membrane.

### Closed Top Microfluidic Chips

The present disclosure relates to Liver-On-Chips, such as fluidic devices comprising one or more cells types for the simulation one or more of the function of liver components. Accordingly, the present disclosure additionally describes closed-top microfluidic Liver-On-Chips, see, *e.g*. schematic in Figure 27.

### Closed Top Microfluidic Chips Without Gels

In one embodiment, closed top Liver-On-Chips do not contain gels, either as a bulk gel or a gel layer or modified gel layer. As additional examples, closed top microfluidic Liver-On-Chips without gels may or may not have added ECM overlays; closed top microfluidic Liver-On-Chips without gels may or may not have added ECM layers. Thus, in some embodiments, in closed top microfluidic Liver-On-Chips without gels ECM may only be provided by the cells contained within the Liver-On-Chip without gels.

Thus, in one embodiment, the device generally comprises (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber, wherein the first and second chambers are enclosed. The first side of the membrane may have an extracellular matrix composition disposed thereon, wherein the extracellular matrix (ECM) composition comprises an ECM coating layer. In some embodiments, an ECM gel layer *e.g.* ECM overlay, is located over the ECM coating layer.

Additional embodiments are described herein that may be incorporated into closed top microfluidic Liver-On-Chips without gels.

### Closed Top Microfluidic Chips With Gels

In one embodiment, closed top Liver-On-Chips do contain gels, such as a gel layer, a modified gel layer, a bulk gel, etc., including but not limited to a gel matrix, hydrogel, etc. As additional examples, closed top microfluidic Liver-On-Chips with gels may or may not have added ECM overlays; closed top microfluidic Liver-On-Chips with gels may or may not have added ECM layers. Thus, in some embodiments, in closed top microfluidic Liver-On-Chips with gels ECM may only be provided by the cells contained within the Liver-On-Chip with gels.

Thus, in one embodiment, the device generally comprises (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber, wherein the first and second chambers are enclosed. In some embodiments, the device further comprises a gel. In some embodiments, the gel is a continuous layer. In some embodiments, the gel is a layer of approximately the same thickness across the layer. In some embodiments, the gel is a discontinuous layer. In some embodiments, the gel has different thicknesses across the layer. In some embodiments, the first side of the membrane may have a gel layer. In some embodiments, a gel is added to the first side of the membrane without an ECM layer. The first side of the membrane may have an extracellular matrix composition disposed thereon, wherein the extracellular matrix (ECM) composition comprises an ECM coating layer. In some embodiments, an ECM gel layer *e.g.* ECM overlay, is located over the ECM coating layer. In some embodiments, the gel layer is above the ECM coating layer. In some embodiments, the ECM coating layer may have a gel layer on the bottom, i.e. the side facing the membrane. In some embodiments, the gel overlays the ECM gel layer.

Additional embodiments are described herein that may be incorporated into closed top microfluidic Liver-On-Chips with gels.

### Closed Top Microfluidic Chips With Simulated Lumens

Closed top Liver-On-Chips comprising gel simulated lumens, as a modified gel layer, may be used for generating a more physiological relevant model of liver tissue. In some embodiments, closed top Liver-On-Chips further comprise gel simulated three-dimensional (3-D) lumens found throughout the liver. In other words, a 3-D lumen may be formed using gels for providing simulated sinusoid liver lumens lined with endothelium and containing white blood cells, such as Kupffer cells, T-cells, etc., and/or providing an attachment for hepatocytes (with or without other types of liver cells) for creating secretary canaliculi mimics as the fluidic region of the device. In vivo, secretary canaliculi are lined by the apical microvillar region of hepatocytes into which they secrete bile intended for transport to an epithelial lined bile duct. In any case, in a preferred embodiment, the gel forms at least one or more lumens, i.e. by viscous fingering patterning. As additional examples, closed top microfluidic Liver-On-Chips with a modified gel layer may or may not have added ECM overlays; closed top microfluidic Liver-On-Chips with a modified gel layer may or may not have added ECM layers. Thus, in some embodiments, in closed top microfluidic Liver-On-Chips with a modified gel layer ECM may only be provided by the cells contained within the Liver-On-Chip without gels.

Using viscous fingering techniques, *e.g*. viscous fingering patterning, simulated liver sinusoidal lumens may be formed. As one example, viscous fingers may be formed and used to mimic sinusoidal lumens in liver.

Methods to create three-dimensional (3-D) lumen structures in permeable matrices are known in the art. One example of a 3-D structure forming at least one or more lumens is referred to as "viscous fingering". One example of viscous fingering methods that may be used to for form lumens, *e.g.* patterning lumens, is described by Bischel, et al. "A Practical Method for Patterning Lumens through ECM Hydrogels via Viscous Finger Patterning." J Lab Autom. 2012 Apr; 17(2): 96-103. Author manuscript; available in PMC 2012 Jul 16, herein incorporated by reference in its entirety. In one example of a viscous finger patterning method for use with microfluidic Liver-On-Chips, lumen structures are patterned with an ECM hydrogel.

"Viscous" generally refers to a substance in between a liquid and a solid, i.e. having a thick consistency. A "viscosity" of a fluid refers to a measure of its resistance to gradual deformation by shear stress or tensile stress. For liquids, it corresponds to an informal concept of "thickness"; for example, honey has a much higher viscosity than water.

"Viscous fingering" refers in general to the formation of patterns in "a morphologically unstable interface between two fluids in a porous medium.

A "viscous finger" generally refers to the extension of one fluid into another fluid. Merely as an example, a flowable gel or partially solidified gel may be forced, by viscous fingering techniques, into another fluid, into another viscous fluid in order to form a viscous finger, *i.e.* simulated liver sinusoidal lumens.

In some embodiments, the lumen can be formed by a process comprising (i) providing the first chamber filled with a viscous solution of the first matrix molecules; (ii) flowing at least one or more pressure-driven fluid(s) with low viscosity through the viscous solution to create one or more lumens each extending through the viscous solution; and (iii) gelling, polymerizing, and/or cross linking the viscous solution. Thus, one or a plurality of lumens each extending through the first permeable matrix can be created.

In another embodiment, gel is added to a microfluidic channel for making a lumen.

In some embodiments as described herein, the first and second permeable matrices can each independently comprise a hydrogel, an extracellular matrix gel, a polymer matrix, a monomer gel that can polymerize, a peptide gel, or a combination of two or more thereof. In one embodiment, the first permeable matrix can comprise an extracellular matrix gel, (*e.g.* collagen). In one embodiment, the second permeable matrix can comprise an extracellular matrix gel and/or protein mixture gel representing an extracellular miroenvironment, (*e.g*. MATRIGEL^{®}. In some embodiments, the first and second permeable matrixes can each independently comprise a polymer matrix. Methods to create a permeable polymer matrix are known in the art, including, *e.g*. but not limited to, particle leaching from suspensions in a polymer solution, solvent evaporation from a polymer solution, sold-liquid phase separation, liquid - liquid phase separation, etching of specific "block domains" in block co-polymers, phase separation to block-co-polymers, chemically cross-linked polymer networks with defined permabilities, and a combination of two or more thereof.

Another example for making branched structures using fluids with differing viscosities is described in "Method And System For Integrating Branched Structures In Materials" to Katrycz, Publication number US20160243738, herein incorporated by reference in its entirety.

Regardless of the type of lumen formed by a gel and/or structure, cells can be attached to theses structures either to lumen side of the gel and/or within the gel and/or on the side of the gel opposite the lumen. Thus, three-dimensional (3-D) lumen gel structures may be used in several types of embodiments for closed top microfluidic chips. Merely as examples, liver sinusoidal endothelial cells (LSECs) can be attached to the outside of the gel lumen, or within the gel lumen. In some embodiments, Kupffer cells may be added within the gel with or without LSECs within the gel. More specifically, in some embodiments, where Kupffer cells are added within the gel, LSECs are attached to the outside of the gel. In further embodiments, hepatocytes with or without other cells, such as stellate cells, are attached to the outside of the gel, on top of the LSECs. In some embodiments, where Kupffer cells are added within the gel, LSECs are also added within the gel. In further embodiments, hepatocytes with or without other cells, such as stellate cells, are attached, to the outside of the gel.

In some embodiments, Kupffer cells may be attached to the outside of the gel. In some embodiments the Kupffer cells are attached to the outside of the gel with LSECs. In some embodiments, where Kupffer cells and LSECs are attached to the outside of the gel, subsequently hepatocytes with or without other cells, such as stellate cells, are attached (added) on top of the Kupffer cells and LSECs.

In some embodiments, microfluidic Liver-On-Chips with lumens do not have ECM layers or ECM overlays added to these chips. Thus, any ECM present is provided by the cells contained in the chips.

### ECM overlays with microfluidic Liver-On-Chips

Additional embodiments are described herein that may be incorporated into closed top microfluidic Liver-On-Chips comprising gel lumens, including but not limited to simulated 3D lumens.

### Open Top Microfluidic Chips

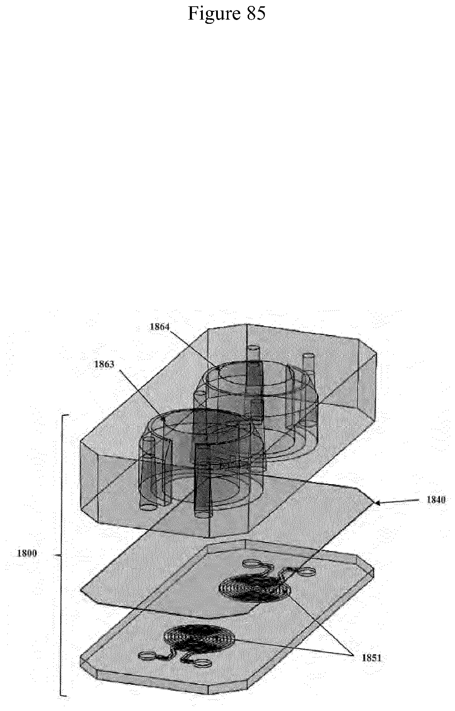

The present disclosure relates to Liver-On-Chips, such as fluidic devices comprising one or more cells types for the simulation one or more of the function of liver components. Open top microfluidic chips include but are not limited to chips having removable covers, such as removable plastic covers, paraffin covers, tape covers, etc. Accordingly, the present disclosure additionally describes open-top Liver-On-Chips, see, *e.g.* schematic in Figure 85. Figure 85 shows an exemplary exploded view of one embodiment of an open-top microfluidic chip device 1800, wherein a membrane 1840 resides between the bottom surface of the first chamber 1863 and the second chamber 1864 and the at least two spiral microchannels 1851.

Many of the problems associated with earlier systems can be solved by providing an open-top style microfluidic device that allows topical access to one or more parts of the device or cells that it comprises. For example, the microfluidic device can include a removable cover, that when removed, provides access to the cells of interest in the microfluidic device. In some teachings, the microfluidic devices include systems that constrain fluids, cells, or biological components to desired area(s). The improved systems provide for more versatile experimentation when using microfluidic devices, including improved application of treatments being tested, improved seeding of additional cells, and/or improved aerosol delivery for select tissue types.

It is also desirable in some teachings to provide access to regions of a cell-culture device. For example, it can be desirable to provide topical access to cells to (i) apply topical treatments with liquid, gaseous, solid, semi-solid, or aerosolized reagents, (ii) obtain samples and biopsies, or (iii) add additional cells or biological/chemical components

Therefore, the present disclosure relates to fluidic systems that include a fluidic device, such as a microfluidic device with an opening that provides direct access to device regions or components (e.g. access to a gel region, access to one or more cellular components, etc.). Although the present disclosure provides an embodiment wherein the opening is at the top of the device (referred to herein with the term "open top"), the present invention contemplates other embodiments where the opening is in another position on the device. For example, in one embodiment, the opening is on the bottom of the device. In another embodiment, the opening is on one or more of the sides of the device. In another embodiment, there is a combination of openings (e.g. top and sides, top and bottom, bottom and side, etc.).

While detailed discussion of the "open top" embodiment is provided herein, those of ordinary skill in the art will appreciate that many aspects of the "open top" embodiment apply similarly to open bottom embodiments, as well as open side embodiments or embodiments with openings in any other regions or directions, or combinations thereof. Similarly, the device need not remain "open" throughout its use; rather, as several embodiments described herein illustrate, the device may further comprise a cover or seal, which may be affixed reversibly or irreversibly. For example, removal of a removable cover creates an opening, while placement of the cover back on the device closes the device. The opening, and in particular the opening at the top, provides a number of advantages, for example, allowing (i) the creation of one or more gel layers for simulating the application of topical treatments on the cells, tissues, or organs, or (ii) the addition of chemical or biological components such as the seeding of additional cell types for simulated tissue and organ systems. The present disclosure further relates to improvement in fluidic system(s) that improve the delivery of aerosols to simulated tissue and organ systems, such as simulated liver tissues.

The present disclosure contemplates a variety of uses for these open top microfluidic devices and methods described herein. In one embodiment, the present invention contemplates a method of topically testing an agent (whether a drug, food, gas, or other substance) comprising 1) providing a) an agent and b) microfluidic device comprising i) a chamber, said chamber comprising a lumen and projections into the lumen, said lumen comprising ii) a gel matrix anchored by said projections and comprising cell in, on or under said gel matrix, said gel matrix positioned above iii) a porous membrane and under iv) a removable cover, said membrane in contact with v) fluidic channels; 2) removing said removable cover; and 3) topically contacting said cells in, on or under said gel matrix with said agent. In one embodiment, said agent is in an aerosol. In one embodiment, agent is in a liquid, gas, gel, semi-solid, solid, or particulate form. These uses may apply to the open top microfluidic chips described below and herein.

### gels.

### Open Top Microfluidic Chips With Gels

Furthermore, the present disclosure contemplates improvements to fluidic systems that include a fluidic device, such as a microfluidic device with an open-top region that reduces the impact of stress that can cause the delamination of tissue or related component(s) (*e.g.,* such as a gel layer or modified gel layer, or a bulk gel, etc.). Thus, in a preferred embodiment, the open-top microfluidic device comprises a gel matrix. In one embodiment, the open-top microfluidic device comprisses a gel overly. In one embodiment, the open-top microfluidic device does not contain a bulk gel.

The present invention also contemplates, in one embodiment, a layered structure comprising i) fluidic channels covered by ii) a porous membrane, said membrane comprising iii) a layer of cells and said membrane positioned below iv) a gel matrix. In one embodiment, there is a removable cover over the gel matrix (and/or cells). It is not intended that the present invention be limited to embodiments with only one gel or gel layer. In one embodiment, the layered structure further comprises a second gel matrix (e.g. positioned under said membrane). The gel(s) or coatings can be patterned or not patterned. Moreover, when patterned, the pattern need not extend to the entire surface. For example, in one embodiment, at least a portion of said gel matrix is patterned. It is not intended that the present invention be limited by the nature or components of the gel matrix or gel coating. In one embodiment, gel matrix comprises collagen. A variety of thickness is contemplated. In one embodiment of the layered structure, said gel matrix is between 0.2 and 6 mm in thickness.

Also described is a simulated lumen further comprising gel projections into the simulated lumen. Thus, in yet another embodiment, the present invention contemplates a microfluidic device comprising i) a chamber, said chamber comprising a lumen and projections in the lumen, said lumen comprising ii) a gel matrix anchored by said projections, said gel matrix positioned above iii) a porous membrane, said membrane in contact with iv) fluidic channels. In one embodiment, said membrane comprises cells. The projections serve as anchors for the gel. The projections, in one embodiment, project outward from the sidewalls. The projections, in another embodiment, project upward. The projects, in another embodiment, project downward. The projections can take a number of forms (e.g. a T structure, a Y structure, a structure with straight or curving edges, etc.). In some embodiments, there are two or more projections; in other embodiments, there are four or more projections to anchor the gel matrix. In one embodiment, the membrane is above said fluidic channels.

In other embodiments, open top microfluidic chips comprise partial lumens as described herein for closed top chips. Thus, in some embodiments, open top microfluidic chips comprise lumens formed by viscous fingering described herein for closed top chips.

Lumen gel structures may be used in several types of embodiments for open top microfluidic chips, e.g. LSECs can be attached to outside of the gel, or within the gel, hepatocytes may be attached outside of, or within the gel.

Additional embodiments are described herein that may be incorporated into open top microfluidic Liver-On-Chips with gels. For examples, open top microfluidic Liver-On-Chips with gels may or may not have added ECM overlays; open top microfluidic Liver-On-Chips with gels may or may not have added ECM layers. Thus, in some embodiments, ECM in open top microfluidic Liver-On-Chips with gels may only be provided by the cells contained within the Liver-On-Chip with gels. Additional examples are provided herein.

### Exemplary methods of using the devices and systems described herein

The ability of the devices described herein to recapitulate a physiological microenvironment and/or function can provide an *in vitro* model versatile for various applications, *e.g.,* but not limited to, modeling a liver-specific physiological condition (*e.g.,* but not limited to normal and disease states) and/or identification of therapeutic agents. Accordingly, methods of using the devices are also described herein. In one teaching, the method comprises: (a) providing at least one device according to one or more embodiments described herein; and (b) flowing a first fluid through the first chamber.

In some embodiments, the method can further comprise flowing the second chamber with a second fluid.

In some embodiments, the method can further comprise detecting response of the endothelial cells and/or tissue specific cells (*e.g*., hepatocytes) in the device and/or detecting at least one component (*e.g*., a cytokine or molecule secreted or consumed by the cells in the device) present in an output fluid from the device. Methods to detect different types of cell response are known in the art, including, *e.g*., but not limited to cell labeling, immunostaining, optical or microscopic imaging (*e.g*., immunofluorescence microscopy and/or scanning electron microscopy), gene expression analysis, cytokine/chemokine secretion analysis, metabolite analysis, polymerase chain reaction, immunoassays, ELISA, gene arrays, and any combinations thereof.

In some embodiments, the devices described herein can be used to create an *in vitro* model that mimics a liver-specific condition. As used herein, the term "liver-specific condition" refers to any condition that can be diagnosed in a liver *in vivo.* The condition can occur naturally in the liver *in vivo* (including, *e.g.,* a normal healthy condition, or a condition induced or caused by a congenital defect), or induced or caused by a condition-inducing agent or stimulant (*e.g*., including, but not limited to an environmental agent such as alcohol).

In some embodiments, liver-specific cells such as hepatocytes can be derived from established cells lines and/or collected from a subject, *e.g*., by a biopsy, and/or differentiated from stem cells. In some embodiments, the stem cells (*e.g.,* induced pluripotent stem cells) can be generated by differentiating somatic cells (*e.g.,* skin fibroblasts) collected from a subject.

In some embodiments, disease-specific cells can be obtained from one or more patients diagnosed with the specific disease.

In other embodiments, the liver-specific cells (*e.g*., normal liver-specific cells) can be contacted with a condition-inducing agent described herein that is capable of inducing the liver-specific cells to acquire at least one characteristic associated with the liver-specific condition.

In some embodiments, a liver-specific condition, *e.g.,* a disease-specific condition can be created by genetically modifying normal healthy cells, *e.g.,* by silencing one or more genes, or over-expressing one or more genes. Methods of gene silencing include, but are not limited to, RNA interference (*e.g*., but not limited to small interfering RNA (siRNA), microRNA (miRNA), and/or short hairpin RNA (shRNA)), antisense oligonucleotides, ribozymes, triplex forming oligonucleotides, and the like.

In some embodiments where the devices described herein are used to create a disease-specific model, the devices can further comprise normal healthy cells (*e.g*., obtained from one or more healthy donors) cultured in a separate chamber, *e.g*., to create a baseline for comparison.

In some embodiments, the device can comprise both healthy and disease-specific cells. In some embodiments, the device can include only disease-specific cells.

In some embodiments, the devices described herein can be used to determine an effect of a test agent (*e.g.,* toxicity) on the cells cultured in the devices described herein. Accordingly, in some embodiments, the method can further comprise contacting the liver-specific cells (*e.g*., hepatocytes) and/or endothelial cell layer with a test agent. Non-limiting examples of the test agents include proteins, peptides, single or double-stranded nucleic acids, antigens, nanoparticles, environmental toxins or pollutant, naturally occurring particles including, *e.g*., pollen, chemical or bioweapons, small molecules, drugs or drug candidates, chemicals or particles used in cosmetic products, aerosols, vaccine or vaccine candidates, pro-inflammatory agents, viruses, bacteria, unicellular organisms, cytokines, and any combinations thereof.

Effects of the test agent on the cells can be determined by measuring response of the cells to the test agent, the fluid exiting the first chamber, the fluid exiting the second chamber, or any combinations thereof; and comparing the measured response with the cells not contacted with the test agent. Various methods to measure cell response are known in the art, including, but not limited to, cell labeling, immunostaining, optical or microscopic imaging (*e.g*., immunofluorescence microscopy and/or scanning electron microscopy), spectroscopy, gene expression analysis, cytokine/chemokine secretion analysis, metabolite analysis, polymerase chain reaction (PCR), immunoassays, ELISA, gene arrays, spectroscopy, immunostaining, electrochemical detection, polynucleotide detection, fluorescence anisotropy, fluorescence resonance energy transfer, electron transfer, enzyme assay, magnetism, electrical conductivity (*e.g*., trans-epithelial electrical resistance (TEER)), isoelectric focusing, chromatography, immunoprecipitation, immunoseparation, aptamer binding, filtration, electrophoresis, use of a CCD camera, mass spectroscopy, or any combination thereof. Detection, such as cell detection, can be carried out using light microscopy with phase contrast imaging and/or fluorescence microscopy based on the characteristic size, shape and refractile characteristics of specific cell types. Greater specificity can be obtained using optical imaging with fluorescent or cytochemical stains that are specific for individual cell types or microbes.

The cellular responses to the test agent, various environmental and/or external cues can be monitored using various systems that can be combined with the devices described herein. For example, one can monitor changes in pH using well known sensors. One can integrate force sensors into the membrane to measure changes in the mechanical properties of the cells. One can also sample cells, continuously or periodically for measurement of changes in gene transcription or changes in cellular biochemistry or structural organization. For example, one can measure reactive oxygen species (ROSs) that are a sign of cellular stress. One can also subject the "tissue" grown on either side or both sides of the membrane to microscopic analysis, immunohistochemical analysis, *in situ* hybridization analysis, or typical pathological analysis using staining, such as hematoxylin and eosin staining. Samples for these analyses can be carried out in real-time, or taken after an experiment or by taking small biopsies at different stages during a study or an experiment.

In some embodiments, the exclusion of fluorescently labeled large molecules (*e.g*. dextrans of different weight or FITCs) can be quantitated to determine the permeability of the endothelium and thus assess the barrier function of the epithelium, *e.g*., in a tissue-specific condition. For example, flowing a fluid containing fluorescently labeled large molecules (*e.g*., but not limited to, inulin-FITC) into a first chamber cultured with differentiated epithelium can provide a non-invasive barrier measurement. As a functional tight junction barrier will prevent large molecules from passing through the epithelium from the first chamber to the second chamber, the absence of the detection of the fluorescently labeled large molecules in the second chamber is indicative of a functional barrier function of the epithelium.

In some embodiments, one can subject the cells grown on the membrane to other cells, such as immune system cells or bacterial cells, to antibodies or antibody-directed cells, for example to target specific cellular receptors. One can expose the cells to viruses or other particles. To assist in detection of movement of externally supplied substances, such as cells, viruses, particles or proteins, one can naturally label them using typical means such as radioactive or fluorescent labels.

In some embodiments where the liver-specific cells to be assayed are adapted to be condition-specific (*e.g*., disease-specific), exposure of the liver-specific cells to a test agent followed by determination of the effect of the test agent on the cells can facilitate identification of a therapeutic agent for treatment of the condition, or measurement of efficacy of a therapeutic agent. As used herein, the term "efficacy" generally refers to ability of a test agent to produce a desired therapeutic effect or outcome. Depending on the nature and/or type of the test agents, examples of desired effects or outcomes include, but are not limited to, therapeutic effect, cytotoxicity, cell growth, cell differentiation, improved or reduced cell function or phenotype (*e.g*., but not limited to, permeability of a cell layer, cell migration, expression and/or secretion of a protein or cytokine that can be affected by cell exposure to the test agent), and any combinations thereof. The term "therapeutic effect" as used herein refers to a consequence of treatment, the results of which are judged to be desirable and beneficial.

In some embodiments where the liver-specific cells are patient-specific, exposure of the patient-specific cells to a test agent, followed by determination of the effect of the test agent on the cells can facilitate identification of a personalized treatment for a subject.

In some embodiments where the tissue-specific cells are patient population-specific, exposure of the patient population-specific cells to a test agent, followed by determination of the effect of the test agent on the cells can facilitate identification of a treatment specified for that particular patient population. As used herein, the term "patient population-specific" refers to cells collected from a population of patients sharing at least one or more phenotypes and/or characteristics (*e.g*., but not limited to, specific gene mutation, ethnicity, gender, life styles, BMI, resistance to treatment, and any combinations thereof) other than the disease or disorder.

In accordance with some embodiments of various teachings described herein, the devices can be used to determine toxicity of a test agent upon exposure to the hepatocytes. For example, the toxicity of a test agent can be determined by measuring response of the hepatocytes and/or at least one component present in a fluid (*e.g*., gaseous and/or liquid fluid) within the device or present in an output fluid (*e.g*., gaseous and/or liquid fluid) from the device after exposure to the test agent. As used herein, the term "toxicity" refers to ability of a test agent to induce or cause any adverse and/or side effect on a cell and/or even cell death. For example, the toxicity of a test agent can be characterized by its ability to induce or cause an adverse effect on cell function and/or phenotype, including, but not limited to, alteration in cell metabolism, mutagenicity, carcinogenicity, teratogenicity, DNA damage, protein or membrane damage, cell energy depletion, mitochondrial damage, genotoxicity, apoptosis, cell death, cell rupture, and any combinations thereof.

In some embodiments, one or more devices described herein can be used in combination with a pharmacokinetic (PK) model, a pharmacodynamic (PD) model, or a PK-PD model to quantitatively analyze the effect of an agent to be tested. For example, a series of devices, each modeling a tissue, *e.g*., one for gut, one for liver, and another one for heart, can be connected to provide a microphysiological system that can be used to determine the fate of an agent administered into the system. The term "pharmacokinetics" is used herein in accordance with the art, and refers to the study of the action of agents, *e.g*., drugs, in the first structure and/or second structure, for example, the effect and duration of drug action, the rate at which they are absorbed, distributed, metabolized, and eliminated by the first structure and/or second structure etc. (*e.g.* the study of a concentration of an agent, *e.g.,* a drug, in the serum of a patient following its administration via a specific dose or therapeutic regimen). The term "pharmacodynamics" is used in accordance with the art, and refers to the study of the biochemical and physiological effects of an agent, *e.g.,* a drug, on a subject's first structure and/or second structure or on microorganisms such as viruses within or on the first structure and/or second structure, and the mechanisms of drug action and the relationship between drug concentration and effect (*e.g.* the study of a pathogen, *e.g.,* a virus, present in a patient's plasma following one or more therapeutic regimens). Methods for PK-PD modeling and analysis are known in the art. See, *e.g*., Bonate, P.L. (2006). Pharmacokinetic-Pharmacodynamic Modeling and Simulation. New York, Springer Science & Business Media; Gabrielsson, J. and D. Weiner (2000); and Pharmacokinetic and Pharmacodynamic Data Analysis: Concepts and Applications. Stockholm, Swedish Pharmaceutical Press. For example, a PK model can be developed to model a microphysiological system comprising a plurality of the devices described herein, wherein each device can model a different tissue that can produce an effect (*e.g*., absorption, metabolism, distribution and/or excretion) on an agent to be administered. To construct a PK model for a device described herein, mass balance equations describing the flow in, flow out, and metabolism of an agent can be set up for each first chamber and second chamber. A PD model can be integrated into each device described herein, describing the kinetics of potential cell response (*e.g*., inflammation, cytokine release, ligand binding, cell membrane disruption, cell mutation and/or cell death) in each device that mimics a tissue or an organ. This *in vitro*/*in* silico system, combining one or more devices described herein with an integrated PK-PD modeling approach, can be used to predict drug toxicity in a more realistic manner than conventional *in vitro* systems. In some embodiments, one or more of the devices described herein can be used to quantify, estimate or gauge one or more physical-chemical, pharmacokinetic and/or pharmacodynamic parameters. Various physical-chemical, pharmacokinetic and pharmacodynamic parameters are known in the art, including, for example, the ones discussed in the aforementioned references. Exemplary physical-chemical, pharmacokinetic and pharmacodynamic parameters include, but are not limited to, permeability, logP, logD, volume of distribution, clearances (including intrinsic clearances), absorption rates, rates of metabolism, exchange rates, distribution rates and properties, excretion rates, IC50, binding coefficients, etc.

In some embodiments, the devices described herein can be cultured with animal cells (*e.g.,* but not limited to, pig cells, rabbit cells, dog cells, mouse cells, and/or rat cells) to determine response of the animal cells to an agent introduced into the devices described herein. The measured response of the animal cells in the devices can then be correlated with the actual response occurred *in vivo* when the agent is administered to a living animal (*e.g.,* a pig, a rabbit, a dog, a mouse, and/or a rat). By identifying the correlation between the *in vitro* and *in vivo* responses in one or more animal models, one can extrapolate or predict effect of the agent on a human subject *in vivo,* based on the measured responses of the human cells to the agent in the devices. Additionally or alternatively, a therapeutic dose of an agent for a human subject can be determined accordingly.

The devices described herein can have many different applications including, but not limited to, cell differentiation, formation of a stratified and/or three-dimensional tissue structure, development of a disease model in liverstudies of mechanism of action of drugs, target identification and/or validation, identification of markers of disease; assessing pharmacokinetics and/or pharmacodynamics of various chemical or biological agents; assessing efficacy of therapeutics and/or vaccines; testing gene therapy vectors; drug and/or vaccine development; molecule or drug screening or drug discovery; determination of an appropriate treatment or drug for a specific patient population or individual patient; identification of a risk population to a disease or disorder; identification of a new drug target for a patient population that is non-responsive to a previously-administered treatment; studies of cell behavior in a physiologically-relevant liver model; studies on biotransformation, absorption, clearance, metabolism, and activation of xenobiotics; studies on bioavailability and transport of chemical or biological agents across epithelial or endothelial layers; studies on transport of biological or chemical agents across the liver epithelial barrier; studies on acute basal toxicity of chemical agents; studies on acute local or acute organ-specific toxicity of chemical agents; studies on chronic basal toxicity of chemical agents; studies on chronic local or chronic organ-specific toxicity of chemical agents; studies on teratogenicity of chemical agents; studies on genotoxicity, carcinogenicity, and/or mutagenicity of chemical agents; detection of infectious biological agents and/or biological weapons; detection of harmful chemical agents and chemical weapons; studies on infectious diseases (*e.g*., bacterial, viral and/or fungal infections); assessing infectivity and/or virulence of a new strain; studies on the optimal dose range of a chemical and/or biological agent to treat a disease; prediction of the response of an organ *in vivo* exposed to a biological and/or chemical agent; studies concerning the impact of genetic content on response to agents; studies on gene transcription in response to chemical or biological agents; studies on protein expression in response to chemical or biological agents; studies on changes in metabolism in response to chemical or biological agents; as well as example uses described below. The devices described herein can also be used to screen on the cells, for an effect of the cells on the materials (for example, in a manner equivalent to tissue metabolism of a drug).

The devices described herein can also allow different growth factors, chemicals, gases and nutrients to be added to different cell types according to the needs of cells and their existence *in vivo.* Controlling the location of those factors or proteins can direct the process of specific cell remodeling and functioning, and also can provide the molecular cues to the whole system, resulting in such beneficial developments as neotissue, cell remodeling, enhanced secretion, and the like.

In yet another teaching, the devices described herein can be utilized as multi cell type cellular microarrays, such as microfluidic devices. Using the devices described herein, pattern integrity of cellular arrays can be maintained. These cellular microarrays can constitute the "lab-on-a-chip", particularly when multiplexed and automated. These miniaturized multi cell type cultures will facilitate the observation of cell dynamics with faster, less noisy assays, having built-in complexity that will allow cells to exhibit *in vivo*-like responses to the array.

In yet another teaching, the devices described herein can be utilized as biological sensors. Cell-based biosensors can provide more information than other biosensors because cells often have multifaceted physiological responses to stimuli, as well as novel mechanisms to amplify these responses. All cell types in the devices described herein can be used to monitor different aspects of an analyte at the same time; different cell type in the devices described herein can be used to monitor different analytes at the same time; or a mixture of both types of monitoring. Cells ranging from E. coli to cells of mammalian lines have been used as sensors for applications in environmental monitoring, toxin detection, and physiological monitoring.

In some embodiments, the devices described herein can be utilized in an overall system incorporating sensors, computers, displays and other computing equipment utilizing software, data components, process steps and/or data structures.

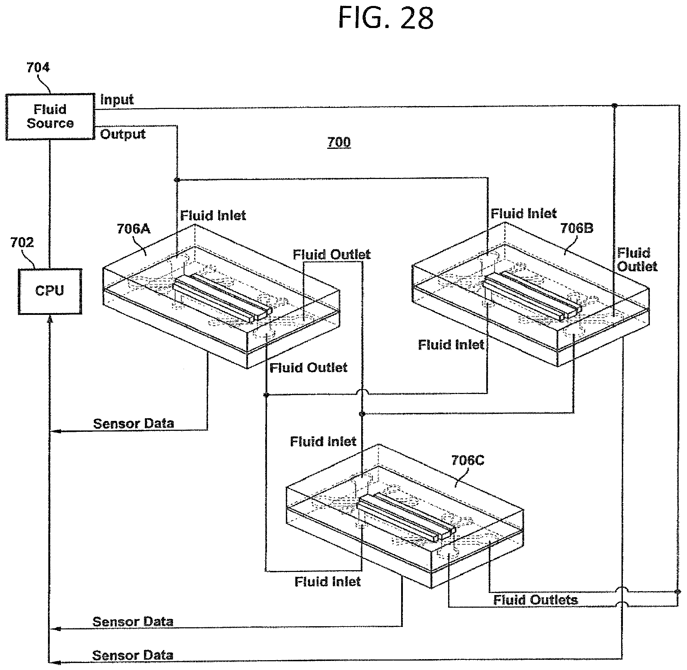

**Fig. 28** illustrates a schematic of a system having at least one device in accordance with an embodiment described herein, *e.g*., 706A, fluidically connected to another device described herein, *e.g.,* 706B, and/or any cell culture device known in the art, *e.g.,* an art-recognized organ-on-a-chip, *e*.*g*., 706C. As shown in **Fig. 28**, the system 700 includes one or more CPUs 702 coupled to one or more fluid sources 704 and external force sources (*e.g*., pressure sources) (not shown), whereby the preceding are coupled to three devices 706A, 706B, and 706C. It should be noted that although three devices 706 are shown in this embodiment, fewer or greater than three devices 706 can be used. In the system 700, two of the three devices (i.e. 706A and 706B) are connected in parallel with respect to the fluid source 704 and devices 706A and 706C are connected in serial fashion with respect to the fluid source 704. It should be noted that the shown configuration is only one example and any other types of connection patterns can be utilized depending on the application. In some embodiments, a system can be the one described in the International Patent Application No. PCT/US12/68725, entitled "Integrated human organ-on-chip microphysiological systems," where one or more devices described herein can be fluidically connected to form the system.

In the example shown, fluid from the fluid source 704 is provided directly to the fluid inlets of devices 706A and 706B. As the fluid passes through device 706A, it is output directly into the fluid inlet port of devices 706B and 706C. Additionally, the fluid outlet from device 706B is combined with the output from device 706A into device 706C. With multiple devices operating, it is possible to monitor, using sensor data, how the cells in the fluid or membrane behave after the fluid has been passed through another controlled environment. This system thus allows multiple independent "stages" to be set up, where cell behavior in each stage can be monitored under simulated physiological conditions and controlled using the devices 706. One or more devices are connected serially can provide use in studying chemical communication between cells. For example, one cell type can secrete protein A in response to being exposed to a particular fluid, whereby the fluid, containing the secreted protein A, exits one device and then is exposed to another cell type specifically patterned in another device, whereby the interaction of the fluid with protein A with the other cells in the other device can be monitored (*e.g*. paracrine signaling). For the parallel configuration, one or more devices connected in parallel can be advantageous in increasing the efficiency of analyzing cell behavior across multiple devices at once instead of analyzing the cell behavior through individual devices separately.

For example, as illustrated in **Fig. 6A**, an organ-on-a-chip device (*e.g.,* as described in the U.S. Patent No. 8,647,861, and in the International Patent App. No. PCT/US2014/071611) can be coupled to a liver-on-a- chip device described herein. A drug introduced into the organ-on-a-chip device can exit the device via a culture medium exiting the organ-on-a-chip device and be directed to the liver-on-a-chip device where hepatocytes can be exposed to the drug. Thus, drug metabolism can be determined, *e.g*., by measuring the activity of CYP450 drug metabolizing enzyme and/or the level of metabolites converted from the drug.

The drug(s) can be introduced into the devices as aerosols and/or by culture medium. By way of example only, as shown in **Fig. 6A**, an aerosol drug can be introduced to the "air" channel" containing lung epithelial cells on one side of the membrane. The other side of the membrane facing the "vascular" channel can be lined with endothelial cells. The aerosol drug diffuses to "vascular" channel across the lung epithelial cells and endothelial cells. The drug-containing fluid flowing through the "vascular" channel can then be directed to hepatocytes in the liver-on-a-chip device, where the activity of CYP3A4 drug metabolizing enzyme activity induced by the aerosol drug can be measured.

### Endothelial cell culture media and methods of using the same

Not only have the inventors created a device for simulating a function of a liver tissue, the inventors have also developed a novel endothelial cell culture medium for long term culture of endothelial cells and/or more importantly for long term co-culture of endothelial cells and epithelial cells from different tissue origins. Accordingly, in one teaching, an endothelial cell culture medium that provides longer viability of endothelial cells than existing media (*e.g.,* by at least about 2 weeks or longer, including, *e.g.,* at least about 3 weeks, at least about 4 weeks or longer) is described herein.

As used herein, the term "medium" or "media", when used in reference to endothelial cell culture media, refers to a basal medium or media for culturing cells and containing nutrients that maintain cell viability and support proliferation. The cell culture basal medium can contain any of the following in an appropriate combination: salt(s), buffer(s), amino acids, glucose or other sugar(s), antibiotics, and other components such as peptide growth factors, etc. Cell culture media ordinarily used for particular cell types are known to those skilled in the art. Examples of cell culture medium include Minimum Essential Medium (MEM), Eagle's Medium, Dulbecco's Modified Eagle Medium (DMEM), Dulbecco's Modified Eagle Medium: Nutrient Mixture F-12 (DMEM F12), FIO Nutrient Mixture, Ham's FlO Nutrient Mix, Ham's F12 Nutrient Mixture, Medium 199, RPMI, RPMI 1640, reduced serum medium, basal medium (BME), DMEM/F12 (1:1), and the like, and combinations thereof. The cell culture basal medium or media can be modified by adding one or more factors or components to suit the need of different applications. For example, to make the endothelial cell culture media described herein, the cell culture basal medium or media can further comprise, essentially consist of, or consist of hEGF, hydrocortisone, hFGF-beta, R3-IGF-1, ascorbic acid, heparin, and serum.

In some embodiments, the endothelial cell culture medium can be in a form of powder (*e.g*., lyophilized powder). The powder can be reconstituted in an aqueous solution (*e.g*., water) upon use to reach the desired concentrations as described herein.

The concentrations of each individual component described are generally the working concentrations for the culturing methods of various teachings described herein. In some embodiments, the concentrations of each individual component in the endothelial cell culture media can be increased, *e.g.,* by 2-fold or more, including, *e.g.,* 3-fold, 4-fold, 5-fold, 6-fold, 7-fold, 8-fold, 9-fold, 10-fold, or higher to create a concentrated endothelial cell culture media. A user can dilute the concentrated endothelium cell culture media to the working concentrations with an aqueous solution (*e.g*., sterilized water) upon use. Accordingly, concentrated endothelial cell culture media are also provided herein.

### Kits

Kits for cell culture are also described herein. In one teaching, the kit comprises (a) a cell culture device; and (b) an endothelial cell culture medium according to one or more embodiments described herein.

As used herein, the term "cell culture device" refers to a device or apparatus suitable for culturing cells. Examples of cell culture devices include, but are not limited to, Transwell systems, plates, microwells, bioreactors, microfluidic devices, and any combinations thereof. In some embodiments, the cell culture device provided in the kit can be a device for simulating a function of a tissue, which comprises (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber. In some embodiments, the first side or the second side of the membrane can comprise endothelial cells adhered thereon. In some embodiments, the first side or the second side of the membrane can comprise tissue-specific cells adhered thereon. Tissue-specific cells can be cells derived from any tissue or organ of interest, including, *e.g*., but not limited to a lung, a liver, a kidney, a skin, an eye, a brain, a blood-brain-barrier, a heart, a gastrointestinal tract, airways, a reproductive organ, and a combination of two or more thereof.

In some embodiments, the basal medium and the supplement cocktail provided in the kit can be pre-mixed to form the endothelial cell culture medium.

In some embodiments, the basal medium and the supplement cocktail provided in the kit can be separately packaged. For example, the basal medium and the supplement cocktail can be packaged independently, *e.g*., as powder or liquid.

In some embodiments where the basal medium and/or the supplement cocktail are in a form of powder, *e.g*., lyophilized powder, the powder can be reconstituted upon use. In some embodiments, the basal medium and/or the supplement cocktail can be in a form of liquid.

In some embodiments, the kit can further comprise at least one vial of cells. In one embodiment, the cells can be endothelial cells. In one embodiment, the cells can be tissue-specific cells.

### Some selected definitions

Unless defined otherwise, all technical and scientific terms used herein have the same meaning as commonly understood by one of ordinary skill in the art to which this invention belongs.

It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims.

Other than in the operating examples, or where otherwise indicated, all numbers expressing quantities of ingredients or reaction conditions used herein should be understood as modified in all instances by the term "about." The term "about" when used to describe NOT described the present invention, in connection with percentages means ±5%.

In one teaching, the present invention relates to the herein described compositions, methods, and respective component(s) thereof, as essential to the invention, yet open to the inclusion of unspecified elements, essential or not ("comprising"). In some embodiments, other elements to be included in the description of the composition, method or respective component thereof are limited to those that do not materially affect the basic and novel characteristic(s) of the invention ("consisting essentially of"). This applies equally to steps within a described method as well as compositions and components therein. In other embodiments, the inventions, compositions, methods, and respective components thereof, described herein are intended to be exclusive of any element not deemed an essential element to the component, composition or method ("consisting of").

As used herein, the term "co-culture" refers to two or more different cell types being cultured in some embodiments of the devices described herein. The different cell types can be cultured in the same chamber (*e.g*., first chamber or second chamber) and/or in different chambers (*e.g*., one cell type in a first chamber and another cell type in a second chamber). For example, in some embodiments, the devices described herein can have hepatocytes in the first chamber and endothelial cells in the second chamber.

As used herein, the term "tri-culture" refers to three different cell types being cultured in some embodiments of the microfluidic devices described herein. The different cell types can be cultured in the same chamber (*e.g*., first chamber or second chamber) and/or in different chambers (*e.g*., one cell or two types in a first chamber and one or two cell types in a second chamber that are different than in the first chamber). For example, in some embodiments, the microfluidic devices described herein can have hepatocytes in the first chamber, endothelial cells in the second chamber and Kupffer cells added to the second chamber.

As used herein, the term "quad-culture" or "quadruple-culture" refers to four different cell types being cultured in some embodiments of the microfluidic devices described herein. The different cell types can be cultured in the same chamber (*e.g*., first chamber or second chamber) and/or in different chambers (*e.g*., one or two or three cell types in a first chamber and one or two or three cell types in a second chamber, where each cell type is different than in the first chamber). For example, in some embodiments, the microfluidic devices described herein can have hepatocytes in the first chamber, endothelial cells in the second chamber with Kupffer cells and Stellate cells added to the second chamber.

The term "statistically significant" or "significantly" refers to statistical significance and generally means a two standard deviation (2 SD) below normal, or lower, concentration of the marker. The term refers to statistical evidence that there is a difference. It is defined as the probability of making a decision to reject the null hypothesis when the null hypothesis is actually true. The decision is often made using the p-value.

A "marker" as used herein is used to describe the characteristics and/or phenotype of a cell. Markers can be used for selection of cells comprising characteristics of interests. Markers will vary with specific cells. Markers are characteristics, whether morphological, functional or biochemical (enzymatic) characteristics of the cell of a particular cell type, or molecules expressed by the cell type. In some embodiments, such markers are proteins, and possess an epitope for antibodies or other binding molecules available in the art, and thus can be monitored by FACs analysis, and immunocytochemistry. However, a marker may consist of any molecule found in a cell including, but not limited to, proteins (peptides and polypeptides), lipids, polysaccharides, nucleic acids and steroids. Examples of morphological characteristics or traits include, but are not limited to, shape, size, and nuclear to cytoplasmic ratio. Examples of functional characteristics or traits include, but are not limited to, the ability to adhere to particular substrates, ability to incorporate or exclude particular dyes, ability to filtrate particles, ability to migrate under particular conditions, and the ability to differentiate along particular lineages. Markers may be detected by any method available to one of skill in the art, including for example, detection of nucleic acid, *e.g.* mRNA, *e.g.* by quantitative PCR.

As used interchangeably herein, the term "substantially" means a proportion of at least about 60%, or preferably at least about 70% or at least about 80%, or at least about 90%, at least about 95%, at least about 97% or at least about 99% or more, or any integer between 70% and 100%. In some embodiments, the term "substantially " means a proportion of at least about 90%, at least about 95%, at least about 98%, at least about 99% or more, or any integer between 90% and 100%. In some embodiments, the term "substantially" can include 100%.

Kupffer cells, also known as Browicz-Kupffer cells, and stellate macrophages, are specialized macrophages located in the liver lining the walls of the sinusoids that form part of the reticuloendothelial system (RES) (or mononuclear phagocyte system).

It is not intended that the present invention be limited by the nature of the "microfluidic device." However, preferred microfluidic devices are described in U.S. Patent No. 8,647,861, and they are microfluidic "organ-on-chip" devices comprising living cells in microchannels, e.g. cells on membranes in microchannels exposed to culture fluid at a flow rate. Flow is important and stands in contrast to static 2D culture. Using a flow in the microchannel(s) allows for the perfusion of cell culture medium throughout the cell culture during in vitro studies and as such offer a more in vivo-like physical environment. In simple terms, an inlet port allows injection of cell culture medium into a cell-laden microfluidic channel or chamber, thus delivering nutrients and oxygen to cells. An outlet port then permits the exit of remaining medium as well as harmful metabolic by-products.

Microfluidic devices are conveniently made of polydimethylsiloxane (PDMS), polyurethane, polyimide, styrene-ethylene-butylene-styrene (SEBS), polypropylene, or any combinations thereof. The present invention contemplates treatment of such substances to promote wetting such as treatments selected from the group consisting of plasma treatment, ion treatment, gas-phase deposition, liquid-phase deposition, adsorption, absorption or chemical reaction with one or more agents.

Additionally, the term "microfluidic" as used herein relates to components where moving fluid is constrained in or directed through one or more channels wherein one or more dimensions are 1 mm or smaller (microscale). Microfluidic channels may be larger than microscale in one or more directions, though the channel(s) will be on the microscale in at least one direction. In some instances, the geometry of a microfluidic channel may be configured to control the fluid flow rate through the channel (e.g. increase channel height to reduce shear). Microfluidic channels can be formed of various geometries to facilitate a wide range of flow rates through the channels.

"Channels" are pathways (whether straight, curved, single, multiple, in a network, etc.) through a medium (e.g., silicon) that allow for movement of liquids and gasses. Channels thus can connect other components, i.e., keep components "in communication" and more particularly, "in fluidic communication" and still more particularly, "in liquid communication." Such components include, but are not limited to, liquid-intake ports and gas vents. Microchannels are channels with dimensions less than 1 millimeter and greater than 1 micron.

As used herein, the phrases "connected to," "coupled to," and "in communication with" refer to any form of interaction between two or more entities, including mechanical, electrical, magnetic, electromagnetic, fluidic, and thermal interaction. For example, in one embodiment, first and second channels in a microfluidic device are in fluidic communication with a fluid reservoir. Two components may be coupled to each other even though they are not in direct contact with each other. For example, two components may be coupled to each other through an intermediate component (e.g. tubing or other conduit).

The surfaces of the microchannels and/or the membrane can be coated with cell adhesive molecules to support the attachment of cells and promote their organization into tissues. Where a membrane is used, tissues can form on either the upper surface, the lower surface or both. In one embodiment, different cells are living on the upper and lower surfaces, thereby creating one or more tissue-tissue interfaces separated by the membrane. The membrane may be porous, flexible, elastic, or a combination thereof with pores large enough to only permit exchange of gases and small chemicals, or large enough to permit migration and transchannel passage of large proteins, as well as whole living cells and/or portions thereof.

All patents, patent applications, and publications identified are expressly incorporated herein by reference for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

### EXAMPLES

The following examples illustrate some embodiments and aspects described herein. Any examples, which do not fall under the literal scope of the appended claims are provided by way of background reference only.

### Example 1. Human liver-on-a-chip device design 1 and characterization

Hepatocytes, unlike other cell types, do not simply adhere to a surface and proliferate upon thawing. To identify suitable cell sources, cryopreserved vials were evaluated for their adhesion ability, their ability to retain their *in vivo* like morphology, and/or their ability to form bile canaliculi network (for biliary clearance determination). Typically, in 2D static culture hepatocytes flatten out and lose the cobblestone morphology.

After identifying suitable cell sources of hepatocytes, the hepatocytes were cultured on a first side of the membrane (coated with collagen I at approx.. 100 ug/mL) facing a first chamber of the device, *e.g.,* as shown in **Fig. 2B** or **Fig. 7B**, to permit growth of a confluent monolayer within the first chamber. **Fig. 3A** is a set of images showing hepatocytes (top panel) and hLSECs (bottom panel) from Day 13. It is noted that the cells were able to continue to grow for at least about 3 weeks or longer. One of the striking things observed is that this morphology was still observed after 3 weeks, which is not normally seen in static culture. In static culture the cells generally begin to deteriorate after about 1 week.

To assess whether the cells formed bile canaliculi, a live stain for using CDFDA (a marker that is excreted into the bile via efflux transporters) was performed. The stain (**Fig. 3A** top panel) showed that active transport occurred between the hepatocytes growing in the devices described herein.

Human liver sinusoidal endothelial cells (LSECs) were cultured on a second side of the membrane facing a second chamber of the device. The LSECs were stained for CD32b (an endothelial cell marker) that is normally expressed by human LSECs, and the image was shown in **Fig. 3A**, bottom panel. A flow of cell culture medium was also introduced into the first chamber and the second chamber at different flow rates to subject the cells to appropriate shear stress. For example, the hepatocytes were exposed to a shear stress of about 0.025 dyne/cm², and the LSECs a shear stress of about 0.05 dynes/cm².

After the cells were determined to maintain proper morphology in the devices described herein, cell viability was assessed over time. Cell viability can be measured by any known methods in the art. For example, lactate dehydrogenase (LDH) levels can be measured to assess cell viability. LDH is an enzyme found in all cells. When a cell is damaged and the membrane is compromised or becomes non-intact (*e.g*., with a break in the membrane), LDH is released into the culture media and thus the culture media can be collected and measured for LDH content. **Fig. 3B** shows that cells cultured in one embodiment of the ***liver-on-a-chip*** device described herein (also referred to as "liver chip" herein) released significantly lower levels of LDH than cells in static culture, indicating that subjecting the cells to a low shear stress (*e.g*., a fluid flow) can facilitate maintenance of cell viability.

Next, the device was evaluated to determine if it could also exhibit or retain normal liver function. To assess the normal function of the liver chip, in one embodiment, albumin secretion (normally measured from blood of a subject) was measured from the culture medium flowing in the LSEC chamber. A reduced level in albumin secretion is indicative of liver injury. **Fig. 4A** shows that albumin secretion was maintained in the liver chip for at least 2 weeks or longer and was significantly higher than albumin secretion measured in conventional static culture.

It was next sought to determine function of the liver chip for drug metabolism. To this end, CYP3A4 was measured because it is the major P450 drug metabolizing enzyme in the liver. **Fig. 4B** shows that the liver chip's basal CYP3A4 enzyme activity levels were at least 4 times greater than the level in static culture. While these levels can vary greatly from donor to donor, the basal CYP3A4 level observed in the liver chip was comparable to what is observed *in vivo.*

Induction of CYP3A4 activity in the liver is one of main causes of drug-drug interactions, which is a mechanism to defend against exposure to drugs and toxin, but can also lead to unwanted side-effects (toxicity) or change the efficacy of a drug. **Fig. 5** illustrates that when taking two drugs that interact with the same enzyme (in this case CYP3A4), Drug A can increase the metabolism of Drug B or decrease the efficacy of drug B. For example, Drug A can bind the nuclear receptor PXR in the hepatocytes and cause upregulation of the CYP3A4 enzyme, which will in turn increase metabolite formation of Drug B and thus reduce the level of Drug B in circulation and its efficacy. In addition, the increased metabolite formation of Drug B can increase toxic metabolites.

Physiological coupling between two devices, each simulating a function of a different tissue, was also evaluated. In one embodiment, measurement of CYP3A4 activity was used to determine physiological coupling between different organ chips. **Fig. 6A** is a schematic diagram showing physiological coupling between a lung chip (*e.g.,* as described in the U.S. Patent No. 8,647,861) and a liver chip for a period of time (*e.g*., about 1 week). Rifampicin was administered to the "air" channel" at a dose of about 50 µM (*e.g*., determined by mass spectrometry). In some embodiments, rifampicin can be administered to the "air" channel at a concentration of about 10 µM. The rifampicin level measured in the "vascular" channel across the lung epithelial cells and endothelial cells was about 1.4 µM, indicating that the lung endothelial cells formed a functional barrier. The rifampicin-containing fluid flowing through the "vascular" channel was then directed to the hepatocytes in the liver chip. A 2 fold increase in CYP3A4 drug metabolizing enzyme activity induced by rifampicin was detected, as compared to control without rifampicin (**Fig. 6B**). According to FDA guidelines, a 2-fold induction in CYP3A4 activity should be observed for drug-drug interaction.

### Example 2. Human liver-on-a-chip device design 2 and characterization

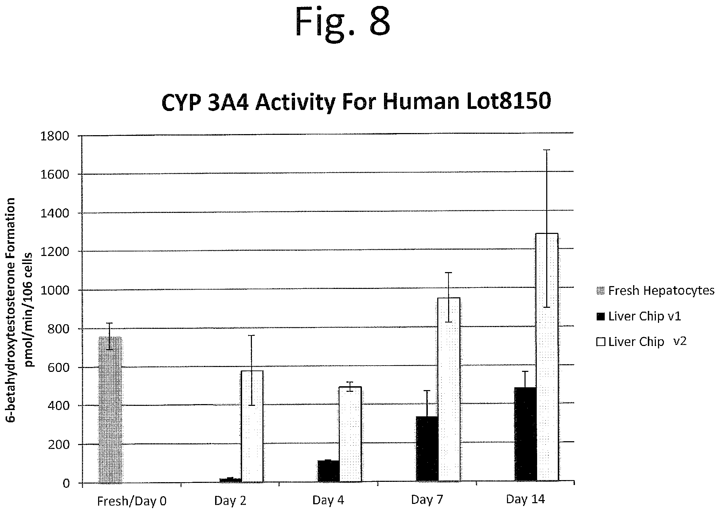

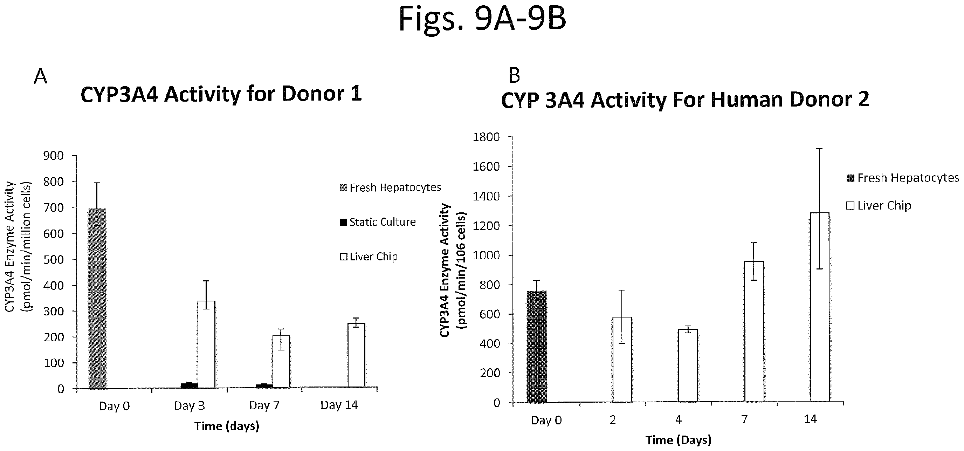

As hepatocytes are much more cuboidal than epithelial cells of other tissue, *e.g*., alveolar flat cell type, the height of the chamber in which the hepatocytes are cultured can be increased to accommodate the cell size as well as the width so that much more confluent monolayers of hepatocytes can be achieved. For example, in one embodiment, the hepatocytes can be cultured in the top chamber of the device shown in **Fig. 7B****.** As described in Example 1, CYP3A4 activity of the hepatocytes growing in the device of **Fig. 7B** (referred to as "Liver chip v2") was measured over a period of time, and compared to the cells grown in the device of **Fig. 7A** (referred to as "Liver chip v1 "). **Fig. 8** shows that CYP3A4 activity levels steadily increased in Liver chip v1, while the Liver chip v2 showed comparable activity levels to fresh hepatocytes and the CYP3A4 activity was maintained in Liver chip v2 for at least about 2 weeks. Similarly, hepatocytes collected from human subjects (Donor 1 and Donor 2) exhibited *in vivo*-relevant CYP3A4 levels for at least about 2 weeks when they were cultured in the liver chips, as compared to static cultures (**Figs. 9A-9B**)**.** Indeed, the CYP3A4 levels measured in the liver chip described herein was also magnitudes higher than the CYP3A4 activity measured in the existing human liver model (*e.g.,* 3D InSight^{™} human liver microtissues from InSphero).

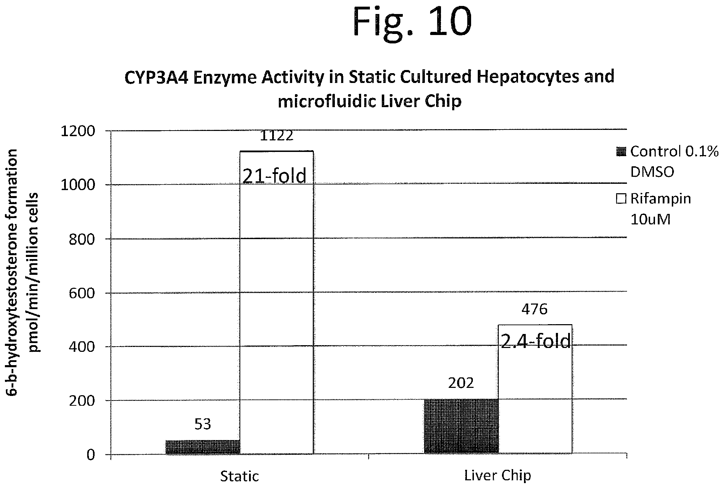

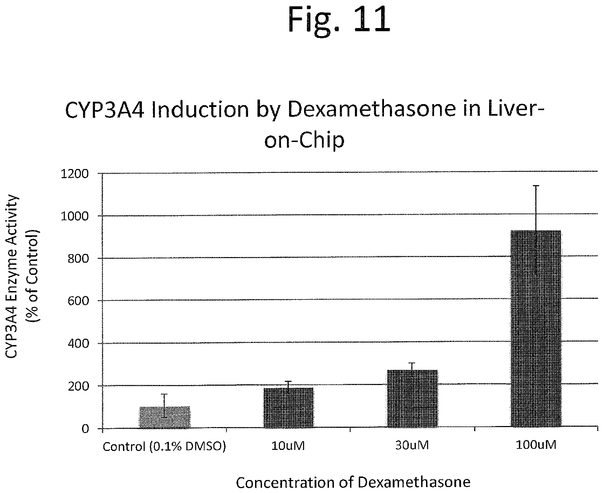

To assess the drug-drug interaction in the liver chip, hepatocytes were cultured for about 2 days in the top chamber of the liver chip and then treated with rifampin and testosterone for about 3 days. Rifampin is known to induce the level of CYP3A4 activity in hepatocytes, which in turn increases the metabolism of testosterone to 6-b-hydroxytestosterone. Thus, CYP450 activity was reflected by measuring formation of 6-b-hydroxytestosterone turnover (from testosterone) and the readout was detected by mass spectrometry. A high level of 6-b-hydroxytestosterone is indicative of a high CYP3A4 activity. **Fig. 10** shows that basal levels of CYP3A4 activity were higher in the liver chip than in static Matrigel^{®} sandwich culture, the industry "gold standard." A robust induction of about 2-fold increase in the CYP3A4 activity in the liver chip (despite PDMS absorption) should enable drug-drug interaction studies. The liver chip was further validated by showing CYP3A4 enzyme activity response to dexamethasone (anti-inflammatory molecule) at various concentrations indicated in **Fig. 11. Fig. 11** shows that induction of CY3A4 activity in the human liver chip increased with concentrations of dexamethasone.

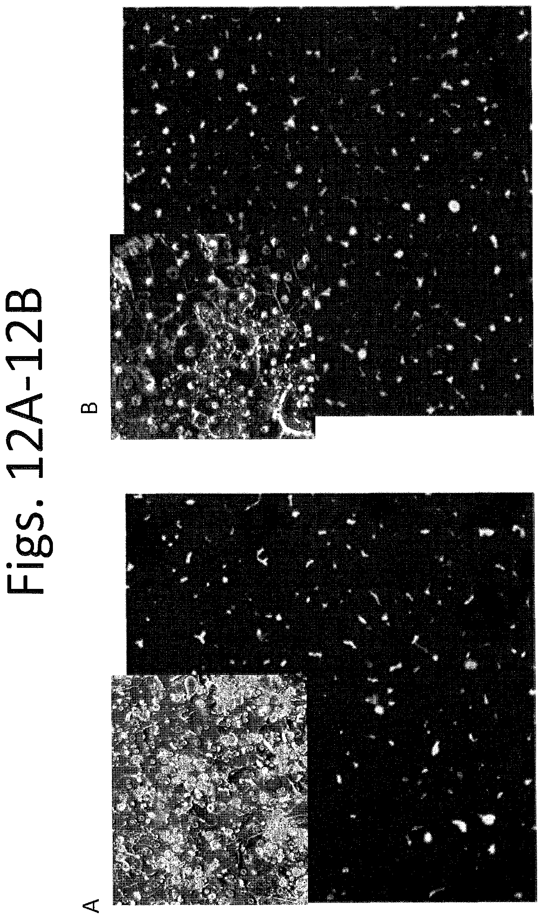

To assess whether the cells formed bile canaliculi, a live stain for using CDCFDA (a marker that is excreted into the bile via efflux transporters) was performed. The stains (**Figs. 12A-12B**) showed increased bile canaliculi active transport by multidrug resistance-associated protein 2 (MRP2) in the liver chip, as compared to static culture. Thus, increased formation of bile canaliculi in the liver chip was observed, as compared to the static culture.

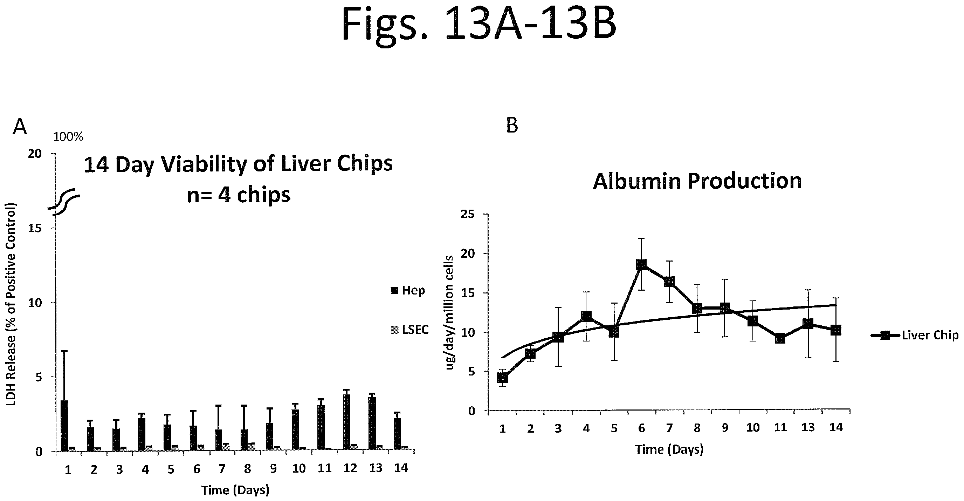

The inventors have also showed that hepatocytes and hLSECs maintained viable and functional in the liver chip for at least about 2 weeks. Cell viability of hepatocytes and hLSECs over time were determined by measuring lactate dehydrogenase (LDH) levels every day. As discussed in Example 1, when a cell is damaged and the membrane is compromised or becomes non-intact (*e.g*., with a break in the membrane), LDH is released into the culture media and thus the culture media can be collected and measured for LDH content. **Fig. 13A** shows that both hepatocytes and hLSECs maintained low LDH release levels over a course of at least about 2 weeks, indicating viability and health of the hepatocytes and hLSEC monolayer being maintained over a period of at least 2 weeks. The initial increase in LDH release by the hepatocytes as shown at Day 1 can be due to the stress of the liver chips being connected to pumps and cells being exposed to fluid flow. **Fig. 13B** showed measurement of albumin production over time, indicating that albumin secretion was maintained in the liver chip for at least 2 weeks or longer.

### Example 3. Rat liver-on-a-chip device design and characterization

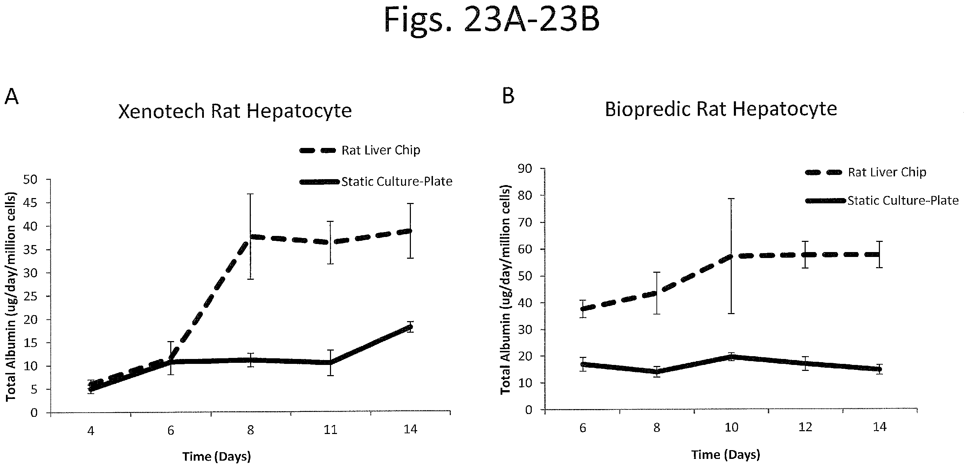

Similar to development of human ***liver-on-a-chip*** devices as described in Examples 1-2, suitable cell sources of rat hepatocytes and rat liver sinusoidal endothelial cells (rLSECs) were first identified. Cryopreserved vials were evaluated for their adhesion ability, their ability to retain their *in vivo* like morphology, and/or their ability to form bile canaliculi network (for biliary clearance determination). Rat hepatocytes from Xenotech Lot: XT1310140 and Biopredic Lot: HEP139031-T and rLSECs from Cell Biologics Lot: R1072 were selected to develop rat ***liver-on-a-chip*** devices. See **Figs. 23A-23B** for characterization of hepatic function of rat hepatocytes obtained from XenoTech and Biopredic.

In order to achieve long-term maintenance (*e.g*., viability and function) of a co-culture model of rat hepatocytes and rLSECs in a device as shown in **Fig. 7B**, *e.g.,* for at least two weeks or longer, the inventors have identified four optimization parameters, namely (1) substrate surface treatment (*e.g*., PDMS surface treatment); (2) extracellular matrix (ECM) coating; (3) ECM overlay on top of hepatocytes and (4) culture medium composition (**Fig.** 14A).

***Substrate surface treatment (e.g., PDMS surface treatment*):** When a surface in contact with cells and/or culture medium is hydrophobic, the surface is desired to be treated such that it becomes more hydrophilic. In some embodiments, a hydrophobic surface (*e.g*., PDMS surface) can be subjected to APTES treatment and/or plasma treatment. APTES (3-Aminopropyl)triethoxysilane is an aminosilane frequently used in the process of silanization, the functionalization of surfaces with alkoxysilane molecules. Plasma oxidation alters the surface chemistry of a hydrophobic surface (*e.g*., PDMS), adding silanol (SiOH) groups to the surface, and renders the hydrophobic surface (*e.g*., PDMS) hydrophilic (Lab Chip, 2010, 10, 548-552).

***ECM coating*:** To determine optimum condition for cell attachment, the surface-treated material (*e.g*., APTES-treated or plasma-treated PDMS) can be coated with an ECM coating of different extracellular matrix molecules at varying concentrations (based on the resulting cell morphology and attachment). For example, the ECM coating can comprise one of the following conditions shown in Table 1.

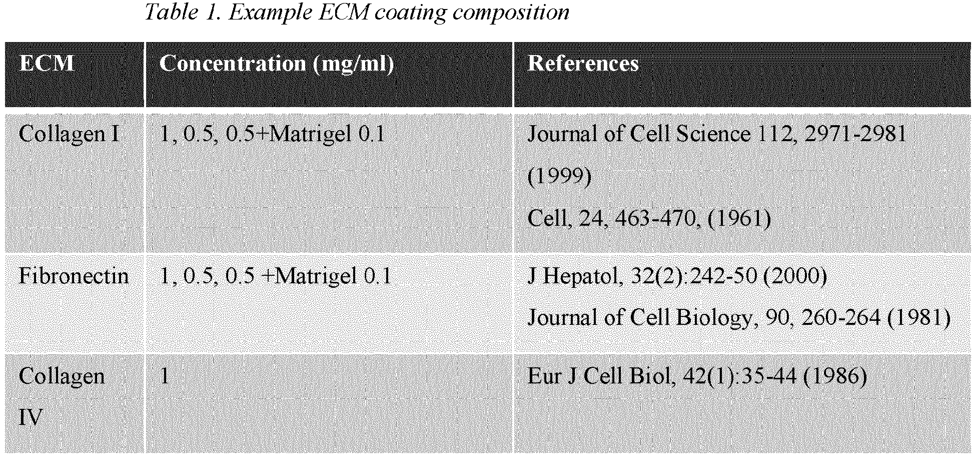

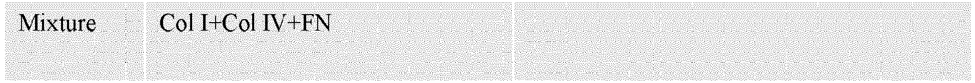

Hepatocytes were then cultured on the ECM coated surface for a period of time (*e.g.,* 5 days) and cell attachment was evaluated. It was determined that a plasma-treated PDMS surface coated with (i) an ECM mixture coating comprising fibronectin (~0.5 mg/mL), collagen IV (∼0.4 mg/mL), and collagen I (∼0.1 mg/mL); or (ii) an ECM coating of fibronectin at a concentration of about 0.5 mg/mL or higher, provided an optimum condition for hepatocyte attachment.

***ECM overlay*:** Similarly, it was determined that an ECM overlay mixture of Matrigel^{®} and fibronectin provide better cell morphology of hepatocytes than Matrigel^{®} overlay alone. In some embodiments, the ECM overlay mixture can have Matrigel^{®} at a concentration of about 0.25 mg/mL, and fibronectin at a concentration of about 0.02 mg/mL. In these embodiments, the ECM coating can have fibronectin at about 1 mg/mL.

***Culture medium composition:*** To determine optimum culture medium composition for rat hepatocytes, the cells were maintained in various culture medium compositions as shown in Table 2 and cell growth after a certain period of time were evaluated. In the experiment, the cells were adhered on an ECM coating of fibronectin at about 1 mg/mL.

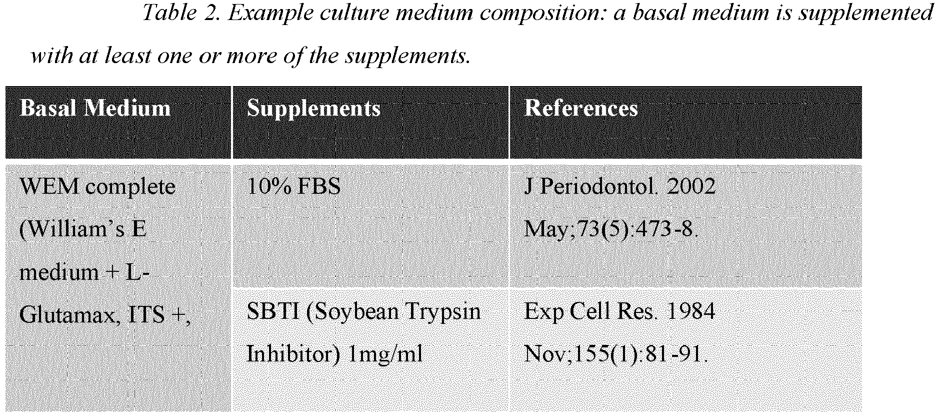

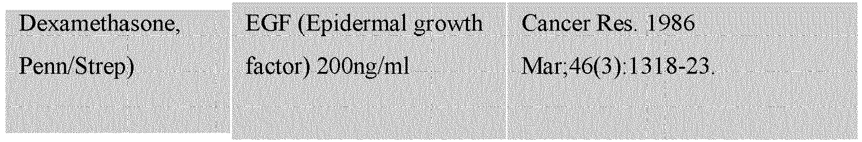

After 7 days of culture, it was determined that WEM complete basal medium supplemented with about 10% FBS provided a more confluent monolayer of hepatocytes and better cell morphology.

**Figs. 15A-15B** are images showing viable co-culture of rat hepatocytes on one side of the membrane facing the top chamber of the device (*e.g.,* as shown in Fig. 7B) and rLSECs on another side of membrane facing the bottom chamber of the device (in WEM plus supplements and 10% FBS). After two weeks, the co-culture remained viable. Prior to culturing hepatocytes, the PDMS surface was plasma-treated, followed by an ECM coating of fibronectin (higher than 0.5 mg/mL) or an ECM coating mixture of fibronectin, collagen IV and collagen I. The ECM coating was further overlaid with a mixture of Matrigel^{®} and fibronectin. The WEM complete basal medium supplemented with about 10% FBS was introduced into the chamber comprising the rat hepatocytes.

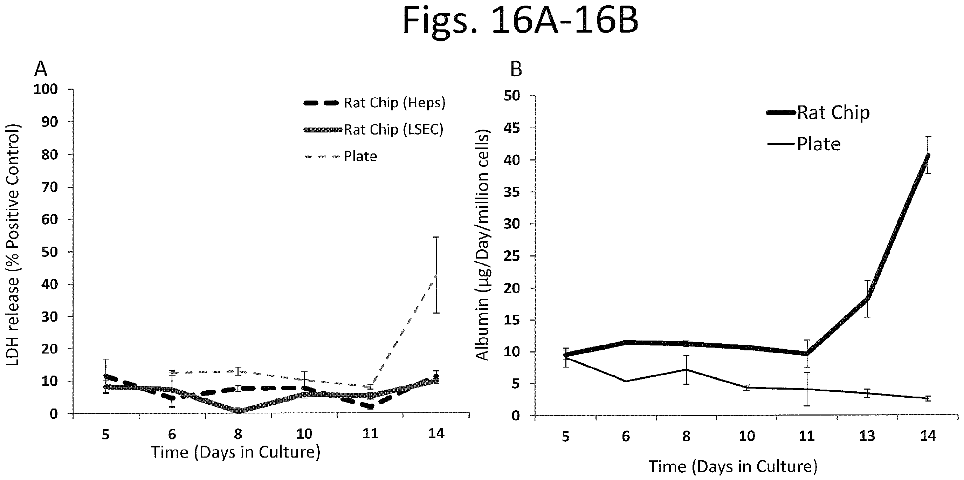

The inventors have also showed that rat hepatocytes and rLSECs maintained viable and functional in the rat liver chip for at least about 2 weeks or longer. Cell viability of rat hepatocytes and rLSECs over time were determined by measuring lactate dehydrogenase (LDH) levels over a period of time. As discussed in Example 1, when a cell is damaged and the membrane is compromised or becomes non-intact (*e.g*., with a break in the membrane), LDH is released into the culture media and thus the culture media can be collected and measured for LDH content. **Fig. 16A** shows that unlike static culture (*e.g*., plate culture), both rat hepatocytes and rLSECs maintained low LDH release levels over a course of at least about 2 weeks, indicating viability and health of the hepatocytes and rLSEC monolayer being maintained in the rat liver chip over a period of at least 2 weeks. **Fig. 16B** showed measurement of albumin production over time, indicating that albumin secretion in the rat liver chip was maintained for at least 2 weeks or longer, or even increased after two weeks.

Similar to **Fig. 16A****,** **Fig. 17C** showed improved viability of rat hepatocytes and rLSECs in the liver chip for a period of at least 4 weeks or longer than in a static culture, as evidenced by lower LDH release levels measured in the liver chip.

F-actin staining of the 4-week cultures shows cuboidal morphology of rat hepatocytes and pericanalicular distribution in rat liver chips but cell deterioration and detachment in static cultures (*e.g*., plates) (data not shown). In addition, multi drug resistance-associated protein 2 (MRP2) staining correctly expressed at the canalicular wall of rat hepatocytes in liver chips whereas static cultures (*e.g*., plates) show only background staining of dead cells (data not shown).

Next, to determine effects of serum on mRNA expression and CYP450 enzyme activity, rat hepatocytes were cultured in plate cultures with different concentrations of serum.

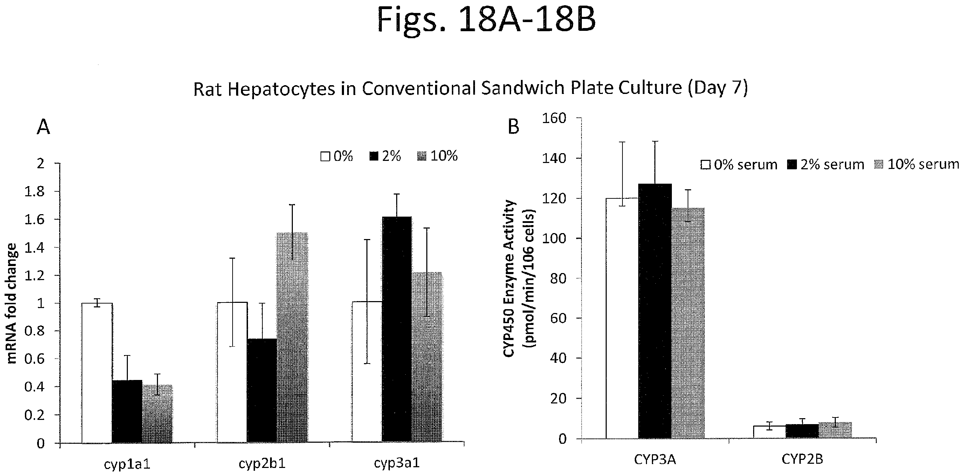

**Fig. 18A** shows that serum inhibited mRNA expression of cyp1a1, but no significant effect on cyp2b1 and cyp3a1. **Fig. 18B** shows that the activity of CYP3A and CYP2B are serum-independent, as different concentrations of serum showed no effect on CYP3A and CYP2B activity.

### Comparison of the rat liver chips described herein to static cultures and/or in vivo models

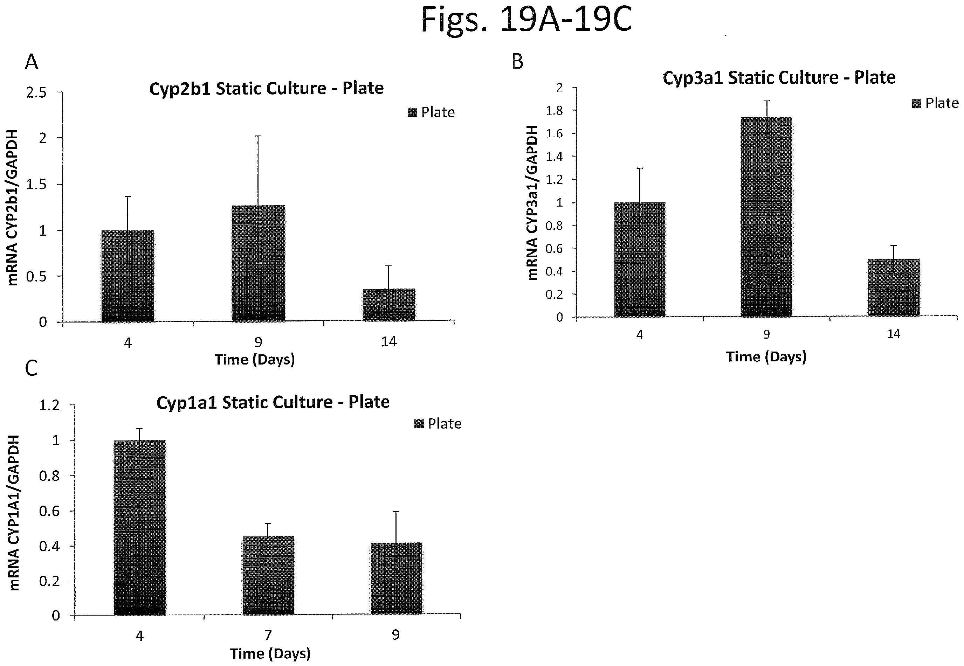

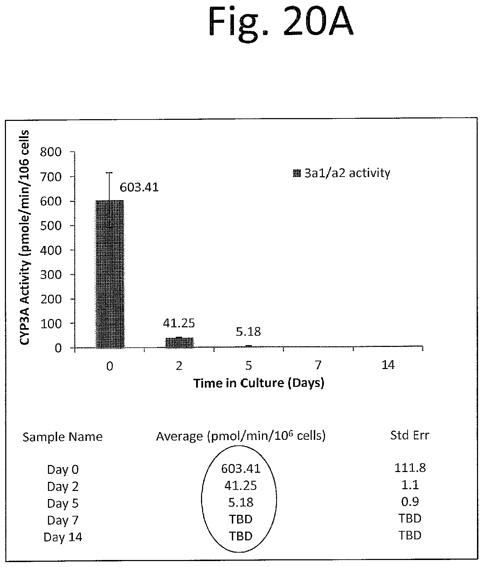

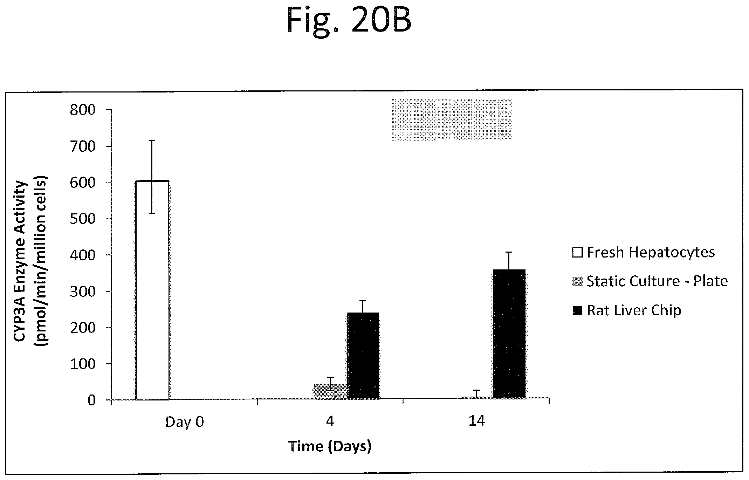

One of the limitations of the static cultures (*e.g.*, plates) of hepatocytes is that CYP mRNA expression (*e.g*., cyp2b1, cyp3a1, cyp1a1) in rat hepatocytes declined over time (**Figs. 19A-19C**). In addition, CYP3A1/A2 enzyme activity in rat hepatocytes also declined over time in static cultures (*e.g*., conventional sandwich plate culture) (**Fig. 20A****,** **Fig. 20B**). In contrast, rat liver chip shows improved CYP3A4 activity over static culture and the CYP3A4 activity was maintained in the liver chip for at least two weeks or longer (**Fig. 20B**).

In addition, the inventors measured one of the major functions of the liver - albumin secretion and other markers of liver injury such as liver transaminases (*e.g*., ALT and AST) in the rat liver chips. A low level of albumin is indicative of liver injury. **Fig. 21A** shows high levels of albumin secretion in the rat liver chip, whereas the static plate shows low levels of albumin, indicating that the rat liver chips had improved hepatocyte viability over the static cultures. In addition, the rat liver chips produced at least 5 times higher albumin than in a co-cultured liver bioreactor as described in Tsang et al. FASEB journal (2007) 21: 3790-3801. The Tsang reference shows that the co-cultured liver bioreactor produced around 8 µg of albumin per a million cells at Day 12 of culture, while the rat liver chips, as shown in **Fig. 21A**, produced about 40 µg of albumin per a million cells by Day 14 of culture.

Levels of alanine transaminase (ALT) and aspartate transaminase (AST) - indicators of severe liver injury- were also measured over a period of 22 days in the rat liver chip and the levels (**Figs. 21B-21C**, and **Table 3**) were within the normal range of ALT and AST in rat and human *in vivo* as shown in **Table 3** below. Thus, the rat liver chips can be used to evaluate hepatotoxic compounds by measuring the levels of albumin, ALT and/or AST.

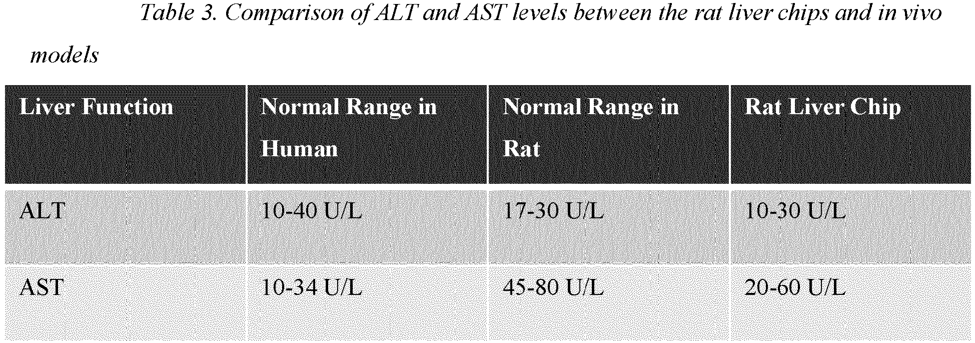

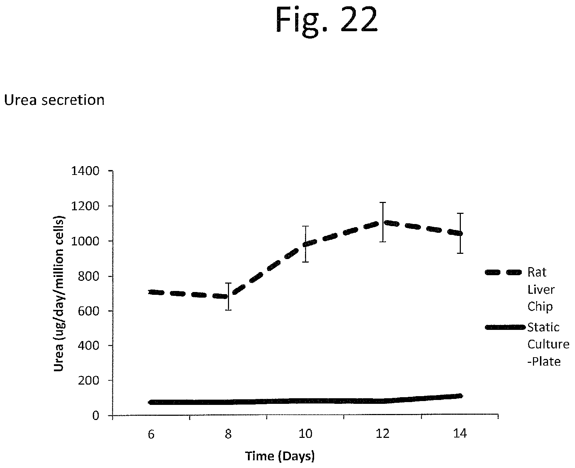

Another specific function of the liver is urea synthesis. If the urea level is high, this indicates healthy liver cells, whereas decreased levels indicate unhealthy liver cells. **Fig. 22** shows that urea levels were maintained in the rat liver chips for at least 2 weeks and the levels were higher than static cultures (*e.g.,* plates) and other existing *in vitro* liver models (*e.g.,* Hepregen).

### Example 4. Dog liver-on-a-chip device design and characterization

Similar to development of human and rat liver-on-a-chip devices as described in Examples 1-3, suitable cell sources of dog hepatocytes and dog liver sinusoidal endothelial cells (dLSECs) were first identified. Cryopreserved vials were evaluated for their adhesion ability, their ability to retain their *in vivo* like morphology, and/or their ability to form bile canaliculi network (for biliary clearance determination).

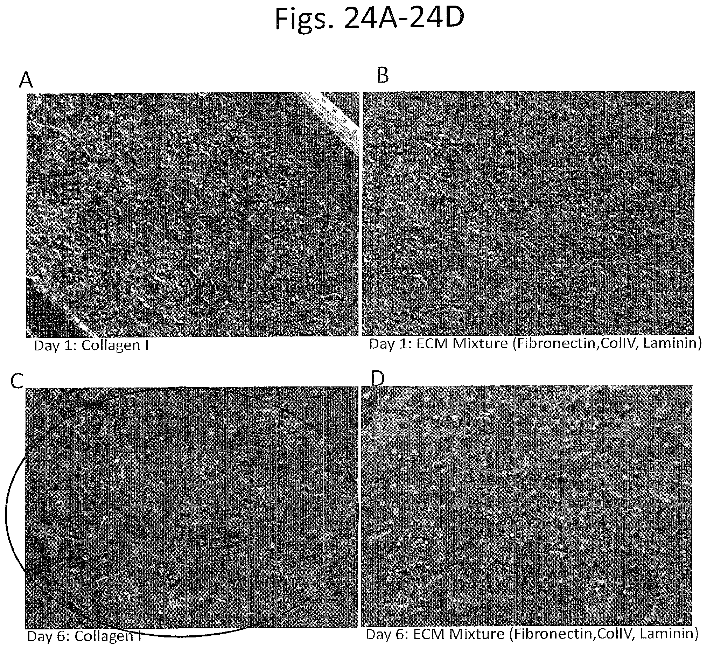

Like in Example 3, in order to achieve long-term maintenance (*e.g*., viability and function) of a co-culture model of dog hepatocytes and dLSECs in a device as shown in **Fig. 7B**, *e.g.,* for at least two weeks or longer, the inventors have optimized an ECM coating of the membrane, on which dog hepatocytes adhered. **Figs. 24A-24D** are images showing cell morphology of dog hepatocytes growing on an ECM coating of collagen I (100 ug/mL) or an ECM mixture coating comprising fibronectin, collagen IV and laminin. It was determined that an ECM coating of collagen I provided improved viability and growth of dog hepatocytes.

The inventors have also showed that dog hepatocytes and dLSECs maintained viable and functional in the dog liver chip for at least about 2 weeks or longer (**Figs. 25A-****25C**). Cell viability of dog hepatocytes and dLSECs over time were determined by measuring lactate dehydrogenase (LDH) levels over a period of time. As discussed in Example 1, when a cell is damaged and the membrane is compromised or becomes non-intact (*e.g*., with a break in the membrane), LDH is released into the culture media and thus the culture media can be collected and measured for LDH content. **Fig. 25B** shows that dog hepatocytes maintained low LDH release levels over a course of at least about 2 weeks, indicating viability and health of the hepatocytes monolayer being maintained in the dog liver chip over a period of at least 2 weeks. **Fig. 25C** showed measurement of albumin production over time, indicating that the dog liver chip remained viable and maintained a high level of albumin secretion for at least about 2 weeks or longer.

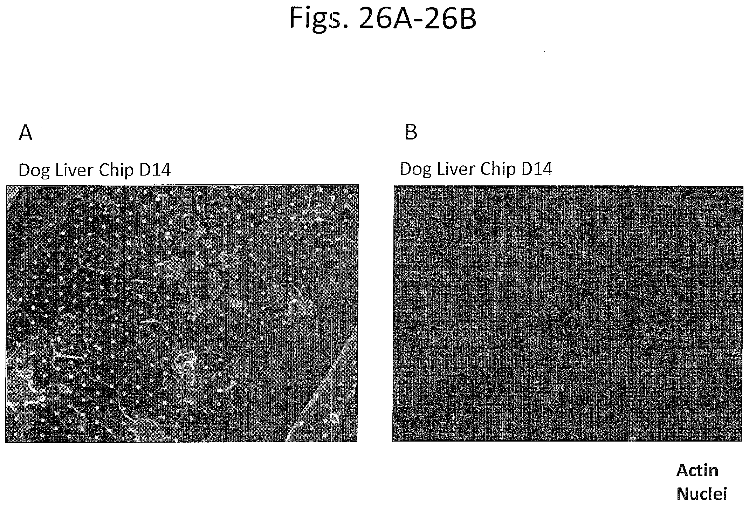

F-actin staining of the 2-week cultures shows cuboidal morphology of dog hepatocytes and pericanalicular distribution in dog liver chips (**Figs. 26A-26B**).

### Example 5. Enzyme Activity in Rat Liver-on-Chip

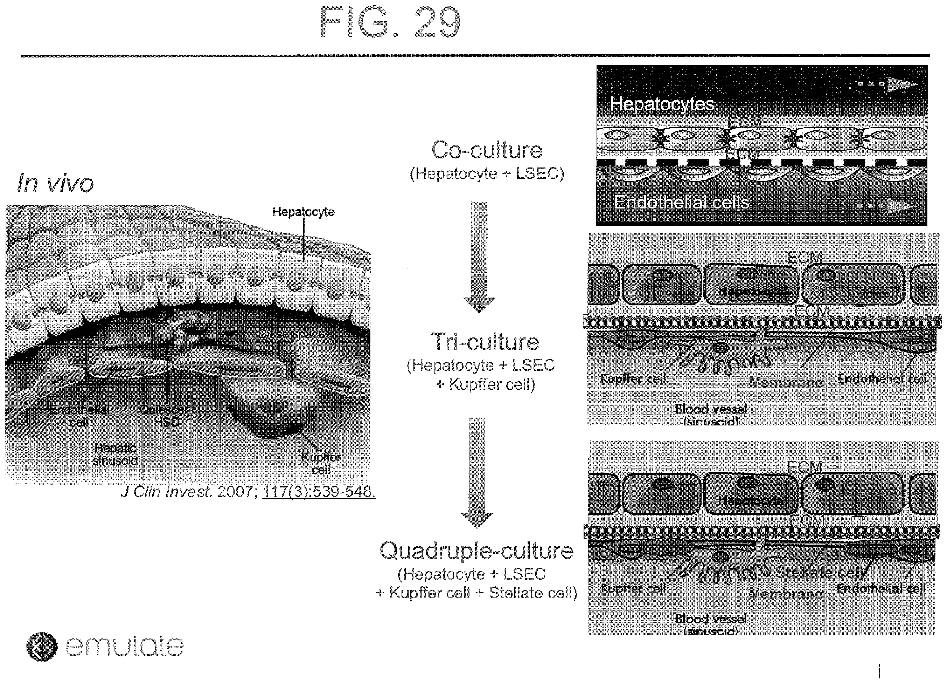

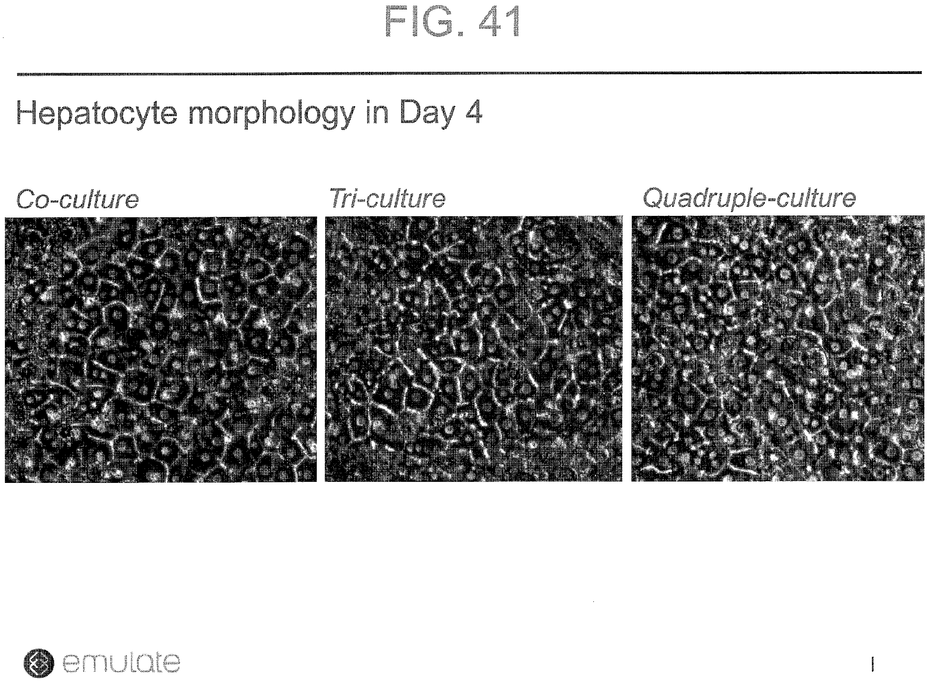

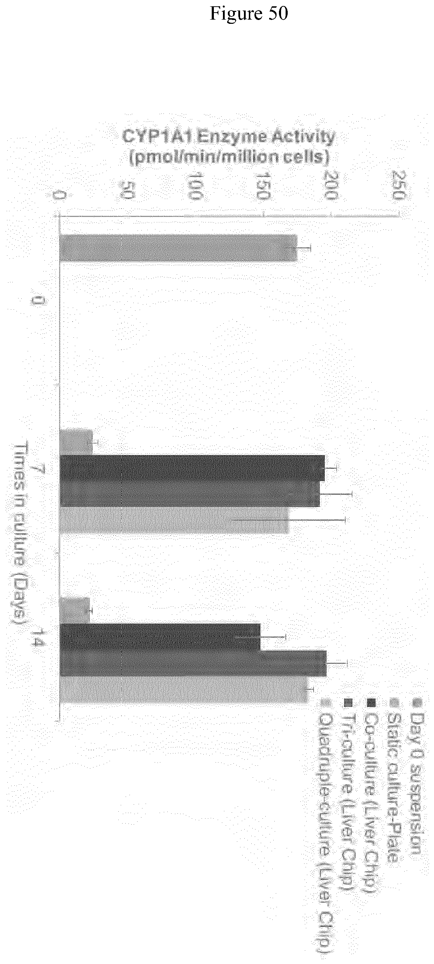

Experiments were extended from Example 3 for comparing enzymatic activity over time, *i.e.* CYP1A1, CYP2B1 and CYP3A1, between static cultures vs. co-cultures, tri-cultures and quad-cultures vs. freshly thawed hepatocytes. For these experiments, a co-culture refers to hepatocytes (upper chamber) and endothelial cells (lower chamber). A tri-culture refers to hepatocytes (upper chamber) and endothelial cells (lower chamber) in addition to Kupffer cells. A quadruple-culture refers to hepatocytes (upper chamber) and endothelial cells (lower chamber) in addition to Kupffer cells and Stellate cells. See, Figs. 50-52. An example of these types of cultures is shown in Figure 29 which provides an *in vivo* schematic (left side) showing the normal cellular architecture of the liver, and three in vitro schematics (right) showing a two cell arrangement (top right), a three cell arrangement (middle right) with the addition of Kupffer cells, and a four cell arrangement (bottom right) with the addition of both Kupffer cells and Stellate cells, on a microfluidic chip comprising two channels separated by a membrane. Another example is shown in Figure 41 that provides three photographs of hepatocyte morphology comparing a two-cell system (left), a three-cell system (middle) and a four-cell system (right) on a microfluidic "Rat Liver-on-chip."

All three types of microfluidic rat Liver-on-Chip appear to have significantly higher levels of CYP1A1 enzyme activity (pmol/min/million hepatocyte cells) than in a static conventional sandwich plate culture over time (0-14 days). All three types of microfluidic rat Liver-on-Chip show in vivo-relevant CYP1A1 enzyme activity over long-term culture (0-14 days). Figure 50 provides a graph measuring CYP1A1 Enzyme Activity in microfluidic Rat Liver-on-Chip.

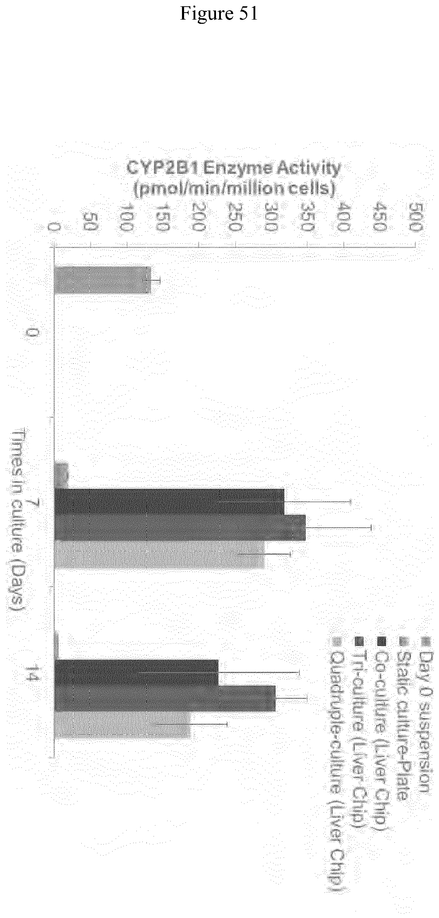

All three types of microfluidic rat Liver-on-Chip appear to have significantly higher levels of CYP2B1 enzyme activity (pmol/min/million hepatocyte cells) than in a static conventional sandwich plate culture over time (0-14 days). All three types of rat Liver-on-Chip show in vivo-relevant CYP2B1 enzyme activity over long-term culture. Figure 51 provides a graph measuring CYP2B1 Enzyme Activity in microfluidic Rat Liver-on-Chip.

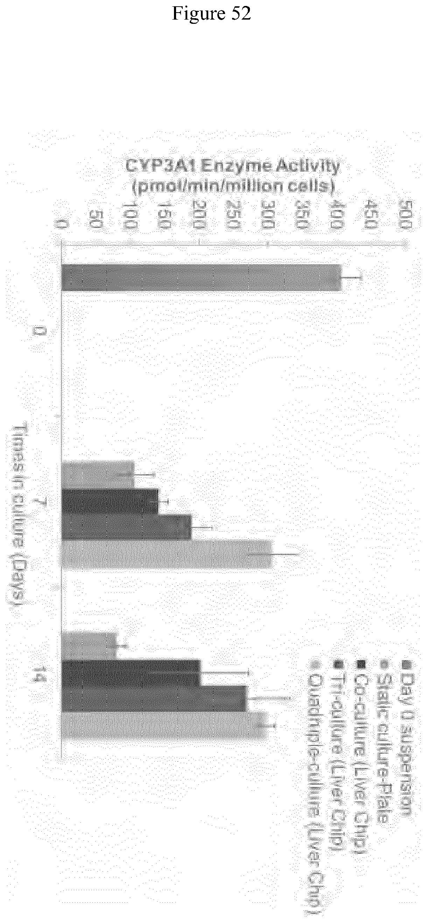

All three types of microfluidic rat Liver-on-Chip show higher levels of CYP3A1 activity (pmol/min/million hepatocyte cells) than in a static conventional sandwich plate culture. Moreover, a quadruple-culture of microfluidic rat Liver-on-Chip shows the highest CYP3A1 activity. Figure 52 provides a graph measuring CYP3A1 Enzyme Activity in microfluidic Rat Liver-on-Chip. As mentioned above, an example of a reliable and practical CYP3A induction assay with human hepatocytes in a 96-well format has been reported, where various 96-well plates with different basement membrane were evaluated using prototypical inducers, rifampicin, phenytoin, and carbamazepine. See Drug Metab. Dispo. (2010) Nov;38(11):1912-6.

### Example 6. Enzyme Activity in Dog Liver-on-Chip

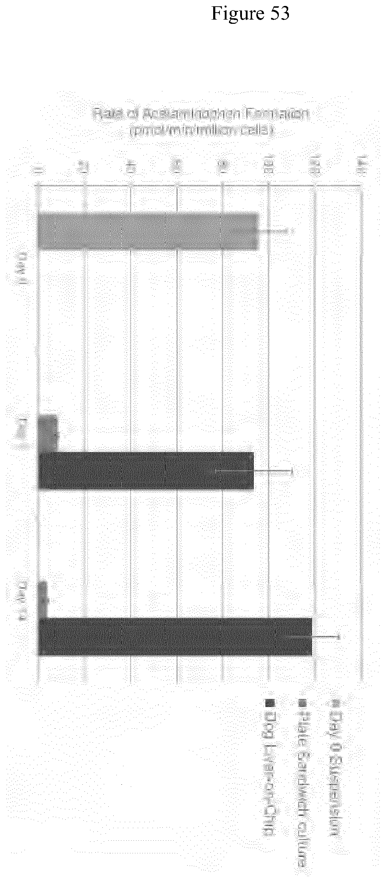

Experiments compared enzymatic activity over time between conventional plate sandwich cultures vs. dog liver-on-chip with a day 0 cell suspension, i.e. freshly isolated hepatocytes, used for a control. See, Figs. 53-57.

Rates of acetaminophen metabolite formation (pmol/min/million hepatocyte cells) from phenacetin show that a microfluidic Dog Liver-on-Chip maintains in vivo-relevant CYP1A2 activity over long-term culture (0-14 days). Additionally, microfluidic Dog Liver-on-Chip shows significantly higher levels of CYP1A2 enzyme activity than conventional plate sandwich culture. A day 0 suspension was used as a control. Figure 53 provides a graph measuring CYP1A2 Enzyme Activity in microfluidic Dog Liver-on-Chip.

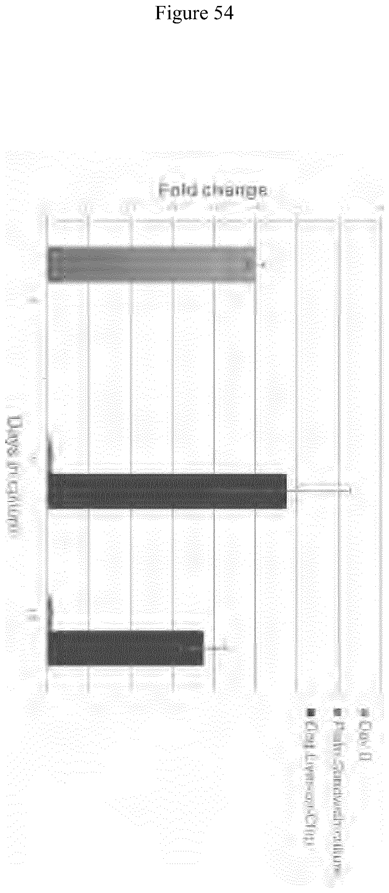

Dog Liver-on-Chip shows in vivo-relevant CYP1A2 gene expression over long-term culture (0-14 days) and maintenance comparing a fold change in expression. The relative expression level of CYP1A2 mRNA normalized to GAPDH mRNA and data is normalized to day 0 freshly isolated hepatocytes. There is a good correlation of expression level with CYP450 enzyme activity. Figure 54 provides a graph measuring CYP1A2 Gene Expression in microfluidic Dog Liver-on-Chip.

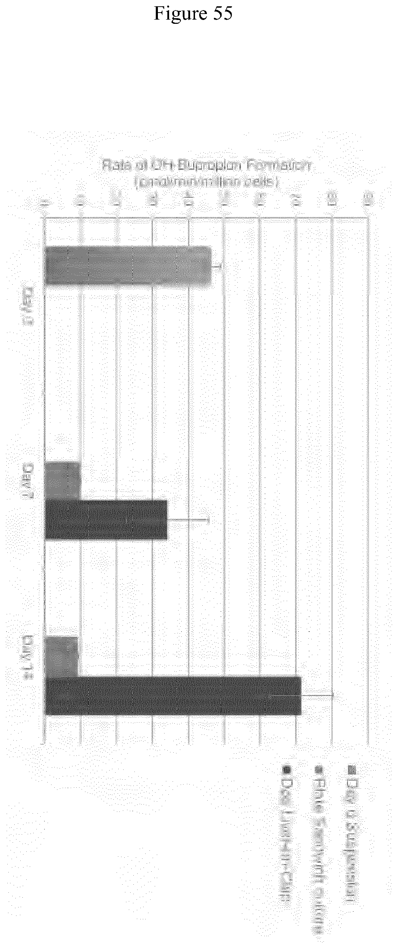

Microfluidic Dog Liver-on-Chip shows significantly higher levels of CYP2B enzyme activity than in conventional plate cultures by comparing a rate of hydroxylation, i.e. OH-bupropion formation (pmol/min/million hepatocyte cells) over time (0-14 days). Dog Liver-on-Chip maintain in vivo-relevant CYP2B11 activity over long-term culture. A day 0 suspension was used as a control indicating *in vivo* relevant enzyme activity level. Figure 55 provides a graph measuring CYP2B 11 Enzyme Activity in microfluidic Dog Liver-on-Chip.

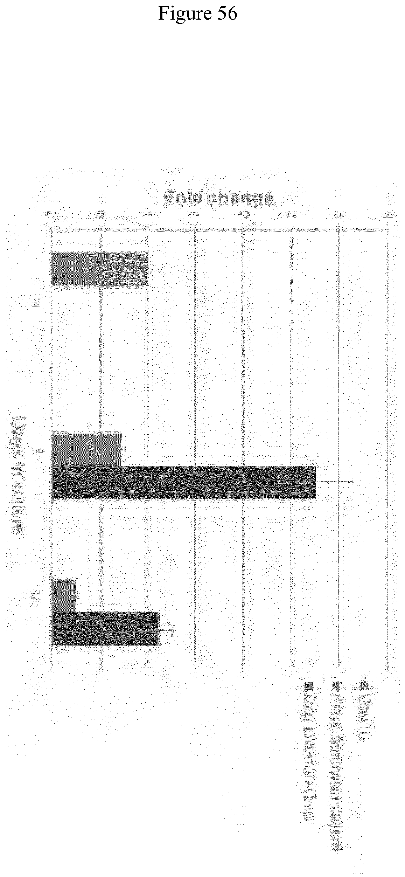

Dog Liver-on-Chip shows in vivo-relevant CYP2B 11 gene expression over long-term culture (0-14 days) and maintenance comparing a fold change in expression. The relative expression level of mRNA normalized to GAPDH mRNA. Data is normalized to day 0 freshly isolated hepatocytes indicating in vivo relevant gene expression level.. There is a good correlation of expression levels with CYP450 enzyme activity. A day 0 suspension was used as a control. Figure 56 provides a graph measuring CYP2B11 Gene Expression in microfluidic Dog Liver-on-Chip.

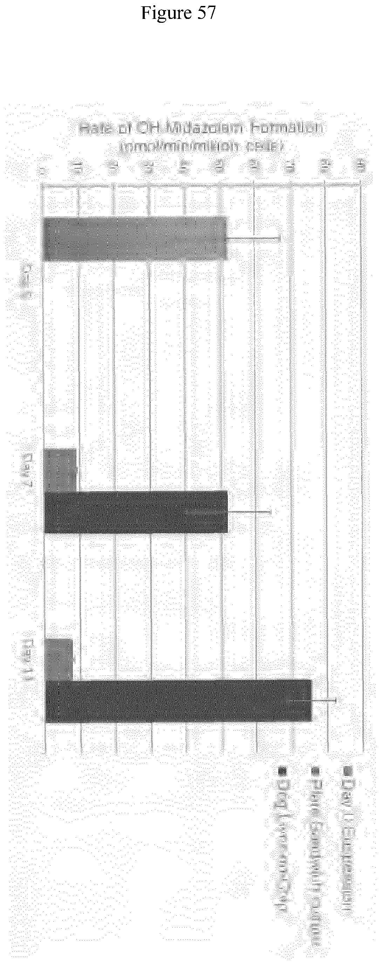

Dog Liver-on-Chip appears to have significantly higher levels of CYP3A12 enzyme activity than conventional plate culture over time (0-14 days) by comparing rates of OH-Midazolam metabolite formation (pmol/min/million cells). Microfluidic Dog Liver-on-Chip maintains in vivo-relevant CYP3A12 activity over long-term culture. A day 0 suspension was used as a control indicating in vivo relevant enzyme activity level. Figure 57 provides a graph measuring CYP3A12 Enzyme Activity in microfluidic Dog Liver-on-Chip.

### Example 7. Hepatic function in Human Liver-on-Chip

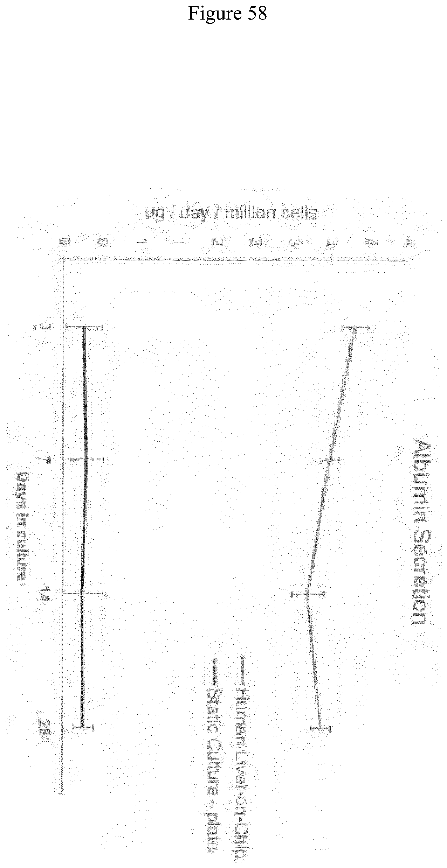

Experiments compared hepatic function over time between conventional plate sandwich cultures vs. Microfluidic Human liver-on-chip. See, Figs. 58-59.

Microfluidic Human Liver-on-Chip displays significantly higher levels of albumin secretion (ug/day/million cells) and maintenance for 28 days over a static plate culture, measured from day 3 to day 28). Figure 58 provides a graph measuring liver specific function: albumin secretion in microfluidic Human Liver-on-Chip Function.

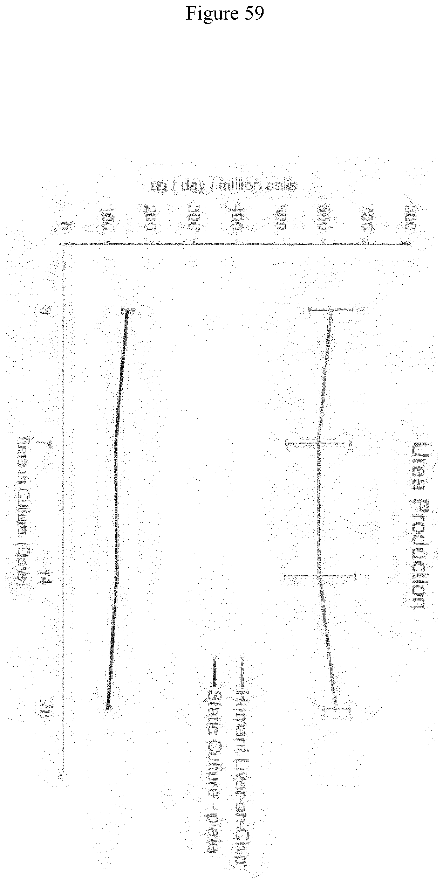

Microfluidic Human Liver-on-Chip displays significantly higher levels of urea production and maintenance (ug/day/million cells) for 28 days over a static plate culture, measured from day 3 to day 28). Figure 59 provides a graph measuring liver specific function: urea secretion in microfluidic Human Liver-on-Chip Function.

### Example 8. Enzyme Activity in Human Liver-on-Chip

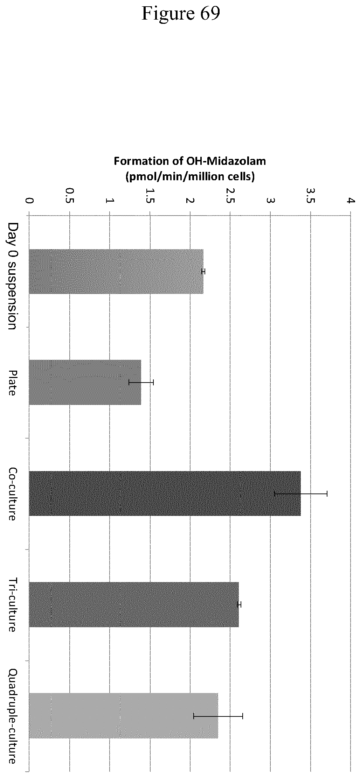

Experiments were extended from Example 7 for comparing enzymatic activity over time, *i.e.* CYP3A4, between static cultures vs. co-cultures, tri-cultures and quad-cultures vs. freshly thawed hepatocytes. For these experiments, a tri-culture refers to hepatocytes (upper chamber) and endothelial cells (lower chamber) in addition to Kupffer cells added to the hepatocytes. A quadruple-culture refers to hepatocytes (upper chamber) and endothelial cells (lower chamber) in addition to Kupffer cells and Stellate cells added to the hepatocytes. See, Figs. 69-70. An example of these types of cultures is shown in Figure 29 which provides an in vivo schematic (left side) showing the normal cellular architecture of the liver, and three in vitro schematics (right) showing a two cell arrangement (top right), a three cell arrangement (middle right) with the addition of Kupffer cells, and a four cell arrangement (bottom right) with the addition of both Kupffer cells and Stellate cells, on a microfluidic chip comprising two channels separated by a membrane.

All three types of microfluidic Human Liver-on-Chip appear to have higher levels of CYP3A4 enzyme activity (pmol/min/million hepatocyte cells) than in a static conventional sandwich plate culture on day 10 in culture. All three types of microfluidic Human Liver-on-Chip show in vivo-relevant CYP3A4 enzyme activity on day 10 in culture. Figure 69 provides a graph measuring CYP3A4 Enzyme Activity in microfluidic Human Liver-on-Chip.

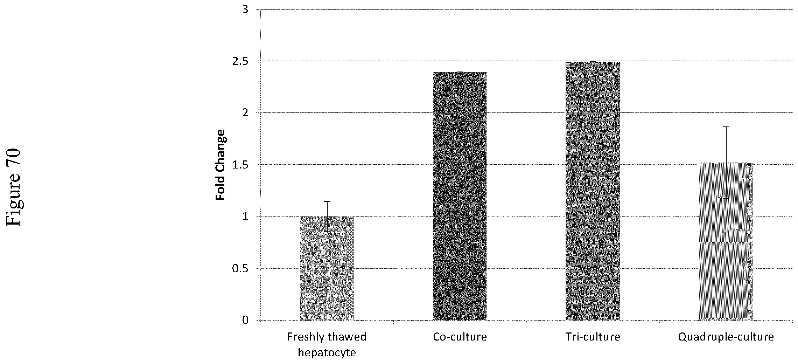

Three types of Human Liver-on-Chip show in vivo-relevant CYP3A4 gene expression on day 10 in culture compared to freshly thawed hepatocytes on day 0. The relative expression level of CYP3A4 mRNA normalized to GAPDH mRNA. Data is normalized to day 0 freshly thawed hepatocyte. There is a good correlation of expression level with CYP450 enzyme activity. A day 0 suspension was used as a control indicating in vivo relevant gene expression level. Figure 70 provides a graph measuring CYP3A4 Gene Expression in microfluidic Human Liver-on-Chip.

### Example 9. Hepatic function in Rat Liver-on-Chip

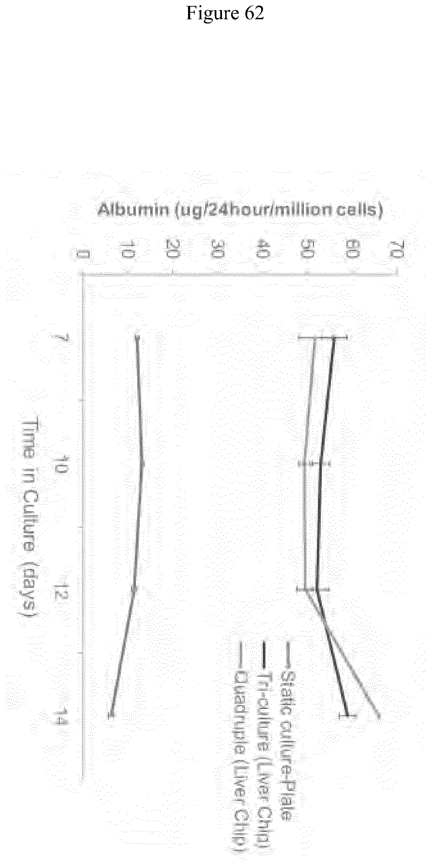

Experiments compared liver specific functions over time, i.e. albumin secretion and urea secretion, between static plate cultures vs. microfluidic Rat Liver-on-chips: tri-cultures and quad-cultures. For these experiments, a tri-culture refers to hepatocytes (upper chamber) and endothelial cells (lower chamber) in addition to Kupffer cells added to the hepatocytes. A quadruple-culture refers to hepatocytes (upper chamber) and endothelial cells (lower chamber) in addition to Kupffer cells and Stellate cells added to the hepatocytes. See, Figs. 62-63. An example of these types of cultures is shown in Figure 29 which provides an in vivo schematic (left side) showing the normal cellular architecture of the liver, and three in vitro schematics (right) showing a two cell arrangement (top right), a three cell arrangement (middle right) with the addition of Kupffer cells, and a four cell arrangement (bottom right) with the addition of both Kupffer cells and Stellate cells, on a microfluidic chip comprising two channels separated by a membrane.

Tri-culture and quadruple-cultures in a microfluidic rat Liver-on-Chip maintain high level of albumin secretion for 2 weeks. Figure 62 provides graph measuring liver specific function: albumin secretion for microfluidic Rat Liver-on-Chip Characterization for culture types: static culture plate; tri-culture chip and quadruple chip.

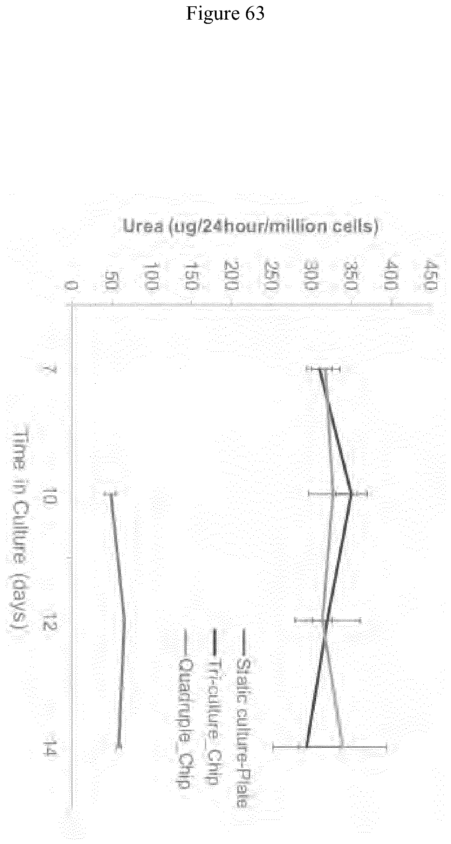

Tri-culture and quadruple-cultures in a microfluidic rat Liver-on-Chip maintain high level of urea synthesis for 2 weeks. Figure 63 provides a graph measuring liver specific function: urea secretion in microfluidic Rat Liver-on-Chip for culture types: static culture plate; tri-culture chip and quadruple chip.

### Example 10. Enzyme Activity in Human Liver-on-Chip

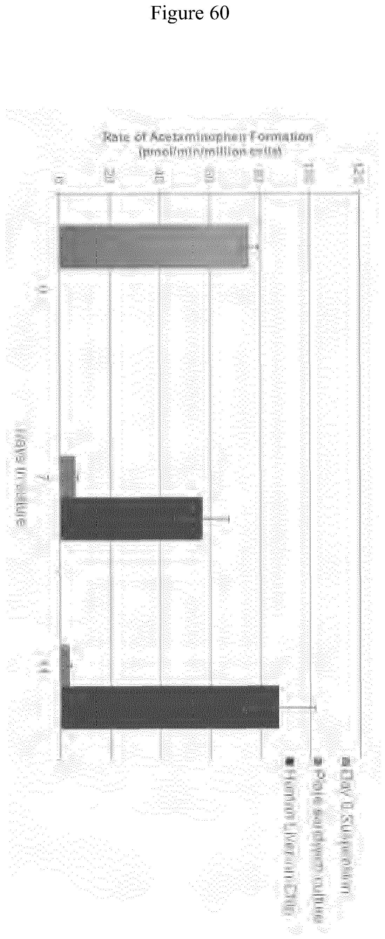

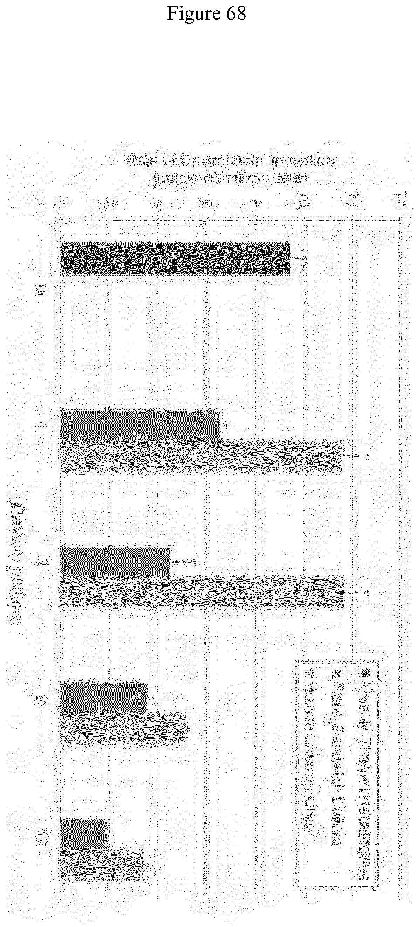

Experiments compared enzymatic activity over time between conventional plate sandwich cultures vs. Microfluidic Human liver-on-chip with freshly thawed hepatocytes used for a control. See, Figs. 60, 64-68.

Microfluidic Human Liver-on-Chips appear to have significantly higher levels of CYP1A2 enzyme activity in hepatocytes than in conventional plate culture by comparing rates of acetaminophen formation (pmol/min/million cells). Microfluidic Human Liver-on-Chip shows in vivo-relevant CYP1A2 activity over long-term culture (days 0-14). A day 0 suspension was used as a control indicating in vivo relevant condition. Figure 60 provides a graph measuring CYP1A2 Enzyme Activity in Microfluidic Human Liver-on-Chip.

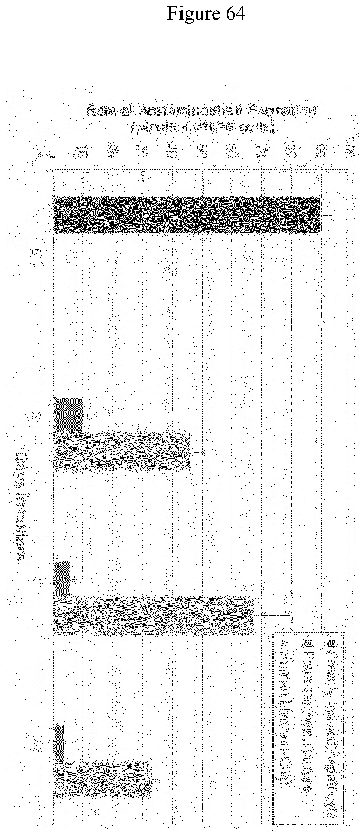

Rates of acetaminophen metabolite formation by from phenacetin (pmol/min/million cells) over time (0-14 days) in a microfluidic Human Liver-on-chip for measuring CYP1A2 activity. Culture conditions included: CYP1A2 enzyme activity was significantly higher in Liver-on-Chip than plate sandwich culture and maintained over 2 weeks in culture. Culture conditions included: 100 µM Phenacetin as probe substrate (2 hour incubation). Figure 64 provides a graph measuring CYP1A2 Enzyme Activity in a microfluidic Human Liver-on-Chip as compared freshly thawed hepatocytes and to static conventional sandwich plate culture over time.

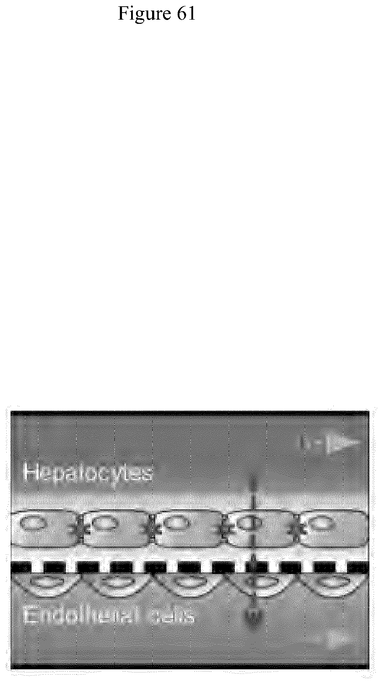

Rates of OH-Bupropion formation (pmol/min/million cells). Microfluidic Human Liver-on-Chip appear to have higher levels of CYP2B6 enzyme activity than conventional plate sandwich culture. A day 0 suspension was used as a control. Figure 61 provides a graph measuring CYP2B6 Enzyme Activity in Microfluidic Human Liver-on-Chip.

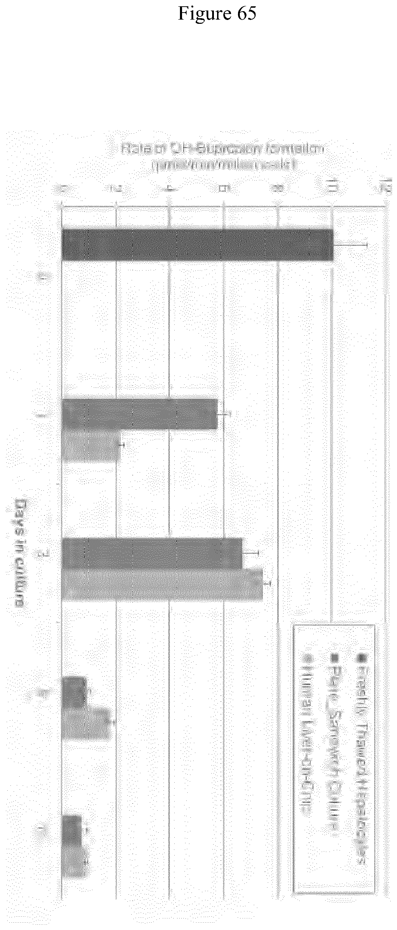

Rates of hydroxylation of Bupropion to OH-Bupropion metabolite formation (pmol/min/million cells) over time (0-15 days) for measuring CYP2B6 activity. Culture conditions included: 100 µM Bupropion as probe substrate. Figure 65 provides a graph measuring CYP2B6 Enzyme Activity in microfluidic Human Liver-on-Chip a compared to freshly thawed hepatocytes and a plate sandwich culture.

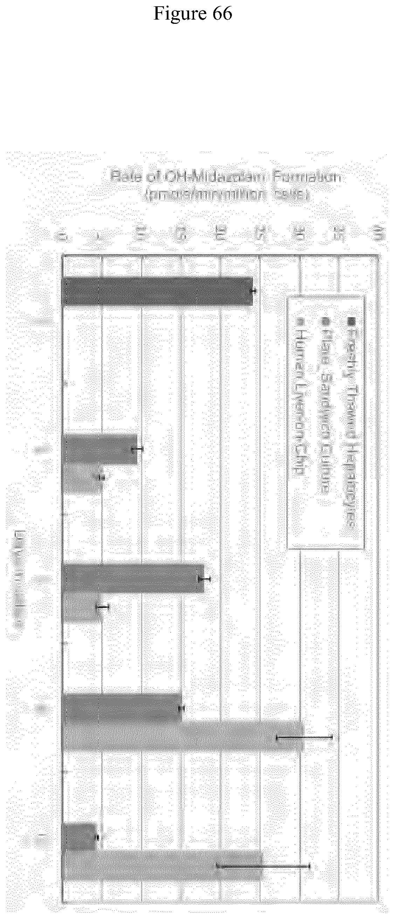

Rates of hydroxylation of Midazolam to OH-Midazolam metabolite formation (pmol/min/million cells) in a microfluidic Human Liver-on-chip over time (0-15 days) for measuring CYP3A4 activity. Culture conditions included: 20 µM Midazolam as probe substrate. Figure 66 provides a graph measuring CYP3A4 Enzyme Activity in Microfluidic Human Liver-on-Chip as compared to freshly thawed hepatocytes and a plate sandwich culture.

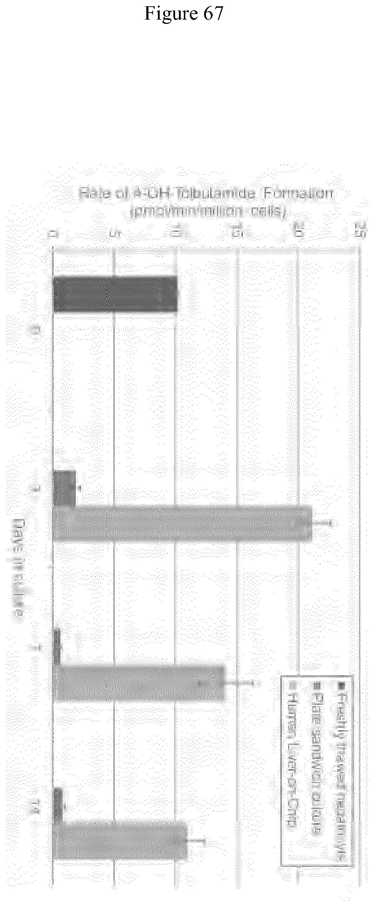

Rates of 4-hydroxy (4-OH)-tolbutamide metabolite formation from tolbutamide (pmol/min/million hepatocyte cells) over time (0-14 days) in a micro fluidic Human Liver-on-chip. CYP2C9 activity was significantly higher in Liver-on-Chip than plate sandwich culture and maintained for 2 weeks in culture. Culture conditions included: 100 µM tolbutamide as probe substrate (2 hour incubation). Figure 67 provides a graph measuring CYP2C9 Enzyme Activity in microfluidic Human Liver-on-Chip as compared to freshly thawed hepatocytes and a plate sandwich culture.

Rates of dextrorphan metabolite formation from dextromethorphan (pmol/min/million hepatocyte cells) showing CYP2D6 enzyme activity in a microfluidic Human Liver-on-chip over time (0-15 days). Microfluidic Human Liver-on-chip CYP2D6 enzyme activity was compared to freshly thawed hepatocytes and a plate sandwich culture. Culture conditions included: 20 µM dextromethorphan as probe substrate. Figure 67 provides a graph measuring CYP2D6 Enzyme Activity in microfluidic Human Liver-on-Chip.

### Example 11. CYP450 Gene Expression for Enzymes in Human Liver-on-Chip

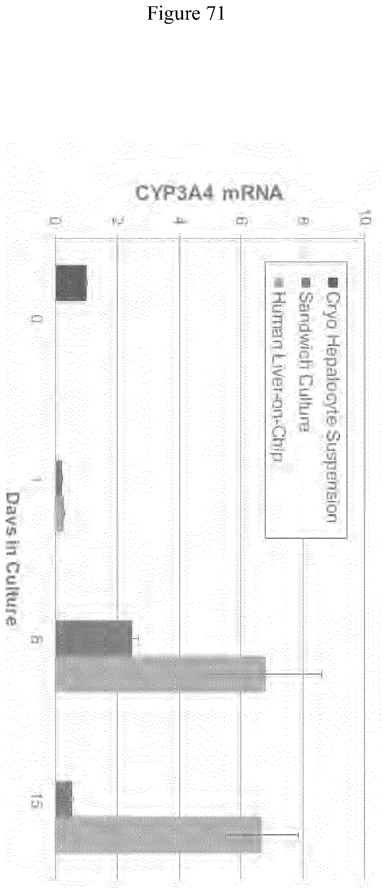

Experiments compared *CYP450* gene expression (*e.g. CYP3A4; CYP1A2;* and *CYP2B6)* over time between conventional plate sandwich cultures vs. Microfluidic Human liver-on-chip with freshly thawed (cryo) hepatocytes in suspension used for a control. See, Figs. 71-73. Isolated hepatocytes were frozen using conventional freezing techniques then as an example, stored in liquid nitrogen tanks until used in methods as described herein.

Human Liver-on-Chip shows *CYP3A4* mRNA expression is significantly higher in microfluidic co-cultures than sandwich plate culture or hepatocytes in suspension, which increased over time in culture (over 0-15 days) as measured by comparing increases as a fold change in level of expression. The relative expression level of mRNA was normalized for each sample value against Hypoxanthine-guanine phosphoribosyltransferase (HRPT1), a housekeeping gene. These graphs show fold-changes in levels relative to day 0 freshly thawed (cryo) hepatocytes in suspension (representative of *in vivo* levels). Figure 71 provides a graph measuring *CYP3A4* mRNA Expression in a microfluidic "Human Liver-on-Chip" as compared to static conventional sandwich plate culture over time (0-15 days) and to freshly thawed (cryo) hepatocyte suspension (Day 0).

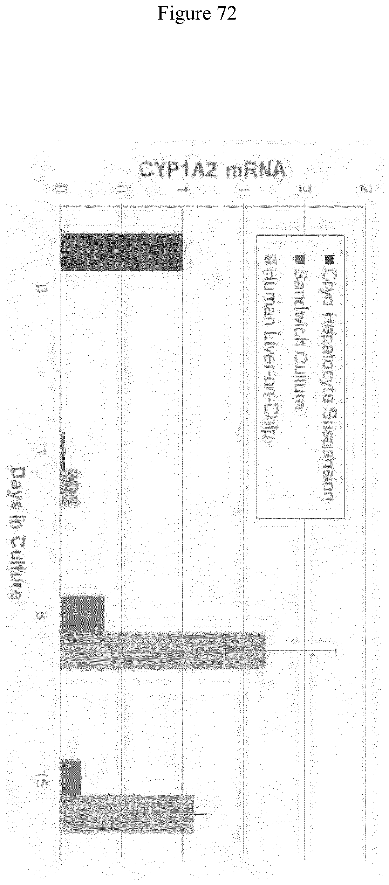

*CYP1A2* mRNA Expression in Human Liver-on-Chip demonstrates that *CYP1A2* expression is significantly higher in Liver-on-Chip than a plate sandwich culture which increased over time in culture (over 0-15 days). Each sample value was normalized against HRPT1 (housekeeping gene). These graphs show fold-change relative to day 0 hepatocyte in suspension representative of *in vivo* levels). Figure 72 provides a graph measuring *CYP1A2* mRNA Expression in a microfluidic "Human Liver-on-Chip" as compared to static conventional sandwich plate culture over time (0-15 days) and to freshly thawed (cryo) hepatocyte suspension (Day 0).

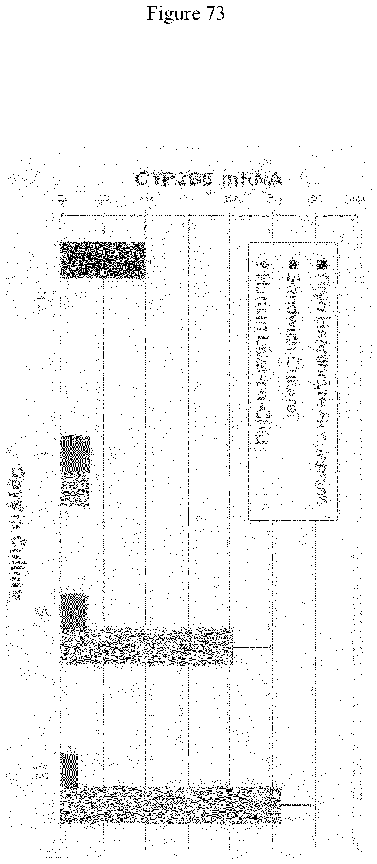

*CYP2B6* expression is significantly higher in Liver-on-Chip than sandwich culture which increased over time in culture (0-15 days). Each sample value was normalized against HRPT1 (housekeeping gene). These graphs show fold-change relative to day 0 hepatocyte in suspension (representative of *in vivo* levels). Figure 73 provides a graph measuring *CYP2B6* mRNA Expression in a microfluidic "Human Liver-on-Chip" as compared to static conventional sandwich plate culture over time (0-15 days) and to freshly thawed (cryo) hepatocyte suspension (Day 0).

### Example 12. Discovering Parameters for Assessing Clinical Relevance Of Microfluidic Liver-on-Chip Model, i.e. Compound Test Plan.

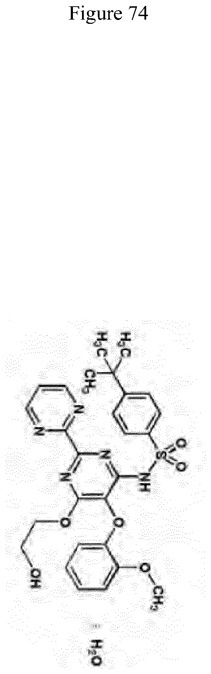

A test plan for evaluating compounds was designed and used in order to begin setting up validation models with certain tool compounds with known liver toxicity issues, or liver toxicity issues discovered during the development of the present inventions, to reproduce these toxicity results in animal liver chips followed by assessing clinical relevance by duplicate testing of these tool compounds in the microfluidic HUMAN Liver-on-Chip. See, Figs. 74-77.

Exemplary Experiment design: 1. Select a tool compound.; 2. Select the "Liver-on-Chip" model (e.g. RAT; DOG; pr HUMAN). Based on the compound's reported liver histopathology and, without limiting to any mechanisms, using information on the tool compound's mechanism of action, then: step 3. Select concentration range for testing in the selected model(s). 4. End point analysis is based on a chosen mechanism of action of the compound, without limiting to any one mechanism.

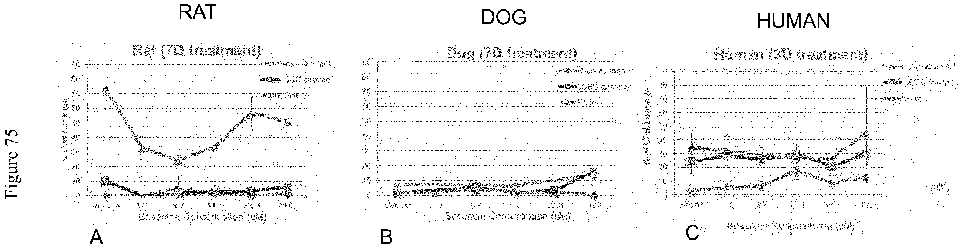

An exemplary tool compound was chosen for a pilot study, as an exemplary step 1: Bosentan (Tracleer), see, Fig. 74 for an exemplary chemical structure. Bosentan refers to a dual antagonist of endothelin (Endothelin Receptor Type A: ETA and Endothelin Receptor Type B (ETB) receptors and is approved in humans for the management of pulmonary arterial hypertension. In clinical trials, approximately 8-18% of human patients treated with Bosentan developed hepatotoxicity, as reflected by elevations in serum Alanine aminotransferase (ALT) greater than 3-fold. In a few patients with multiple drug therapies, cases of severe hepatotoxicity were reported. It was proposed that Bosentan-induced liver injury is due to accumulation of bile acids in the hepatocyte resulting from inhibition of the canalicular bile salt export pump (BSEP). Step 2: RAT and DOG "Liver-on-Chip" models were chosen for comparison to a HUMAN "Liver-on-Chip". Step 3: A concentration range of 1.2 to 100 µM Bosentan was selected. Step 4, a first type of mechanism, e.g. exemplary LDH leakage was chosen for analysis of toxicity. Figure 75 provides graphs measuring Bosentan Toxicity as a percentage of LDH Leakage in three different species of microfluidic chips: A) "RAT Liver-on-Chip" (7 days of treatment); B) microfluidic "DOG Liver-on-Chip" (7 days of treatment); and C) microfluidic "Human Liver-on-Chip" (3 days of treatment) increasing concentrations from 1.2 (1.3 for human)-100 µM. Samples were removed from the hepatocyte channel and the LSEC channel of the microfluidic chip with a sample from a plate sandwich culture shown for comparison. As shown in Fig. 75A, rat hepatocytes on conventional plates does not show LDH toxicity, and neither hepatocytes or cells in the liver sinusoidal endothelial cells (LSEC) channel showed LDH leakage, i.e. no toxicity effects, similar to a lack of effects for all three types of dog cell samples, until the highest dose at 100 uM, Fig. 75B. Both types of microfluidic "Liver-on-chips" were incubated for 7 days. In contrast, microfluidic "HUMAN Liver-on-Chip", incubated for 3 days, showed a general dose response from 1.3 to 100 µM Bosentan for hepatocytes, while hepatocytes on plates showed an effect at 100 µM Bosentan. *See,* Fig. 75C.

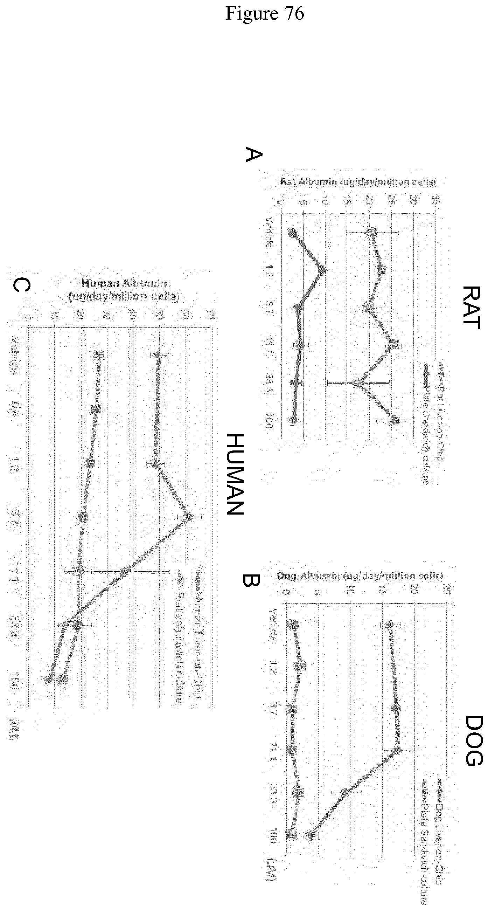

Because neither the microfluidic RAT or DOG "Liver-on-Chips" showed LDH leakage toxicity of their hepatocytes comparable to that of hepatocytes in microfluidic "HUMAN Liver-on-Chips, another effect of Bosentan toxicity, *i.e.* Albumin secretion (at µg/day/million hepatocytes cells) was chosen for analysis. Using the same range of Bosentan concentrations on microfluidic RAT and DOG "Liver-on-Chips", with plate sandwich cultures as controls, a clear dose response associated with albumin secretion, i.e. increasing levels of Bosentan inducing a loss of albumin secretion in fluid samples from the upper chamber of the chips, was observed with DOG, see, Fig. 76B, but not RAT, *see,* Fig. 76A, chips. Further, loss of albumin secretion was observed at either 3.7 µM or 11.1 µM, the same for LDH on HUMAN Liver-on-Chips, *see,* Fig. 76C, revealing a clear dose response of Bosentan that paralleled, in part, the DOG response. Moreover, HUMAN hepatocytes in the microfluidic chips were more sensitive than DOG hepatocytes in microfluidic chips to 11.1 µM Bosentan such that DOG hepatocytes were producing relatively constant amounts of albumin, while HUMAN hepatocytes were showing toxicity effects by secreting significantly less albumin, *see,* Fig. 76. Thus, for step 4, one exemplary toxicity mechanism is a loss of albumin secretion.

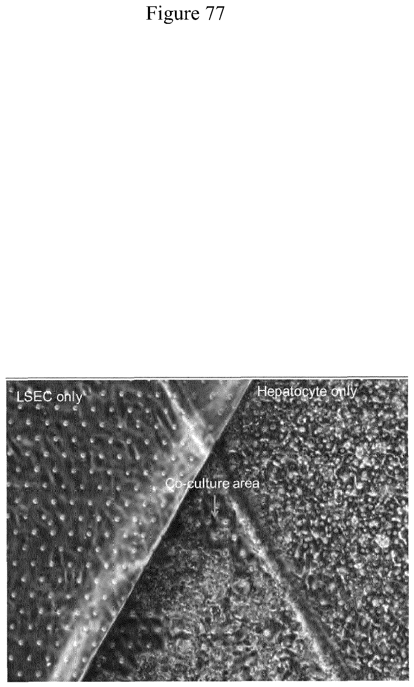

One contemplated difference between unsuccessful modeling experiments *i.e.* measuring cell death by LDH release, as in Fig, 75 vs. successful modeling measuring reduced cell function by a reduction in albumin secretion, as in Fig. 76, was potential crosstalk (e.g. exchange of secretions or physical cell-cell interactions) between Hepatocyte and liver sinusoidal endothelial cells (LSEC) after treatment with Bosentan. Therefore, a Human Liver-on-Chip was designed and seeded with areas of LSEC only and hepatocytes only, allowing a co-culture area in between, see, micrograph in Fig. 77. This modified Human Liver-on-Chip was treated with Bosentan at 11 uM, the amount where hepatocytes have increased LDH leakage while the LSEC channel shows no change, see, Fig. 75C, and where a noticeable loss of albumin secretion was measured, see, Fig. 76C. After 3 days of treatment, significant hepatocyte degradation in co-culture area with LSEC was visually observed but not in either the LSEC area or area of hepatocytes alone. Therefore, co-culturing with LSEC increases hepatocytes sensitivity to Bosentan in these microfluidic HUMAN Liver-on-Chips.

Furthermore, differences in Bosentan Toxicity between species observed with the data generated during the development of the present inventions, correlate with findings reported in literature, with examples described in this paragraph. Specifically, it was reported that Bosentan-treated rats do not develop hepatotoxicity. Therefore, either BSEP inhibition is not a mechanism underlying Bosentan hepatotoxicity or factors that mitigate the impact of BSEP inhibition in the rat do not have the same effect on human hepatocytes. Additionally, rats may be less susceptible to Bosentan-induced hepatotoxicity because rat bile acids are reported to be inherently less toxic than human bile acids.

In experiments by others, Bosentan was found to be a more potent inhibitor of Na(+)-dependent taurocholate uptake in rat (IC(50) 5.4 µM) than human (IC(50) 30 µM) in suspended hepatocytes. In addition, Bosentan was a more potent inhibitor of taurocholate uptake by rat Na(+)-dependent taurocholate co-transporting polypeptide (Ntcp/S1c10a1) (IC(50) 0.71 µM) than human NTCP (SLC10A1) (IC(50) 24 µM) expressed in HEK293 cells, see, Leslie, et al., "Differential inhibition of rat and human Na+-dependent taurocholate cotransporting polypeptide (NTCP/SLC10A1) by bosentan: a mechanism for species differences in hepatotoxicity." J Pharmacol Exp Ther. 321(3): 1170-8 2007.

### Example 13: Kupffer Cells For Use In Microfluidic Liver-On-Chips

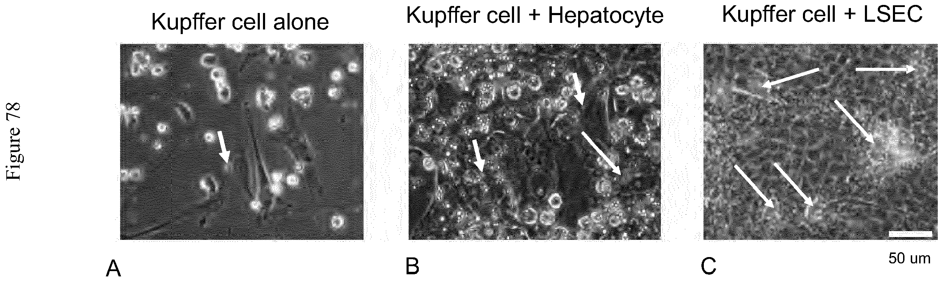

This example provides an exemplary successful method of Kupffer cell isolation, optimized during the development of the present inventions using dog cells. Results from using these isolated cells in microfluidic Liver-On-Chips are also provided in this Example. See, Figs. 78-81.

### Exemplary Dog Kupffer Cell Isolation.

Isolated Kupffer cells were obtained in supernatant from a first centrifugation of a dog hepatocyte cell isolation, as adapted from a known method (*e.g*. Ramm. "Isolation and culture of rat hepatic stellate cells." J Gastroenterol Hepatol. 13:846-851 (1998). This method uses series of centrifugations and cell density gradients to isolate specific types of liver cells. In brief, the non-parenchymal cell suspension undergoes a series of centrifugations, i.e. 1,000g (g: gravity, refers to a relative centrifugal force) then 2,000g, then 3,000g, followed by centrifugation in a Nycodenz density gradient (11.5%-17.5%). Kupffer cells are harvested from the buffy coat located in between two other layers, a pink upper layer and white lower layer. Kupffer cells were confirmed with CD68 (CD68+) positive staining. Although in general, CD68 refers to a macrophage lineage marker (*e.g*. monocytes, osteoclasts, Kupffer cells, etc.), in the cultures described herein, CD68 is specifically staining Kupffer cells.

A hallmark of macrophage function is phagocytosis of particles, debris, and pathogens followed by their processing and presentation as antigen, which is often accompanied by the release of inflammatory cytokines causing the activation or recruitment of other cells of the immune system. Therefore, these isolated dog Kupffer cells were tested for phagocytic activity. This method was also used to isolate rat Kupffer cells for use in phagocytic assays and RAT microfluidic Liver-on-Chip, *see,* Fig. 78, Example 14 below and an exemplary schematic in Fig. 29 for a quad-culture microfluidic Liver-On-Chip.

### Characterization of Kupffer Cell Function.

Rat Kupffer Cell Phagocytosis. Kupffer cells, in co-cultures with LSECs, showed markedly increased particle engulfment shown by the red particles that were phagocytosed by kupffer cells. A representative phagocytosis assay in the plate culture is shown, i.e. Day 4, see, Fig. 78. Fig. 78A shows Kupffer cells alone; Fig. 78B, shows Kupffer cells + Hepatocytes; and Fig. 78C shows Kupffer cells+ LSECs.

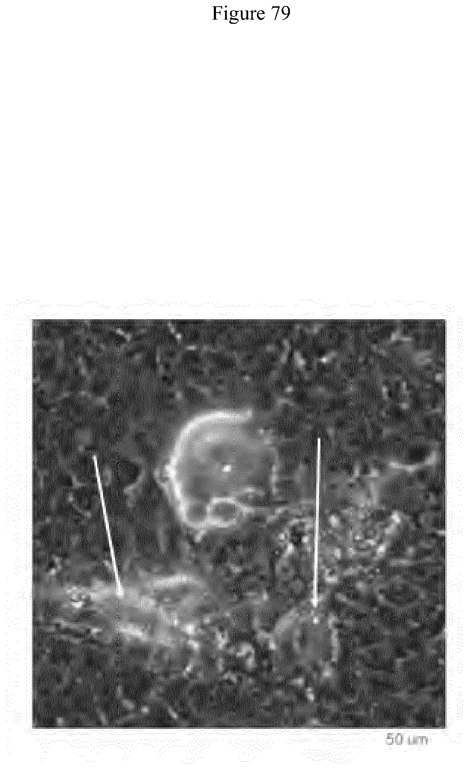

Rat Kupffer Cell Migration. Phase contrast time-lapse microscopy was used to study the morphodynamics of Kupffer cells cultures on sinusoidal endothelial cells in plates. Time-lapse duration was over 1.5 hours, at 30-second intervals. An exemplary micrograph from Day 7 is shown in Fig. 79. Kupffer cells with (+) LSECs in a plate, exposed to red particles, indicates active migration by Kupffer cells having migratory morphology, i.e. exemplary two elongated cells containing red particles, one cell in the lower half and one cell in the lower right area of the micrograph.

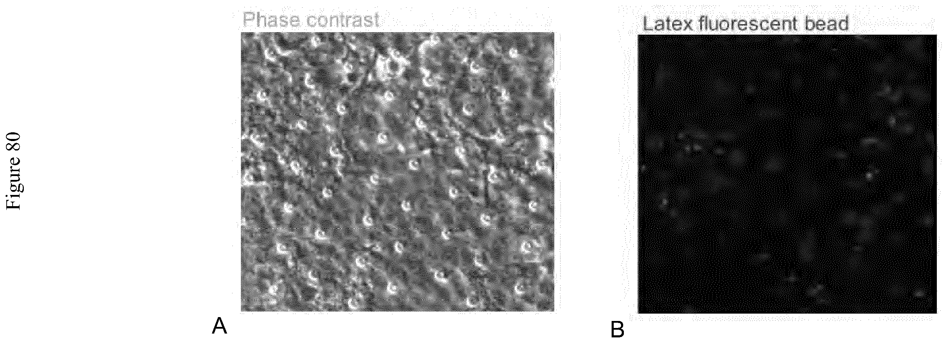

Rat Kupffer Cell Phagocytosis. Rat kupffer cells cultured with LSECs in Rat Liver-on-Chip showed phagocytic kupffer cell function. A Tri-culture Rat Liver-on-Chip (Hepatocyte + LSEC + Kupffer cell) was tested with a phagocytosis assay on Day 7 of culture. For this assay, latex fluorescent beads were fed to chip cultures, see Fig. 80B, with a phase contrast image of this field shown in Fig. 80A.

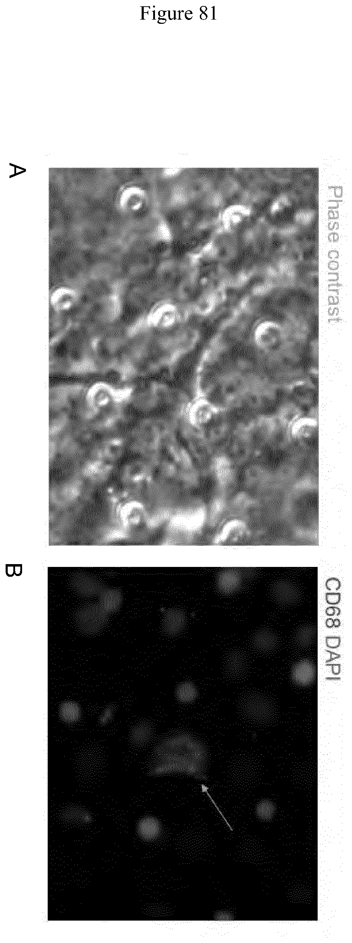

Rat Kupffer Cell CD68 identification for characterization of Kupffer Cells within Chip on Day 7 of incubation. A microfluidic tri-culture demonstrated expression of CD68 in rat Kupffer cells co-cultured with endothelial cells located in the bottom channel of the Rat Liver-on-Chip. A Kupffer cell marker was identified using anti-CD68 antibody, fluorescing red. CD68 staining of Kupffer cells is shown using CD68 (red) and DAPI (blue) stained nuclei (4',6-diamidino-2-phenylindole), see, Fig. 81B, compared to a phase contrast microscope image of this field, see, Fig. 81A.

### Example 14: Stellate Cells For Use In Microfluidic Liver-On-Chips

This example provides an exemplary successful method of rat Stellate cell isolation, optimized during the development of the present inventions using dog cells. Results from using isolated rat cells in microfluidic Rat Liver-On-Chips are also provided in this Example. See, Figs. 79-82.

### Exemplary Dog Stellate Cell Isolation.

Isolated Stellate cells were obtained in supernatant from a first centrifugation of a dog hepatocyte cell isolation, as adapted from a known method (*e.g.* Riccalton-Banks, et al., "A simple method for the simultaneous isolation of stellate cells and hepatocytes from rat liver tissue." Molecular and Cellular Biochemistry 248: 97-102 (2003). This method uses series of low speed centrifugations to isolate Stellate cells. In brief, the non-parenchymal cell suspension undergoes a series of centrifugations, i.e. 50g, then 200g, followed by surface adherence to plastic cell culture plates. Cells were stained for specific markers, i.e. vimentin and desmin. This method was used to isolate rat Stellate Cells for use in a microfluidic Liver-on-Chip, see an exemplary schematic in Fig. 29 for a quad-culture microfluidic Liver-On-Chip.

### Characterization of Stellate Cell Function.

A microfluidic quadruple-culture of Rat Liver-on-Chip was created by adding rat hepatocytes (rhepatocytes), rat LSEC (rLSEC), rat Kupffer (rKupffer) cells and rat Stellate (rStellate) cells that represent a highly functional miniature liver tissue that models liver injuries. This microfluidic Liver-on-chip demonstrates a successful adapted liver chip design for optimal quadruple-culture.

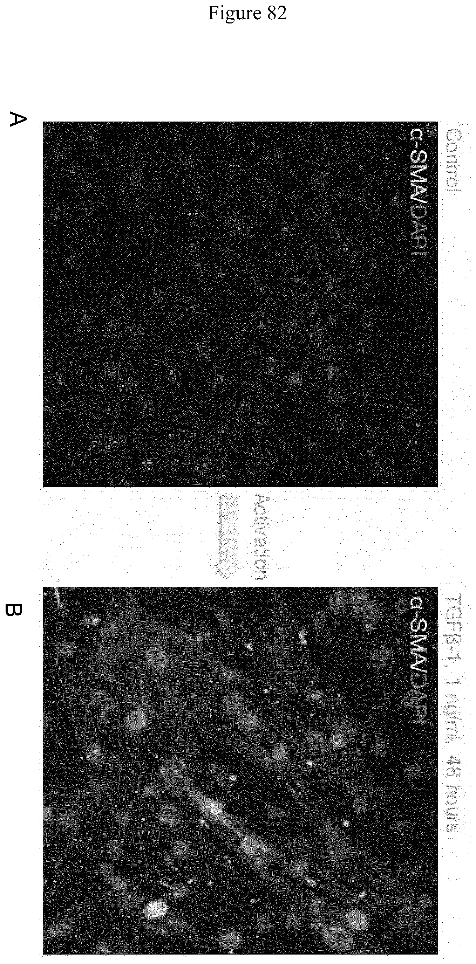

A quadruple-culture microfluidic Rat Liver-on-Chip at Day 7 has viable Stellate cells. On Day 7, Stellate cell activation by TGFβ-1 (1ng (nanogram)/ml) for 48 hours resulted in increased (strong) expression of activation marker Alpha-smooth muscle actin (α-SMA) (green) fibers with DAPI (blue) nuclear staining, see, Fig. 82B. Fig. 82A shows an exemplary culture before activation having DAPI (blue) nuclear staining with faint staining of a few α-SMA) (green) fibers.

### Example 15: Antipyrine Clearance Test In Microfluidic HUMAN Liver-On-Chips

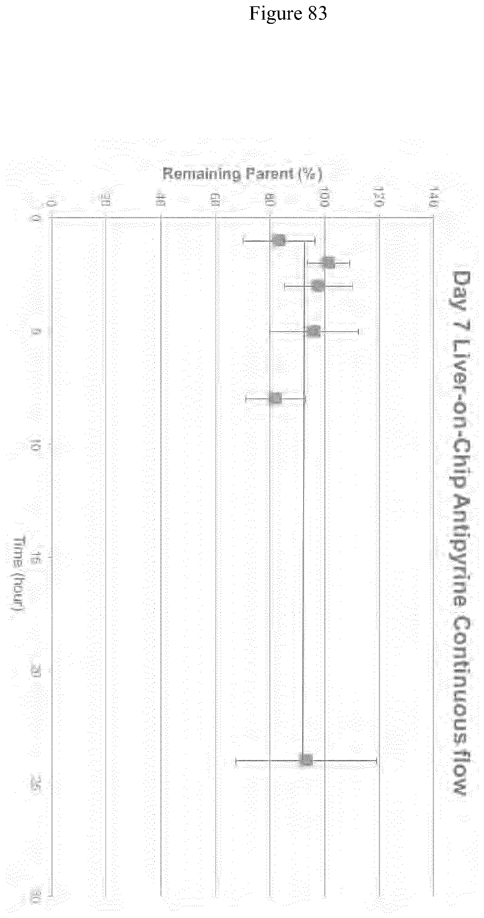

Each drug molecule has a limited time to interact with hepatocytes, i.e. an interaction time. Under conditions of flow, the interaction time is reduced. However the interaction time can be increased by either using non-dynamic conditions (absence of flow) or by reticulating the media containing the drug. The present invention contemplates both embodiments for increasing interaction time, and in particular for low clearance compounds. Therefore, this is an exemplary example for demonstrating interaction time using a test agent, Antipyrine. See, Figs. 83-84.

### Antipyrine Clearance Test in the Liver-on-Chip

Under dynamic conditions: Antipyrine medium removed from the Liver-on-Chip at 5 sample points over 10 hours of incubation, then again after 25 hours of incubation, was tested for an amount (%) of remaining compound. After 25 hours, around 90% of the compound remained in the media. See, Fig. 83.

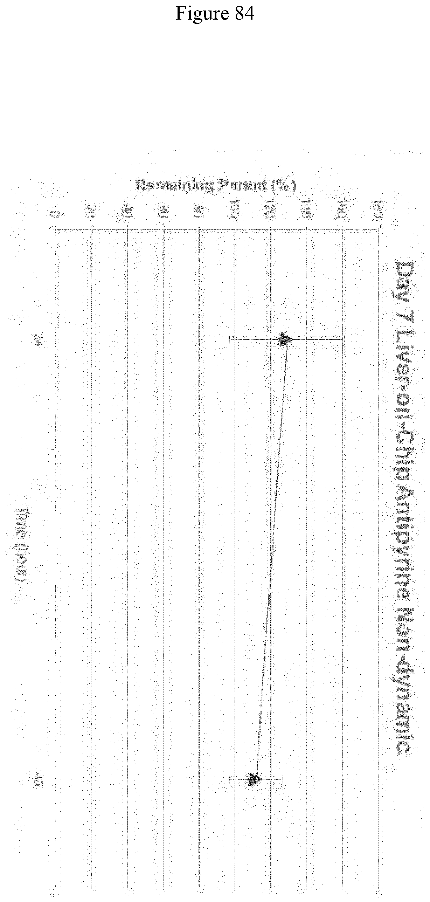

Under non-dynamic conditions: 50 µl (total) of Antipyrine medium in the Liver-on-Chip was tested for an amount (%) of remaining compound, after incubation for 24 hours and 48 hours. At 48 hours, around 100% of the compound appeared to be present in the media. Initial parent concentration was higher due to an evaporation issue while running low volume of medium. See, Fig. 84.

### Example 16: Enzyme Test In Microfluidic HUMAN Liver-On-Chips

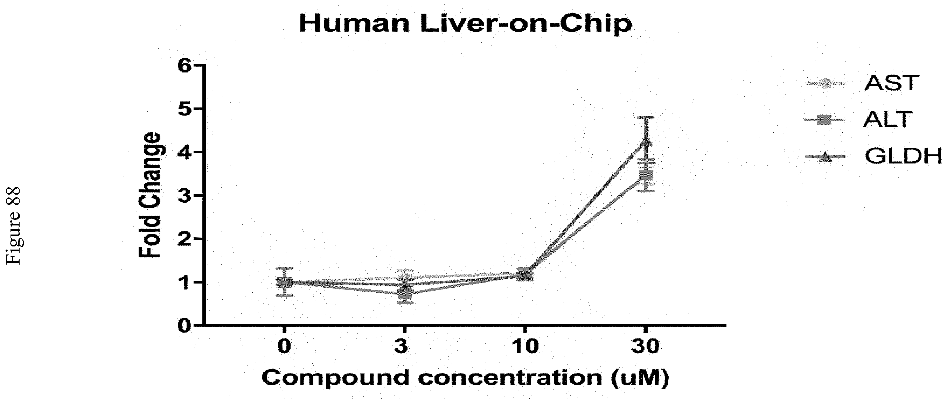

Alanine aminotransferase (ALT), aspartate aminotransferase (AST) enzyme levels are reliable tests for liver damage. Hepatic toxicity of test compound X was evaluated on the human liver-on-chip by the quantification of ALT, AST and Glutamate dehydrogenase (GLDH) released. Figure 88 provides a graph measuring mRNA shows alanine aminotransferase (ALT), aspartate aminotransferase (AST) and Glutamate dehydrogenase (GLDH) (as fold changes over changes in compound concentration) in response to a test compound X added to a microfluidic "Human Liver-on-Chip." This graph shows an increased release of AST, ALT and GLDH on the human liver-on-chip under interaction of high concentrations of test compound X demonstrating compound toxicity effect under 30µM concentration of this compound.

### Example 17: Hepatocyte Seeding Guidelines for Microfluidic HUMAN Liver-On-Chips

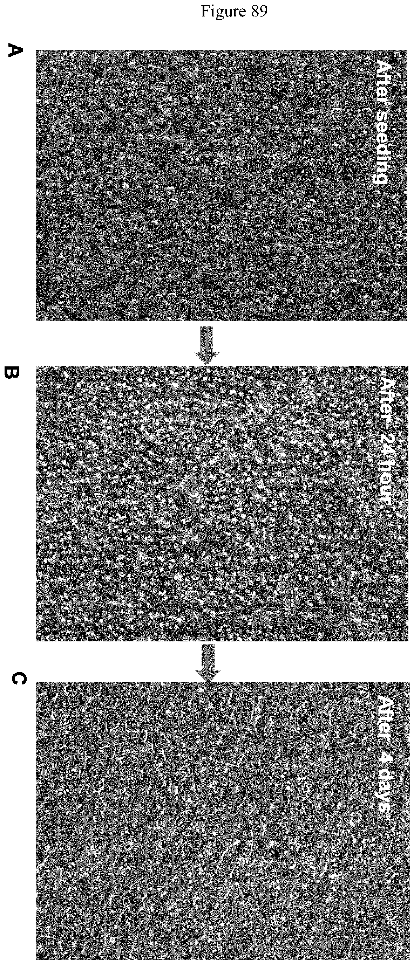

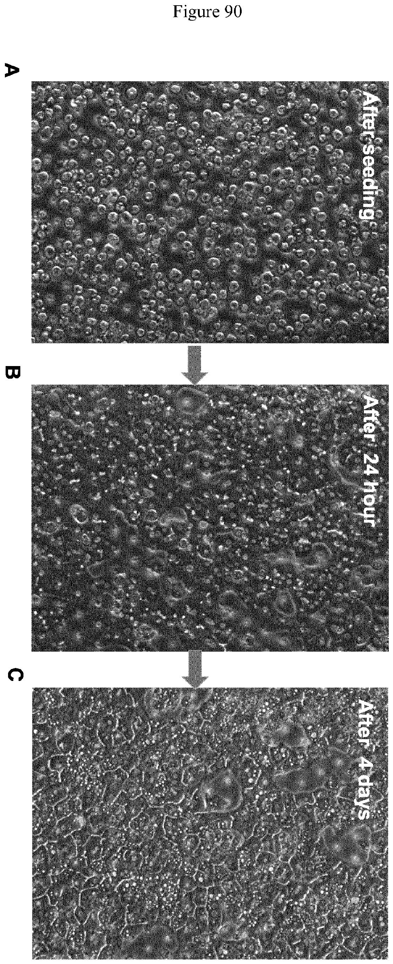

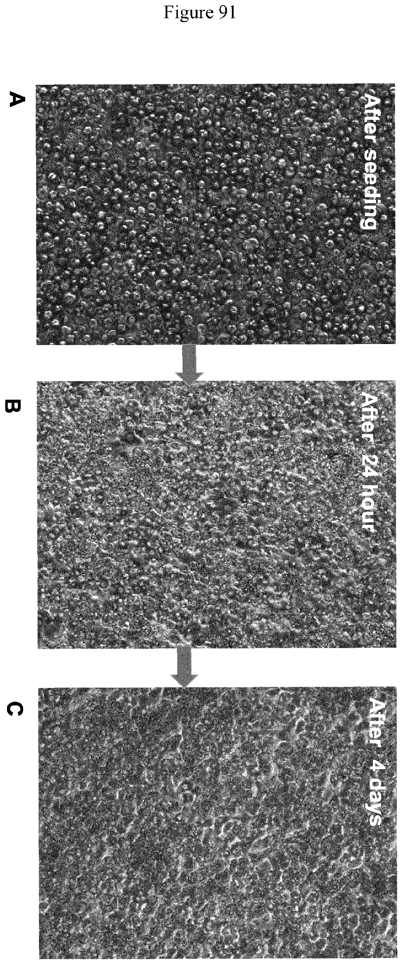

During the development of the present inventions seeding density of hepatocytes into Liver-On-Chips was discovered to have an effect on Liver-Chip viability and function. Specifically, overseeding or underseeding reduces Liver-Chip viability and function while optimal seeding, ranging from 2.1, 2.2, 2.3, 2.4, 2.5, 2.6, 2.7, 2.8, 2.9, 3.0, 3.1, 3.2, 3.4 up to 3.4 million cells/ml, preferably around 3.0 million cells/ml, provides a healthy Liver-On-Chip. The following show morphological examples of healthy cells in Figure 89, while less optimal cells are shown in exemplary Figures 90 and 91. Figure 89 provides exemplary micrographs showing results from hepatocyte cells seeding density experiments for providing Hepatocyte Seeding Guidelines in the microfluidic "Human Liver-on-Chips." Seeding density in a tall channel chip at 3 million cells/ml, in 40 µl. Phase contrast micrographs: A) immediately after seeding hepatocytes onto chips, B) 24 hours after seeding and C) 4 days after seeding hepatocyte cells. Figure 90 provides exemplary micrographs showing results from hepatocyte cells seeding density experiments showing underseeded microfluidic "Human Liver-on-Chips." Seeding density in a tall channel chip at 2 million cells/ml, in 40 µl. Phase contrast micrographs: A) immediately after seeding hepatocytes onto chips, B) 24 hours after seeding and C) 4 days after seeding hepatocyte cells. Figure 91 provides exemplary micrographs showing results from hepatocyte cells seeding density experiments for showing overseeded microfluidic "Human Liver-on-Chips." Seeding density in a tall channel chip at 3.5 million cells/ml, 40 µl. Phase contrast micrographs: A) immediately after seeding hepatocytes onto chips, B) 24 hours after seeding and C) 4 days after seeding hepatocyte cells.

## Claims

1. A method of culturing cells, comprising a) providing i) a microfluidic device comprising a top chamber, a bottom chamber, and a membrane, said membrane comprising a top surface and a bottom surface, ii) hepatocytes, stellate cells and endothelial cells and iii) material capable of forming a gel; b) introducing said material into said microfluidic device such that an extracellular matrix (ECM) gel forms in said top chamber; and c) introducing, in any order, i) said hepatocytes into said top chamber such that said ECM gel is between said hepatocytes and said top surface of said membrane, ii) stellate cells in said top chamber such that said stellate cells are within said ECM gel; and iii) said endothelial cells such that said endothelial cells affix to said bottom surface of said membrane; and d) culturing said cells under flow conditions with media.

2. The method of Claim 1, wherein said microfluidic device further comprises a removable top and said method further comprises, prior to step b), removing said removable top so that said material can be introduced in step b).

3. The method of Claim 1, wherein said hepatocytes are selected from the group consisting of viable rat hepatocytes, viable dog hepatocytes and viable human hepatocytes.

4. A microfluidic device comprising a first structure defining a first chamber comprising hepatocytes and stellate cells, a second structure defining a second chamber comprising liver sinusoidal endothelial cells and Kupffer cells, and a membrane located at an interface region between the first chamber and the second chamber, the membrane including a first side facing toward the first chamber and a second side facing toward the second chamber, wherein the first side of the membrane comprises an ECM gel layer and an ECM overlay, wherein said ECM gel layer is between the hepatocytes and said first side of said membrane and said stellate cells are embedded in said ECM gel layer, and wherein said hepatocytes are covered by said ECM overlay.

5. The device of Claim 1, wherein said stellate cells are primary cells.

6. The device of Claim 1, wherein said stellate cells are human cells.

7. The device of Claim 1, wherein said stellate cells are rat cells.

## Patentansprüche

1. Verfahren zum Kultivieren von Zellen, umfassend a) das Bereitstellen i) von einer mikrofluidischen Vorrichtung, die eine obere Kammer, eine untere Kammer und eine Membran umfasst, wobei die Membran eine obere Fläche und eine untere Fläche umfasst, ii) von Hepatozyten, Sternzellen und Endothelzellen und iii) von Material, das in der Lage ist, ein Gel zu bilden; b) das Einführen des Materials in die mikrofluidische Vorrichtung, sodass sich ein Extrazellulärmatrix(ECM)-Gel in der oberen Kammer bildet; und c) das Einführen, in beliebiger Reihenfolge, i) von den Hepatozyten in die obere Kammer, sodass sich das ECM-Gel zwischen den Hepatozyten und der oberen Fläche der Membran befindet, ii) von Sternzellen in die obere Kammer, sodass sich die Sternzellen innerhalb des ECM-Gels befinden; und iii) von den Endothelzellen, sodass sich die Endothelzellen an die untere Fläche der Membran anheften; und d) das Kultivieren der Zellen unter Strömungsbedingungen mit Medien.

2. Verfahren nach Anspruch 1, wobei die mikrofluidische Vorrichtung ferner einen abnehmbaren oberen Teil umfasst und das Verfahren ferner, vor Schritt b), das Abnehmen des abnehmbaren oberen Teils umfasst, sodass das Material in Schritt b) eingeführt werden kann.

3. Verfahren nach Anspruch 1, wobei die Hepatozyten ausgewählt sind aus der Gruppe bestehend aus lebensfähigen Rattenhepatozyten, lebensfähigen Hundehepatozyten und lebensfähigen humane Hepatozyten.

4. Mikrofluidische Vorrichtung, umfassend eine erste Struktur, die eine erste Kammer umfassend Hepatozyten und Sternzellen definiert, eine zweite Struktur, die eine zweite Kammer umfassend Leber-Sinusendothelzellen und Kupffer-Zellen definiert, und eine Membran, die sich an einem Grenzflächenbereich zwischen der ersten Kammer und der zweiten Kammer befindet, wobei die Membran eine erste Seite, die der ersten Kammer zugewandt ist, und eine zweite Seite, die der zweiten Kammer zugewandt ist, einschließt, wobei die erste Seite der Membran eine ECM-Gelschicht und einen ECM-Überzug umfasst, wobei sich die ECM-Gelschicht zwischen den Hepatozyten und der ersten Seite der Membran befindet und die Sternzellen in der ECM-Gelschicht eingebettet sind, und wobei die Hepatozyten durch den ECM-Überzug bedeckt sind.

5. Vorrichtung nach Anspruch 1, wobei die Sternzellen Primärzellen sind.

6. Vorrichtung nach Anspruch 1, wobei die Sternzellen humane Zellen sind.

7. Vorrichtung nach Anspruch 1, wobei die Sternzellen Rattenzellen sind.

## Revendications

1. Procédé de mise en culture de cellules, comprenant a) la fourniture i) d'un dispositif microfluidique comprenant une chambre supérieure, une chambre inférieure et une membrane, ladite membrane comprenant une surface supérieure et une surface inférieure, ii) d'hépatocytes, de cellules stellaires et de cellules endothéliales et iii) d'un matériau capable de former un gel ; b) l'introduction dudit matériau dans ledit dispositif microfluidique de sorte qu'un gel de matrice extracellulaire (ECM) se forme dans ladite chambre supérieure ; et c) l'introduction, dans n'importe quel ordre, i) desdits hépatocytes dans ladite chambre supérieure de sorte que ledit gel ECM se trouve entre lesdits hépatocytes et ladite surface supérieure de ladite membrane, ii) de cellules stellaires dans ladite chambre supérieure de sorte que lesdites cellules stellaires se trouvent à l'intérieur dudit gel ECM ; et iii) desdites cellules endothéliales de sorte que lesdites cellules endothéliales se fixent à ladite surface inférieure de ladite membrane ; et d) la mise en culture desdites cellules dans des conditions d'écoulement avec des milieux.

2. Procédé selon la revendication 1, dans lequel ledit dispositif microfluidique comprend en outre un couvercle amovible et ledit procédé comprend en outre, avant l'étape b), le retrait dudit couvercle amovible afin que ledit matériau puisse être introduit à l'étape b).

3. Procédé selon la revendication 1, dans lequel les hépatocytes sont choisis dans le groupe constitué d'hépatocytes viables de rats, d'hépatocytes viables de chiens et d'hépatocytes viables d'humains.

4. Dispositif microfluidique comprenant une première structure définissant une première chambre comprenant des hépatocytes et des cellules stellaires, une deuxième structure définissant une deuxième chambre comprenant des cellules endothéliales sinusoïdales de foie et des cellules de Kupffer, et une membrane située dans une région d'interface entre la première chambre et la deuxième chambre, la membrane incluant une première face orientée vers la première chambre et une deuxième face orientée vers la deuxième chambre, dans lequel la première face de la membrane comprend une couche de gel ECM et un revêtement ECM, dans lequel ladite couche de gel ECM se trouve entre les hépatocytes et ladite première face de ladite membrane et lesdites cellules stellaires sont incrustées dans ladite couche de gel ECM, et dans lequel lesdits hépatocytes sont recouverts par ledit revêtement ECM.

5. Dispositif selon la revendication 1, dans lequel lesdites cellules stellaires sont des cellules primaires.

6. Dispositif selon la revendication 1, dans lequel lesdites cellules stellaires sont des cellules humaines.

7. Dispositif selon la revendication 1, dans lequel lesdites cellules stellaires sont des cellules de rat.
